# EUROPÄISCHE PATENTSCHRIFT

(11) **EP 0 799 224 B1**
(45) Veröffentlichungstag und Bekanntmachung des Hinweises auf die Patenterteilung: **17.04.2002**
(21) Anmeldenummer: 95904415.7
(22) Anmeldetag: 21.12.1994
(51) Int. Cl.: C07D 403/04, C07D 401/14, A61K 31/495

(54) **CHINOXALINE UND ARZNEIMITTEL DARAUS**
QUINOXALINES AND DRUGS PREPARED THEREFROM
QUINOXALINES ET MEDICAMENTS PREPARES A PARTIR DESDITES QUINOXALINES

(43) Veröffentlichungstag der Anmeldung: 08.10.1997
(73) Patentinhaber: BASF AKTIENGESELLSCHAFT, 67056 Ludwigshafen (DE)
(72) Erfinder: LUBISCH, Wilfried, D-68159 Mannheim (DE); BEHL, Berthold, D-67117 Limburgerhof (DE); HOFMANN, Hans, Peter, D-67117 Limburgerhof (DE)
(74) Vertreter: Grünecker, Kinkeldey, Stockmair & Schwanhäusser Anwaltssozietät
(86) Internationale Anmeldenummer: EP9403839
(87) Internationale Veröffentlichungsnummer: WO9619476

(56) Entgegenhaltungen:
- EP-A- 0 572 852

## Beschreibung

Die vorliegende Erfindung betrifft Chinoxalin-2,3-(1H, 4H)-dione der allgemeinen Formel I 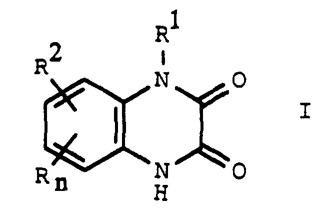 und ihre tautomeren und enantiomeren Formen sowie ihre physiologisch verträglichen Salze, wobei die Variablen folgende Bedeutung haben:
R¹
   - Wasserstoff,
   - ein cycloaliphatischer Rest mit bis zu 8 C-Atomen
   - ein aliphatischer Rest mit 1 bis 6 C-Atomen, der einen oder zwei gleiche oder verschiedene der Substituenten Phenyl, Cyclopentyl, Cyclohexyl, -COR³, -CO-O-R³,
      -CO-NH-R³, OR³, 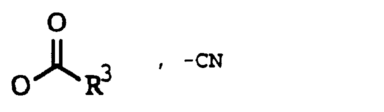 tragen kann, wobei R³ Wasserstoff, C₁-C₄-Alkyl, Phenyl, Benzyl, 1-Phenylethyl oder 2-Phenylethyl bedeutet, und wobei der in R¹ enthaltene oder R¹ darstellende cycloaliphatische Rest oder Phenylring bis zu drei gleiche oder verschiedene der folgenden Substituenten tragen kann:
      C₁-C₄-Alkyl, C₁-C₄-Halogenalkyl, C₁-C₄-Alkoxy, C₁-C₄-Halogenalkoxy, Halogen, Nitro, Cyano, -CO-OR⁵, -CO-NH-R⁵, -OH, 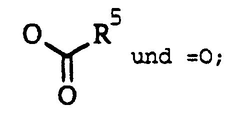 und wobei R⁵ unabhängig von R³ die gleichen Bedeutungen wie R³ hat,
R²
   - eine Pyrrolgruppe 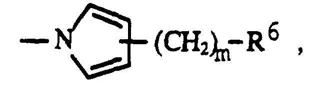
   wobei R⁶ einer der folgenden Reste ist : 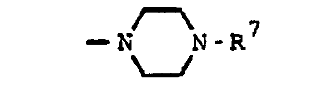 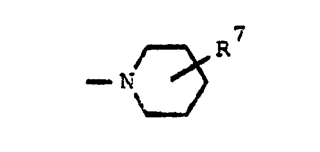 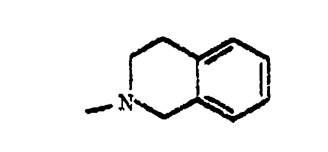 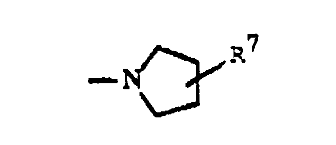 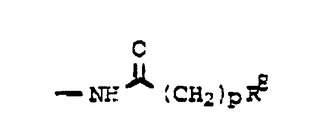 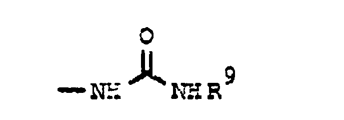 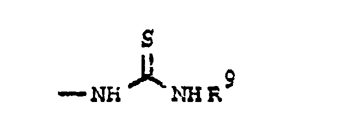 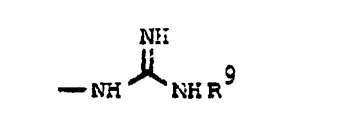 wobei R⁷ Wasserstoff, verzweigtes oder geradliniges
   C₁-C₄-Alkyl, das noch einen oder zwei Pherylringe tragen kann, bedeutet, und R⁶ einen der Reste 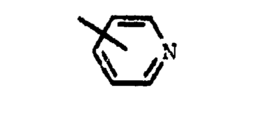 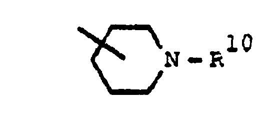 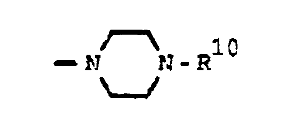 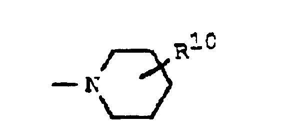 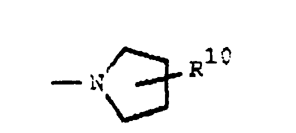 bedeutet, wobei R¹⁰ Wasserstoff und ein verzweigtes oder geradliniges C₁-C₄-Alkyl, das noch einen oder zwei Phenylringe tragen kannn, bedeutet,
R⁹ Wasserstoff, Phenyl, ein verzweigtes oder geradliniges C₁-C₄-Alkyl, das noch einen oder zwei Phenylringe tragen kann, sowie 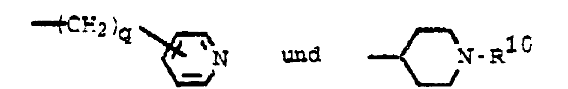 bedeutet,
m eine ganze Zahl von 1 bis 4 ist,
p und q unabhangig voneinander eine Zahl von 0 bis 4 bedeuten,
wobei die in R⁷, R⁹ und R¹⁰ ggf. enthaltenen Phenyl-Gruppen sowie der für R⁸ und R⁹ ggf. stehende oder in R⁹ ggf. enthaltene Pyridinring durch Halogen, -NO₂, -CF₂, -CN, -OH, -OCH₃, -NH₂, -NHCOCH₃, -OCF₃, -CO₂-(C₁-C₄-Alkyl), -CO₂H, -CONH₂, -CONH-(C₁-C₄-Alkyl. -CH₂-NHCOCF₃. -CH₂NH₂ und C₁-C₄-Alkyl substituiert sein können,
R
   - gleiche oder verschiedene der folgenden Reste:
      C₁-C₄-Alkyl, C₁-C₄-Alkoxy, Trifluormethyl, Trichlormethyl, Trifluormethoxy, Trichlormethoxy, Fluor, Chlor, Brom, Jod, Nitro und Cyano, sowie einen anellierten Benzolring, der seinerseits bis zu zwei der oben für R genannten Reste (ohne den anellierten Benzolring) tragen kann, und
n
   - eine ganze Zahl von 0 bis 2, für den anellierten Benzolring 0 oder 1 bedeutet.

Außerdem betrifft die Erfindung die Verwendung der Verbindungen I als Arzneimittel für die Human-und Veterinärmedizin.

Derivate des Chinoxalin-2,3(1H,4H)-dions 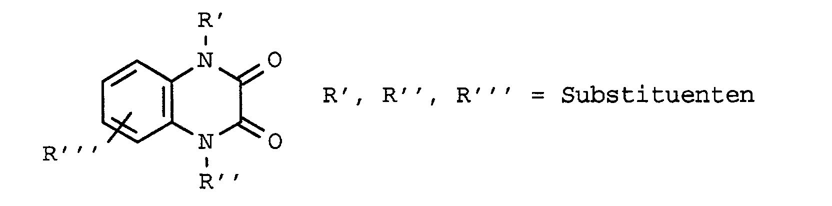 wurden in mehreren Veröffentlichungen zur Behandlung von Erkrankungen des zentralen Nervensystems sowie als Schlaf- und Beruhigungsmittel vorgeschlagen. Beispielsweise werden in EP-A315959, EP-A374534 und EP-A377112 Verbindungen beschrieben, in denen R''' Halogen, Nitro, Cyano, Hydroxy, Trifluormethyl, C₁-C₄-Alkoxy and die Gruppen -SO₂H, SO₂R^{x}, -SONH₂, -SO₂NHR^{x} und SO₂NR₂^{x}, wobei R^{x} für C₁-C₄-Alkyl steht, sowie einen anellierten Benzolring, der auch substituiert sein kann, bedeuten. In US-A3992378 handelt es sich bei dem Substituenten R''' um einen C₁-C₂-Fluoralkylrest und in PCT 91/13878 neben Halogen und Nitro um C₁-C₆-Alkyl, Alkoxy, Aryloxy und Aralkoxy.

In der EP-A 572 852 sind ebenfalls strukturell ähnliche Pyrrolsubstituierte Chinoxalin-dione als Arzneimittel beschrieben, die sich von den in der vorliegenden Anmeldung beanspruchten Verbindungen im Substitutionsmuster am Pyrrolring unterscheiden.

Darüber hinaus sind in der EP-A 8864 auch Piperidinyl, Pyrrolidinyl und Piperazinyl als Substituenten R''' erwähnt. Verbindungen der letztgenannten Art sind auch aus Ind. J. Chem. 28B (1989), S. 888-890 bekannt; in einer darüber hinaus hier noch erwähnten Überprüfung ihrer Verwendbarkeit als Wurmmittel zur Bekämpfung von Haken- und Bandwürmern erwiesen sich diese Verbindungen als ungeeignet.

Imidazolyl- und Triazolyl-chinoxaline wurden in WO92/07847 beansprucht, und zwar ebenfalls als Inhibitoren der Glutamatrezeptoren.

Die bekannten Verbindungen zeigen den Nachteil, daß sie die Blut-Hirn-Schranke nicht oder nur schlecht überwinden können und daher in ihrer Wirkung auf das Zentralnervensystem zu wünschen übrig lassen.

Der Erfindung lagen daher neue, wirksamere Chinoxalin-2,3-(1H,4H)-dione als Aufgabe zugrunde.

Es wurde gefunden, daß die eingangs definierten Verbindungen I diese Aufgabe losen.

Ferner wurden verschiedene, im folgenden näher beschriebene Verfahren für die Herstellung der Chinoxalin-2,3(1H,4H)-dione I sowie deren Verwendung als Arzneimittel für die Human- und Veterinârmedizin gefunden.

Prinzipiell kann man die erfindungsgemäßen Verbindungen I auf mehreren Wegen herstellen, wobei man ein Aminophenylen-1,2-diamin II 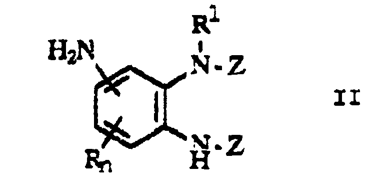 wobei z für eine als schutzgruppe fungierende Acylgruppe steht, mit einem Bernsteindialdehyd-Derivat oder seinem acyclischen oder cyclischen Acetal oder Hemiacetal (III) zu IV 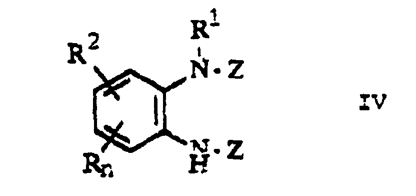 umsetzt, und nach Abspaltung der Schutzgruppe Z den Ringschluß zu I in an sich bekannter Weise mit Oxalsäure oder einem ihrer funktionellen Derivate vornimmt,
oder indem man II in schutzgruppenfreier Form zunächst mit Oxalsäure oder einem ihrer funktionellen Derivate in die entsprechenden Aminochinoxalin-2,3-(1H,4H)-dione überführt und diese danach mit III zu Pyrrolylchinoxalin umsetzt, das danach in geeigneter Weise zu I derivatisiert wird.

Im einzelnen sind die erfindungsgemäßen Verbindungen I folgendermaßen erhältlich:

Man geht von einem Aminophenylen-1,2-diamin II aus, in welchem Z für eine als Schutzgruppe fungierende Acylgruppe, vorzugsweise Acetyl oder Trifluoracetyl, steht, und setzt dieses in bekannter Weise, z.B. gemäß A.R. Katritzky und C.W. Rees, "Comprehensive Heterocyclic Chemistry", Bd. 4, Teil 3.06, S. 313 ff, in Gegenwart von katalytischen Mengen einer Säure, wie Essigsäure, mit einer Verbindung III, vorzugsweise dem cyclischen Acetal, unter Wasserabspaltung zu IV um.

Die Säure kann auch als Lösungsmittel dienen, wenn sie in größeren Mengen verwendet wird. Im allgemeinen ist es jedoch üblich, die Umsetzung in einem Lösungsmittel wie Toluol oder in einem Lösungsmittelgemisch wie Toluol/Dimethylformamid unter Säurekatalyse bei einer Reaktionstemperatur von 50 bis 150°C, vorzugsweise 100 bis 150°C, oder in konzentrierter Essigsäure bei Temperaturen von 50°C bis zum Siedepunkt vorzunehmen.

Nach Abspaltung der Schutzgruppe erfolgt in an sich bekannter Weise der Ringschluß von IV mit Oxalsäure oder einem ihrer funktionellen Derivate, vorzugsweise Oxalsäurediester wie Oxalsäuredimethylester oder Oxalsaurediethylester, zu I. Der Ringschluß ist hinsichtlich der Reaktionstemperatur und Reaktionszeit allgemein bekannt. Ebenso kann mit Oxalsäuremonoethylesterchlorid ein Monoamid hergestellt werden, das bei erhöhter Temperatur zum Chinoxalin-dion cyclisiert.

Aminophenylen-1,2-diamine II sind z. B. aus US-A3992378 bekannt oder hiernach erhältlich. Ferner lassen sie sich aus kommerziell erhältlichen o-Phenylendiaminen nach Einführung von Schutzgruppen auf übliche Weise durch Nitrierung und anschließende Reduktion der Nitrogruppe herstellen.

Eine weitere Möglichkeit zur Herstellung der Ausgangsverbindung II besteht in der allgemein bekannten Umsetzung von 2-Nitrochlorbenzolen mit Aminen wie Ethylamin, Cyclohexylamin, 1-Phenylethylamin und α-Aminoessigsauren zu den entsprechenden 2-Nitroanilinen, der anschließenden Reduktion der Nitrogruppe, Einführung von Schutzgruppen sowie Nitrierung und Reduktion der weiteren Nitrogruppe.

Die Umsetzung der 2-Nitro-chlorbenzole mit Aminen ist allgemein bekannt und wird üblicherweise in polaren Lösungsmitteln wie Dimethylformamid, Dimethylsulfoxid und Ethanol in Gegenwart von basischen Salzen wie Kaliumcarbonat bei einer Reaktionstemperatur von 25 bis 180°C, vorzugsweise von 25 bis 140°C durchgeführt.

Ebenso kann man 2,4-Difluornitrobenzol schrittweise mit 2 unterschiedlichen Aminen umsetzen, wobei zuerst das Fluor-Atom in 2-Stellung zur Reaktion gebracht werden kann (vgl. z.B. D.D.Davey et al., J. Med. Chem. 1991, 34, 2671). Der nachfolgende Austausch des zweiten Halogens mit H₂NR¹¹ (mit z. B. R¹¹ = H oder Benzyl) führt zu Diaminen VI, die anschließend analog den unten beschriebenen 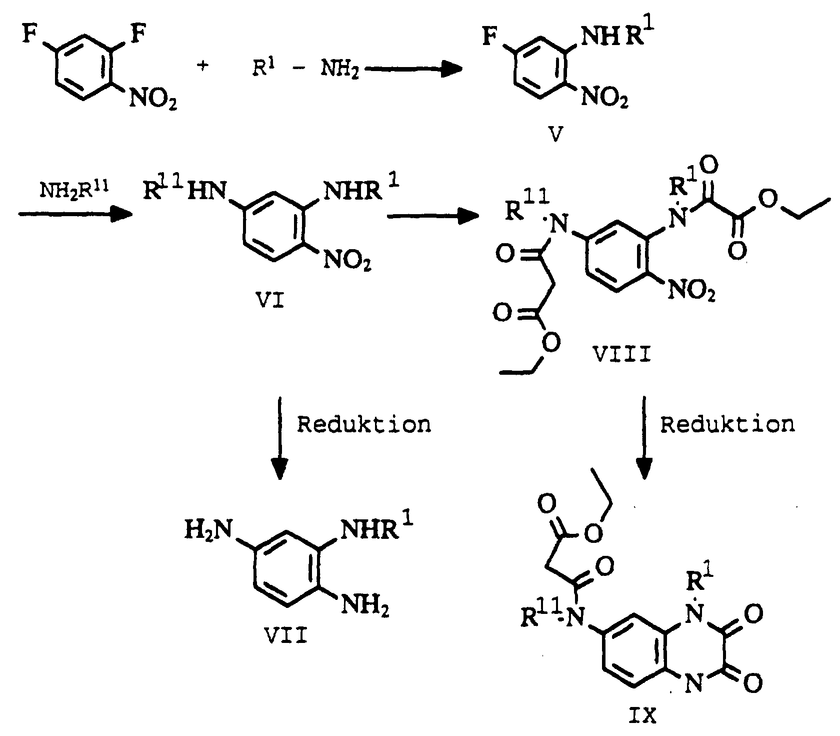 Methoden zu Triaminen reduziert werden, die danach entsprechend den Verbindungen IV in die Chinoxaline überführt werden können. Das Anilin VI kann ebenso mit einem Oxalsäuremonochlorid oder entsprechend aktivierten Oxalsäuremonoestern nach bekannten Verfahren (vgl. Houben-Weyl, Methoden der org. Chemie, Bd. E5, *Kap.* V) in die Anilide VIII überführt werden. VIII wird durch Reduktion, analog den unten beschriebenen Methoden, insbesondere aber durch Reduktion mit Pd/C und Wasserstoff, Pd/C und Wasserstoffträgern wie Ammoniumformiat oder mit Eisen und Essigsäure in Chinoxaline IX überführt. Durch anschließende Hydrolyse nach bekannten Verfahren, wie Erwärmung mit Salzsäure, und katalytische Abspaltung der Benzylgruppe (falls R¹¹ = Benzyl) erhält man Verbindungen des Typs II, die wie beschrieben in die Pyrrolderivate überführt werden können.

Chinoxaline sind weiterhin aus Nitroanilinen X zugänglich, indem man mit Oxalsäure-Derivaten das Amid XI herstellt, das dann durch Zugabe einer Base wie Natriumhydrid oder Kalium-tert.butanolat in das Amid-Anion überführt wird. Durch Zugabe eines Halogenids entsteht das Amid XII, das durch Reduktion, analog der Überführung von VIII nach IX, in ein Chinoxalin XIII überführt wird, das, wie unten beschrieben wird, zu den Produkten I derivatisiert werden kann. Die Alkylierung wird in Lösungsmitteln, bevorzugt polaren Lösungsmitteln wie Tetrahydrofuran und Dimethylformamid, bei Temperaturen von 25 - 150°C durchgeführt.

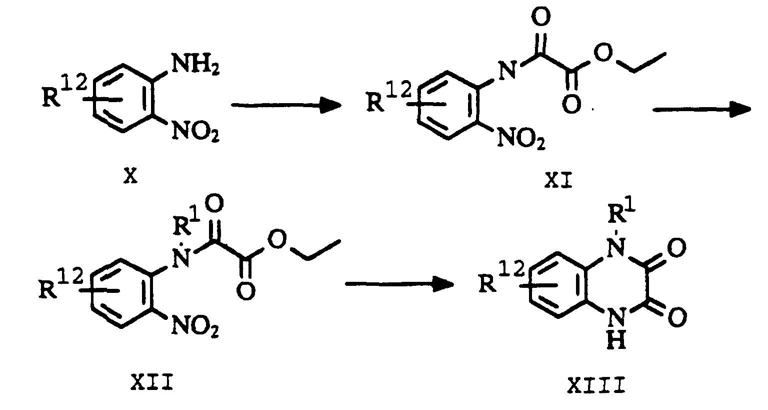

Die Einführung und Abspaltung der Schutzgruppen erfolgt jeweils nach allgemein üblichen Methoden, welche beispielsweise in T.W. Greene, "Procective Groups in Organic Synthesis", Wiley and Sons, New York 1982, Kap. 7, S. 249ff, zusammenfassend beschrieben sind.

Die organischen Syntheseschritte Nitrierung und Reduktion können jeweils nach den üblichen in Houben-Weyl, "Methoden der organischen Chemie", Bd. 10/1 bzw. Bd. 11/1 beschriebenen Methoden durchgeführt werden. Für die Nitrierung eignen sich Gemische aus Essigsäure-Salpetersäure und Schwefelsäure-Natriumnitrat.

Die Reduktion kann nach der chemischen und nach der katalytischen Methode erfolgen. Bei der katalytischen Variante wird z.B. mit Wasserstoff an den Katalysatoren Palladium/Aktivkohle und Platin/ Aktivkohle in Gegenwart eines Lösungsmittels reduziert, wobei aber auch als Wasserstoffüberträger chemische Substanzen wie Ammoniumformiat eingesetzt werden können; bei der chemischen Variante kann die Reduktion mit Natriumborhydrid/Kupfersulfat in Dimethylformamid und in Alkoholen wie Ethanol durchgeführt werden. Ferner ist es auch ublich, die Nitrogruppen mit Redoxsystemen wie Eisen/Salzsäure und Zink/Salzsäure zu reduzieren.

Eine weitere Möglichkeit zur Herstellung der erfindungsgemäßen Verbindungen I besteht darin, daß man die oben beschriebenen Syntheseschritte - Umsetzung mit III und Ringschlaß - in umgekehrter Reihenfolge durchfuhrt.

Man führt zunächst den Ringschluß durch, indem man ein in schutzgruppenfreier Form vorliegendes Aminophenylen-1,2-diamin II mit Oxalsäure oder einem ihrer funktionellen Derivate wie Oxalsäuredichlorid oder Oxalsäurediester nach den üblichen Methoden zu den entsprechenden Aminochinoxalin-2,3 (1H,4H)-dionen umsetzt. Vor der sich nun anschließenden Umsetzung mit III zu I empfiehlt es sich, wenn weitere Substituenten R, beispielsweise Nitro, in das Aminochinoxalindion eingeführt werden, die Aminogruppe durch eine Acylgruppe zu schützen, um dann - nach Abspaltung der Schutzgruppe in Gegenwart von Salzsäure - entweder die freien Aminochinoxalin-2,3(1H,4H)-dione oder ihre Hydrochloride mit III umsetzen.

In den so hergestellten Produkten I bzw. IV kann die Substitution des in R² beanspruchten Pyrrolyl-Ringes in geeigneter Weise umgewandelt werden. So kann z.B. der Aldehyd XIV durch reduktive Aminierung mit Aminen HR⁶ in die erfindungsgemäßen Verbindungen I überführt werden. Die reduktive Aminierung wird im allgemeinen bei Temperaturen von 5 bis 80°C, vorzugsweise 10 bis 30°C, in Gegenwart von Reduktionsmitteln wie Natriumcyanoborhydrid oder Wasserstoff in Gegenwart von Hydrierkatalysatoren wie Pd/Kohle, Pt/Kohle oder Raney-Nickel, zweckmäßig in polaren organischen Lösungsmitteln wie Alkoholen oder Dimethylformamid, durchgeführt.

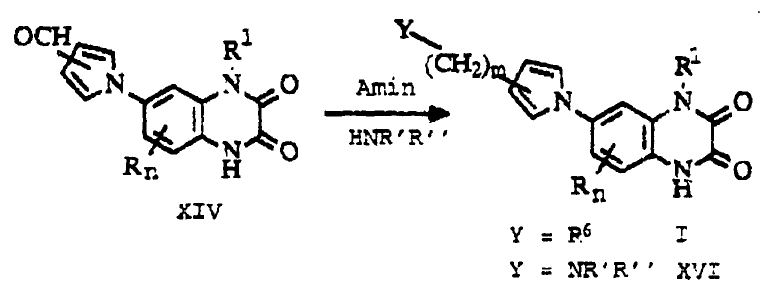

Andererseits können so zugängliche Pyrrolylalkylamine XVI mit Säuren R⁸(CH₂)ₚCO₂H, die in geeigneter Weise zu R⁸(CH₂)ₚCOX aktiviert werden, wobei X für eine Abgangsgruppe wie Azid, Imidazol und andere, die bei R.C. Larock, Comprehensive Organic Transformations, New York 1989, S. 972ff, aufgelistet sind, in die erfindungegemäßen Amide I'' überführt werden. Diese Kupplung geschieht nach bekannten Verfahren, die z. B. im Houben-Weyl "Methoden der organischen Chemie", Band E5, Kapitel V, aufgelistet sind.

Die Pyrrolylalkylamine XVI (R' = R" = H) können ebenfalls mit Isocyanaten zu den Harnstoffen I umgesetzt werden, wobei man anstelle der Isocyanate auch Amine HNHR³ verwenden kann, die in bekannter Weise zuvor mit Phosgen oder analogen verbindungen, wie Carbonyldiimidazol, umgesetzt werden. Diese und vergleichbare Verfahren sind beispielsweise in Houben-Weyl "Methoden der organischen Chemie", Band E4, S. 334ff. beschrieben. Bei diesen Verfahren arbeitet man mit oder ohne Lösungsmittel, wobei dieses bevorzugt Dimethylformamid ware, und bei Temperaturen von 25-150°C.

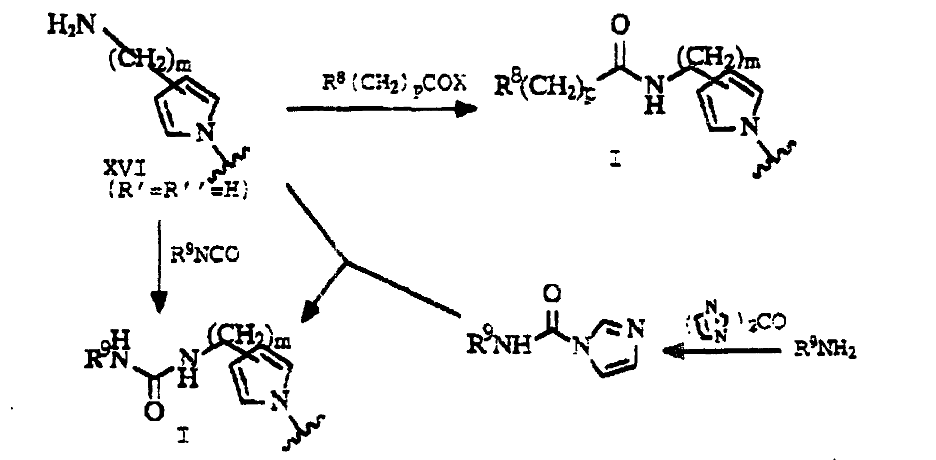

Die erfindungsgemäßen Verbindungen I stellen Antagonisten der exzitatorischen Aminosäuren. insbesondere Glycin-, AMPA- und Kainat-Antagonisten dar.

Die sogenannten exzitatorischen Aminosäuren wie Glutaminsäure sind im Zentralnervensystem (ZNS) häufig verbreitet. Diese exzitatorischen Aminosäuren fungieren als Transmittersubstanzen für die Glutamat-Rezeptoren, von denen man verschiedene Subtypen kennt. Ein Subtyp wird z. E. nach dem spezifischen Agonisten N-Methyl-D-Aspartat NMDA-Rezeptor genannt. Dieser NMDA-Rezeptor weist verschiedene Bindungsstellen für Agonisten bzw. Antagonisten auf. Die Aminosäure Glycin bindet ebenfalls am NMDA-Rezeptor und moduliert die Wirkung des natürlichen Agonisten Glutaminsäure. Antagonisten an dieser Glycin-Bindungsstelle können demnach antagonistische Effekte am NMDA-Rezeptor zeigen und eine "Übererregung" dieses Rezeptors hemmen.

Zwei andere Subtypen der Glutamat-Rezeptoren stellen der AMPAund der Kainat-Rezeptor dar, die jeweils nach den spezifischen Antagonisten 2-amino-3-hydroxy-5-methyl-4-isoxazole propionic acid (AMPA) und Kainic acid bezeichnet werden. Analog zum bereits genannten NMDA-Rezeptor können Antagonisten dieser Rezeptoren ebenfalls eine "Übererregung" hemmen.

Bei einer Reihe von neurodegenerativen Krankheiten oder psychischen Storungen treten erhöhte Glutamat-Spiegel auf, die zu übererregten Zuständen oder toxischen Effekten im ZNS führen können.

Antagonisten gegen die Glutamat-Rezeptor-Subtypen können somit zur Behandlung dieser Krankheiten dienen. Glutamat-Antagonisten, dazu gehören insbesondere auch NMDA-Antagonisten bzw. deren Modulatoren (beispielsweise Glycin-Antagonisten) und die AMPA-Antagonisten, eignen sich daher zur therapeutischen Anwendung als Mittel gegen neurodegenerative Krankheiten (Alzheimersche und Parkinsonsche Krankheitet), neurotoxische Störungen nach Hypoxie, Anoxie oder Ischämie, wie sie nach "Stroke" auftreten, oder auch als Antiepileptike, Antidepressiva und Anxiolytika (vgl. Arzneim. Forschung 1990, 40, 511-514; TIPS, 1990, 11, 334-338 und Drugs of the Future 1989, 14 (11), 1059-1071).

Die pharmakologische Wirksamkeit der erfindungsgemäßen Verbindungen I wurde an isoliertem Membranmaterial von Rattenhirnen untersucht. Hierzu wurde das Memoranmaterial in Gegenwart der erfindungsgemäßen Substanzen mit den radioaktiv markierten Substanzen ³H-2-Amino-3-hydroxy-5-methyl-4-isoxazolpropionsäure (³H-AMPA), ³H-5,7-Dichlorkynurensäure oder ³H-Kainat inkubiert. Dabei binden die genannten Radioliganden an jeweils für sie spezifische Glutamatrezeptoren, d.h. an AMFA-Rezeptoren, (³H-5,7-Dichlorkynurensäure-bindende) NMDA-Rezeptoren bzw. Kainat-Rezeptoren. Nach der Inkubation wurden die Membranen mit den rezeptorgebundenen Radioliganden von den freien Radioliganden getrennt und die mit den Membranen assozilerte Radioaktivität durch Flüssigkeitsscintillationszählung ermittelt. Über die Radioaktivität der Membranen läßt sich die Konzentration an rezeptorgebundenen Liganden sowie das Ausmaß der Verdrängung eines gegebenen Radioliganden durch bestimmte Konzentrationen der erfindungsgemäßen Verbindungen I bestimmen. Die sich hieraus ergebende Dissoziationskonstante K_{I} (I = Inhibitor), welche ein Maß für die Verdrängungswirkung des erfindungsgemäßen Wirkstoffes ist, wurde durch iterative nichtlineare Regressionsanalyse mit dem Statistical Analysis System (SAS) an einem IBM-Rechner, ähnlich dem Programm "Ligand" von P.J. Munson und D. Rodbard (Analytical Biochem. 107, 220 (1980), Ligand: Versatile Computerized Approach for Charakterization of Ligand Binding Systems) ermittelt.

Folgende In-Vitro-Untersuchungen wurden durchgeführt:
1. Bindung von ³H-2-Amino-3-hydroxy-5-methyl-4-isoxazolpropionsäure (³H-AMPA)
   Für die Präparation des Membranmaterials wurden frisch entnommene Rattengroßhirne zusammen mit dem ca. 15fachen Volumen einer Pufferlösung A aus 30 mM α,α,α-Tris-(hydroxymethyl)-methylamin-Hydrochlorid (TRIS-HCl) und 0,5 mM Ethylendiamintetraessigsaure (EDTA) - pH 7,4 - mittels eines Ultra-Turrax-Rührers homogenisiert. Die Suspension wurde 20 Minuten bei 48000 g zentrifugiert. Nach Abtrennung der überstehenden Flüssigkeit wurde das im Bodensatz enthaltene proteinhaltige Membranmaterial dreimal durch Suspendieren in der Pufferlösung A und anschließendes, jeweils 20minütiges Zentrifugieren bei 48000 g gewaschen. Danach wurde das Membranmaterial in einem 15fachen Volumen der Pufferlösung A suspendiert und 30 Minuten bei 37°C inkubiert. Anschließend wurde das Proteinmaterial zweimal durch Zentrifugieren und Suspendieren gewaschen und bis zur Verwendung bei -70°C eingefroren.
   Für den Bindungstest wurde das bei 37°C aufgetaute Proteinmaterial zweimal durch Zentrifugieren bei 48000 g (20 Minuten) und anschließendes Suspendieren in einer Pufferlösung B aus 50 mM TRIS-HCl, 0,1 M Kaliumthiocyanat und 2.5 mM Calciumchlorid - pH 7,1 - gewaschen. Angchlieβend wurden 0,25 mg Membranmaterial. 0,1 µCi ³H-AMPA (60 Ci/mmol) sowie Verbindung I bzw. I' in 1 ml Pufferlösung P gelöst und 60 Minuten auf Eis inkubiert. Die inkubierte Lösung wurde uber einen CF/B-Filter (Firma Whatman), der zuvor mindestens 2 Stunden mit einer 0,5 %igen wäßrigen Lösung von Polyethylenimin behandelt worden war, filtriert. Anschließend wurde der Membranrückstand mit 5 ml kalter Pufferlosung B gewaschen, um gebundene und freie ³H-AMPA voneinander zu trennen. Nach Messung der Radioaktivität der gebundenen ³H-AMPA im Membranmaterial durch Scintillatichszählung wurde durch Auswertung der Verdrangungskurven mittels Regressionsanalyse der K_{I}-Wert bestimmt.
   Für N-(1-(6-Nitrochinoxalin-2,3-(1H,4H)-dion-7-yl)-pyrrol-3-yl)methyl)-4-carbamoylpyridiniumacetat (Beispiel 3) und 1-Carboxymethyl-6-nitro-7-(3-((1-piperidinyl)methyl)pyrrol-1-yl)chinoxalin-2,3-(1H,4H)-dion (Beispiel 14) wurden ermittelt: K_{I} < 10 µM.
2. Bindung von ³H-5,7-Dichlorkynurensäure
   Für die Präparation des Membranmaterials wurden frisch entnommene Rattengroßhirne zusammen mit dem ca. 10fachen Volumen einer Pufferlösung A' aus 50 mM TRIS-HCl und 10mM EDTA - pH 7,4 - homogenisiert. Die Suspension wurde 20 Minuten bei 48000 g zentrifugiert. Nach Abtrennung der überstehenden Flüssigkeit wurde das im Bodensatz enthaltene Membranmaterial zweimal durch Suspension in der Pufferlösung A' und anschließendes jeweils 20minütiges Zentrifugieren gewaschen. Nach erneutem Suspendieren der Membrane in der Pufferlösung A' und Einfrieren in flüssigem Stickstoff wurde die Suspension wieder bei 37°C aufgetaut und nach einem weiteren Waschvorgang 15 Minuten bei 37°C inkubiert. Anschließend wurde das Proteinmaterial viermal durch Zentrifugieren und Suspendieren gewaschen und bis zur Verwendung bei -70°C eingefroren.
   Für den Bindungstest wurde das bei 37°C aufgetaute Proteinmaterial zweimal durch Zentrifugieren bei 48000g (20 Minuten) und anschließendes Suspendieren in einer Pufferlösung B' aus 50 mM TRIS-HCl - pH 7,4 - gewaschen. Anschließend wurden 0,15 mg Membranmaterial, 0,3 µCi ³H-5,7-Dichlorkynurensäure (16 Ci/mmol) sowie Verbindung I bzw. I' in 1 ml Pufferlösung B' gelöst und 30 Minuten auf Eis inkubiert. Die inkubierte Lösung wurde 2 Minuten bei 150000g zentrifugiert. Nach Abtrennung der überstehenden Flüssigkeit wurden die Bodensätze zweimal mit je 1,5 ml kalter Pufferlösung B' suspendiert. Nach Messung der Radioaktivität der an die Membranen gebundenen ³H-5,7-Dichlorkynurensäure im Bodensatz ergab sich der K_{I}-Wert durch Auswertung der Verdrängungskurven mittels der Regressionsanalyse.

Die erfindungsgemäßen Verbindungen I sind als Arzneimittel für die Human- und Veterinarmedizin geeignet und können zur Herstellung von Arzneimitteln zur Behandlung neurodegenerativer Erkrankungen und neurotoxischer Störungen des zentralen Nervensystems sowie zur Herstellung von Antiepileptika, Anxiolytika und Antidepressiva verwendet werden.

Die erfindungsgemäßen. Arzneimittelzubereitungen enthalten neben den üblichen Arzneimittelhilfsstoffen eine therapeutisch wirksame Menge der Verbindungen I. Für die lokale äußere Anwendung, z.B. in Puder und Salben, sind die Wirkstoffe in einer Konzentration von 0,0001 bis 1 Gew.-%. vorzugsweise 0.001 bis 0,1 Gew.-%, enthalten.

Bei der inneren Anwendung werden die Präparationen in Einzeldosen verabreicht. In einer Einzeldosis werden pro kg Körpergewicht 0,1 bis 100 mg gegeben. Die Zubereitungen können täglich in einer oder mehreren Dosierungen je nach Art und Schwere der Erkrankung verabreicht werden.

Entsprechend der gewünschten Applikationsart enthalten die erfindungsgemäßen Arzneimittelzubereitungen neben dem Wirkstoff die ublichen Trägerstoffe und Verdünnungsmittel. Für die lokale äußere Anwendung können pharmazeutisch-technische Hilfsstoffe, wie Ethanol, Isopropanol, oxethyliertes Ricinusöl, oxethyliertes hydriertes Ricinusöl, Polyacrylsäure, Polyethylenglykol, Polyethylenglykolstearat, ethoxylierte Fettalkohole, Paraffinöl, Vaseline und Wollfett verwendet werden. Für die innere Anwendung eignen sich z. B. Milchzucker, Propylenglykol, Ethanol, Stärke, Talk und Polyvinylpyrrolidon.

Ferner können Antioxidationsmittel wie Tocapherol und butyliertes Hydroxyanisol sowie butyliertes Hydroxytoluol, geschmacksverbessernde Zusatzstoffe, Stabilisierungs-, Emulgier- und Gleitmittel enthalten Bein.

Die neben dem Wirkstoff in der Zubereitung enthaltenen Stoffe sowie die bei der Herstellung der pharmazeutischen zubereitung verwendeten Stoffe sind toxikologisch unbedenklich und mit dem jeweiligen Wirkstoff verträglich.

Die Herstellung der Arzneimittelzubereitungen erfolgt in üblicher Weise, z. s. durch Vermischen des Wirkstoffes mit den anderen üblichen Trägerstoffen und Verdünnungsmitteln.

Die Arzneimittelzubereitungen können in verschiedenen Applikationsweisen verabreicht werden, wie peroral, parenteral, subkutan, intraperitoneal und topisch. So sind Zubereitungsformen wie Tabletten, Emulsionen, Infusions- und Injektionslösungen, Pasten, Salben, Gele, Cremes, Lotionen, Puder und Sprays möglich.

### Beispiele

### Beispiel 1

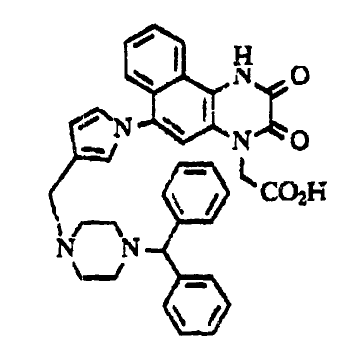

1-Carboxymethyl-9(3-(4(1,1-diphenylmethyl)-piperazin-1-ylmethyl)-pyrrol-1-yl)benzo[f]chinoxalin-2,3-(1H,4H)-dion
a) 2-Methoxy-1-nitro-naphthalin
   100 g (0,63 Mol 2-Methoxynaphthalin wurden in 1,2 l Essigsäure gelöst und bei 10°C langsam 100 ml 65%iger Salpetersäure zugetropft. Man rührte noch 2 h bei 10°C. Anschließend wurde der anfallende Niederschlag abgesaugt. Man erhielt 72,5 g (57 %). Schmp. 129 - 130°C.
   ¹H-NMR (D₆-DMSO):
   - δ =: 4,1 (3H); 7,5 - 7,8 (4H); 8,05 (1H) und 8.2 (1H) ppm.
b) N-(1-Nitro-3-naphthyl)-aminoessigsäure
   50 g (0,246 Mol) des Produktes 1 a, 100 g (1,3 Mol) Glycin und 100 g (0,7 Mol) Kaliumkarbonat wurden in 400 ml Diethylenglykol 10 Min, auf 140°C erwärmt. Anschließend wurde der Ansatz sofort auf Eiswasser gegessen, mit konzentrierter Salzsäure sauer gestellt und mit 1,5 l Essigsäureethylester extrahiert. Der resultierende Niederschlag wurde abgesaugt. Man erhielt 29,6 g (49 %) des Prooduktes, Schmp. > 155°C (Z.).
   ¹H-NMR (D₆-DMSO):
   - δ =: 4,3 (2H); 7.2 (1H); 7,35 (3H); 7.6 (1H); 7,8 (1H);8,0 (1H); 8,4 (1H); 8,7 (1H,NH) und ca*.* 12 (breic) ppm.
c) N-(1-Amino-2-naphthyl)-aminoessigsaure
   28 g (0,11 Mol) des Produktes 1 b wurden in 300 ml Ethanol gelöst und nach Zugabe von 10 ml Essigsäure und 2 g Palladium/Kohle (10 %) bei Raumtemperatur und 1 bar hydriert. Anschließend wurde filtriert und das Filtrat im Vakuum eingeengt. Man erhielt 23,9 g (98 %) des Produktes.
   ¹H-NMR (D₆-DMSO):
   - δ =: 3,8 (2H); 6,2 (NH); 7,1 (1H); 7,2 (1H); 7,3 - 7,5 (2H); 7,7 (1H); 8,1 (1H) und 10,5 (1H, CO₂H) ppm.
d) 1-(Carboxymethyl)-benzo[f]chinoxalin-2,3-(1H,4H)-dion
   22 g (0,1 Mol) des Produktes 1 c und 28 ml (0,2 Mol) Triethylamin wurden in 300 ml wasserfreiem Tetrahydrofuran gelöst. Bei 0 bis 5°C wurden danach 12,5 ml (0,11 Mol) Oxalsäureethylesterchlorid, gelöst in 50 ml wasserfreiem Tetrahydrofuran, zugetropft. Man rührte alles 1 h bei 0 bis 5°C. Anschließend wurde der Ansatz im Vakuum eingeengt und der Rückstand zwischen Essigsäureethylester und Wasser verteilt. Die organische Phase wurde getrocknet und im Vakuum eingeengt. Dieser Rückstand wurde in einem Gemisch aus 50 ml Ethanol und 200 ml konzentrierter Salzsäure 1,5 h unter Rückfluß gekocht. Anschließend wurde alles auf Eiswasser gegossen und der Niederschlag abgesaugt. Man erhielt 16,8 g (61 %) des Produktes. Schmp. 288 - 291°C (Z.).
   ¹H-NMR (D₆-DMSO):
   - δ =: 5,0 (2H); 7,4 - 7,6 (3H); 7,7 (1H); 7,9 (1H); 8,6 (1H); 12,3 (1H) und ca. 13 (breit) ppm.
e) 1-(Ethoxycarbonylmethyl)-benzo[f]chinoxalin-2,3-(1H,4H)-dion
   17 g (62,9 mmol) des Produktes 1 d wurden in einem Gemisch aus 250 ml konzentrierter Schwefelsäure und 70 ml Ethanol 3 h bei 55°C gerührt. Anschließend wurde alles auf Eiswasser gegossen und der Niederschlag abgesaugt. Man erhielt 18,3 g (98 %) des Produktes. Schmp. > 250°C.
   ¹H-NMR (D₆-DMSO):
   - δ =: 1,2 (3H); 4,2 (2H); 5,1 (2H); 7,3 - 7,6 (4H); 7,85 (1H) und 8,7 (1H) ppm.
f) 1-(Ethoxycarbonylmethyl)-9-nitro-benzo[f]chinoxalin-2,3-(1H,4H)-dion
   18 g (60,3 mmol) des Produktes 1 e wurden in 200 ml Essigsäure suspendiert und bei Raumtemperatur vorsichtig mit 50 ml 65 %iger Salpetersäure versetzt. Danach wurde auf 80°C erwärmt. Nach Beendigung der Reaktion (Farbumschlag der Lösung von dunkel auf hellrot) wurde alles auf Eis gegossen und der Niederschlag abgesaugt. Man erhielt 16,9 g (82 %) des Produktes, Schmp. > 250°C.
   ¹H-NMR (D_{δ}-DMSO):
   - δ =: 1,3 (3H); 4,2 (2H); 5.2 (2H); 7,7 (2H); 8,3 - 8,4 (2H); 8,8 (1H) und 12,7 (1H) ppm.
g) 9-Amino-1-(ethoxycarbonylmethyl)-benzo[f]chinoxalin-2,3-(1H,4H)-dion
   16,5 g (4B mmol) des Produktes 1 f wurden in 150 ml Dimethylformamid gelost und nach Zusatz von 1,5 g Palladium/Kohle (10 %) bei 1 bar und Raumtemperatur hydriert. Anschließend wurde filtriert und das Filtrat im Vakuum eingeengt. Man erhielt 13,1 g (88 %) des Produktes. Schmp. >250°C.
   ¹H-NMR (D₆-DMSO):
   - δ =: 1,2 (3H); 4,2 (2H); 5,0 (2H); 5,7 - 6,0 (2H, NH, breit); 6,6 (1H); 7,4 (1H); 7,5 (1H); 8.2 (1H); 8,5 (1H) und 12,0 (1H) ppm.
h) 1-Ethoxycarbonylmethyl-9(3-formylprrol-1-yl)-penzo[f]-chinoxalin-3,3-(1H,4H)-dion
   1,8 g (4,6 mmol) der Verbindung 1 g und 1,3 g (8 mmol) 2,5-Dimethoxy-tetrahydrofuran-3-yl-canbaldehyd wurden 15 Min, in 100 ml Eisessig am Rückfluß gekocht. Danach wurde im Vakuum eingeengt und der Rückstand an Kieselgel chromatographisch (Fließmittel = Toluol/Aceton/Essigsäure = 10/10/1) gereinigt. Man erhielt 2.0 g (65 %) des Produktes. Schmp. 176 - 177°C.
   ¹H-NMR (D₆-DMSO) :
   - δ =: 1,2 (3H); 4,2 (2H); 5,15 (2H); 6,8 (1H); 7,25 (1H) ; 7,5 - 7,8 (3H); 7,9 (1H), 8,0 (1H); 8,75 (1H); 9,8 (1H) und 12.5 (1H) ppm.
i) 1-Carboxymethyl-9(3-formylpyrrol-1-yl)-benzo[f]chinoxalin-2,3-(1H,4H)-dion
   1,8 g (4,6 mmol) der Verbindung 1 h wurden in 30 ml Tetrahydrofuran suspendiert und mit 0,4 g (13,8 mmol) Lithiumhydroxid, gelöst in 40 ml Wasser, versetzt. Man rührte 1,5 h. Danach wurde mit 1 M Salzsäure neutralisiert, das Tetrahydrofuran im Vakuum entfernt und der ausgefallene Niederschlag abgesaugt. Man erhielt 1,6 g (96 %) des Produktes.
   ¹H-NMR (D₆-DMSO):
   - δ =: 5,1 (2H); 6,8 (1H); 7,25 (1H); 7,5 (1H); 7,6 (1H); 7,7 (1H); 7,9 (1H); 8,0 (1H); 8,8 (1H); 9,95 (1H) und 12,5 (breit) ppm.
j) 1-Carboxymethyl-9(3-(4(1,1-diphenylmethyl)-piperazin-1-ylmethyl)pyrrol-1-yl)benzo[f]chinoxalin-2,3-(1H,4H)-dion
   0,7 g (1,9 mmol) der Verbindung 1 i, 1,0 g (3,9 mmol) 4-(1,1-Diphenylmethyl)piperazin und 0,12 mol Eisessig wurden in 75ml Dimethylformamid/Ethanol (1:4) suspendiert und bei Raumtemperatur portionsweise mit 0,12 g (1,9 mmol) Natriumcyanoborhydrid versetzt. Es wurde 16 h bei Raumtemperatur gerührt. Der Ansatz wurde im Vakuum eingeengt und zwischen Methylenchlorid und Wasser verteilt. Die organische Phase wurde getrocknet und im Vakuum eingeengt. Der Rückstand wurde in wenig Methanol gelöst und mit Ether/Pentan gefällt. Man erhielt 0,38 g (34 %) des Produktes. Schmp. > 250°C.
   ¹H-NMR (D₆-DMSO):
   - δ =: 2,2 - 2,6 (8H); 3,4 (2H); 4,6 (2H); 6,2 (1H); 6,9 (1H); 6,95 (1H); 7,1 - 7,6 (14H) und 8,8 (1H) ppm.

### Beispiel 2

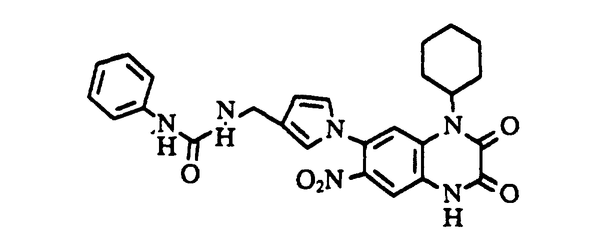

N-(1-(1-Cyclohexyl-6-nitro-chinoxalin-2,3-(1H,4H)-dion-7-yl)-pyrrol-3-yl)methyl-N'-phenylharnstoff
a) 4-Chlor-N-cyclohexyl-2-nitro-anilin
   51,5 g (0,27 Mol) 2,5-Dichlor-nitrobenzol, 22,3 g (0,27 Mol) Cyclohexylamin, 74,6 g (0,54 Mol) Kaliumkarbonat und 0,5 g 18-crown-6 wurden in 300 ml Dimethylformamid 4 h auf 100°C erwärmt. Anschließend wurde das Gemisch auf Wasser gegossen und mit Essigsäureethylester extrahiert. Die organische Phase wurde getrocknet und im Vakuum eingeengt. Der Rückstand wurde aus i-Propanol umkristallisiert, wobei 42,3 g (62 %) des Produktes anfielen. Schmp. 101 - 103°C.
   ¹H-NMR (D₆-DMSO) :
   - δ =: 1,2 - 2,0 (10H); 3,7 (1H); 7,1 (1H); 7,5 (1H); 8,0 (1H) und 8,05 (1H) ppm.
b) 2-Amino-4-chlor-N-cyclohexyl-anilin
   41,6 g (0,16 Mol) des 4-Chlor-N-cyclohexyl-2-nitroanilins wurden in 400 ml Ethanol gelöst, mit 4,2 g Raney-Nickel versetzt und bei 1 bar und 25°C hydriert. Der Ansatz wurde anschließend filtriert und das Filtrat im Vakuum eingeengt. Man erhielt 37,3 g (100 %) des Produktes.
   ¹H-NMR (D₆-DMSO):
   - δ =: 1,2 - 2,0 (10H); 3,1 (1H); 4,0 - 5,0 (3H, breit) und 6,3 - 6,6 (3H) ppm.
c) 6-Chlor-1-cyclohexyl-chinoxalin-2,3-(1H,4H)-dion
   34,5 g (0,15 Mol) des 2-Amino-4-chlor-N-cyclohexylanilins wurden in 500 ml Oxalsäurediethylester 4 h unter Rückfluß erwärmt. Nach dem Abkühlen wurde der Niederschlag abgesaugt, mit n-Pentan gewaschen und getrocknet. Man erhielt 26,8 g (63%) des Produktes. Schmp. 265 - 266°C.
   ¹H-NMR (D₆-DMSO) :
   - δ =: 1,1 - 1,9 (8H); 2,3 - 2,5 (2H); 4,4 (1H); 7,1 (2H); 7,6 (1H) und 12 (breit) ppm.
d) 6-Chlor-1-cyclohexyl-7-nitro-chinoxalin-2,3-(1H,4H)-dion
   26,3 g (94 mmol) 6-Chlor-1-cyclohexyl-chinoxalin-2,3-(1H,4H)-dion wurden in 275 ml konzentrierter Schwefelsäure gelöst und anschließend bei 0°C 9,5 g (94 mmol) Kaliumnitrat portionsweise zugefügt. Man rührte dann 30 Min. bei 0°C und 2 h bei 25°C. Das Reaktionsgemisch wurde auf Eiswasser gegossen und der Niederschlag abgesaugt. Man erhielt 29,5 g (97 %) des Produktes. Schmp. > 300°C.
   ¹H-NMR (D₆-DMSO) :
   - δ =: 1,1 - 1,9 (8H); 2,3 - 2,5 (2H); 4,4 (1H); 7,3 (1H) und ca. 12,5 (1H) ppm.
e) 7-Amino-6-chlor-1-cyclohexyl-chinoxalin-2,3-(1H,4H)-dion (e1) und 7-Amino-1-cyclohexyl-chinoxalin-2,3(1H,4H)-dion (e2)
   28,9 g (89 mmol) 6-Chlor-1-cyclohexyl-7-nitro-chinoxalin-2,3-(1H,4H)-dion wurden in 300 ml Tetrahydrofuran/Methanol/ Dimethylformamid (3:3:1) gelöst und nach Zugabe von 3 g Palladium/Kohle (10 %) hydriert. Der Ansatz wurde filtriert, die Kohle mit methanolischer Ammoniak-Lösung gewaschen und die vereinigten Filtrate im Vakuum eingeengt. Der Rückstand wurde chromatographisch an Kieselgel mit dem Fließmittel Toluol/Aceton/Eisessig (10:10:1) getrennt. Man erhielt 2,2 g (8 %) des Produktes e1 und 18,5 g (80 %) des Produktes e2.
   e1:
   ¹H-NMR (D₆-DMSO):
   - δ =: 1,1 - 2,0 (8H) ; 2,3 - 2,5 (2H); 4,4 (1H); ca. 5,2 (2H, breit), 7,0 (1H); 7,1 (1H) und ca. 11,5 (breit) ppm.
   e2:
   ¹H-NMR (D₆-DMSO):
   - δ =: 1,1 - 2,0 (8H); 2,3 - 2,5 (2H); 4,4 (1H); ca. 5,1 (2H, breit); 6,4 (1H); 6,8 (1H); 6,9 (1H) und ca. 11,5 (breit) ppm.
f) 7-Acetamido-1-cyclohexyl-chinoxalin-2,3(1H,4H)-dion
   18,3 g (70 mmol) 7-Amino-1-cyclohexyl-chinoxalin-2,3(1H,4H)-dion (Produkt e2 aus Beispiel 2) wurden in 250 ml Essigsäure gelöst, und nachdem man eine Spatelspitze 4-(N,N-Dimethylamino)-pyridin zugesetzt hatte, wurden 7,2 g (70 mmol) Essigsäureanhydrid zugetropft. Man rührte noch 30 Min. bei Raumtemperatur. Anschließend wurde der entstandene Niederschlag abgesaugt und getrocknet. Man erhielt 20,8 g (98%) des Produktes. Schmp. 227 - 230°C (Z.).
   ¹H-NMR (D₆-DMSO):
   - δ =: 1,1 - 1,9 (8H); 2,3 - 2,5 (2H); 4,5 (1H); 7,1 (1H); 7,3 (1H); 8,0 (1H); 10,0 (1H) und ca. 12,0 (breit) ppm.
g) 7-Amino-1-cyclohexyl-6-nitro-chinoxalin-2,3(1H,4H)-dion
   20,6 g (68 mmol) 7-Acetamido-1-cyclohexyl-chinoxalin-2,3-(1H,4H)-dion wurden in 250 ml konzentrierter Schwefelsäure gelöst. Anschließend wurden bei 0 - 5°C 7,2 g (71 mmol) Kaliumnitrat portionsweise zugegeben. Man rührte danach 30 Min. bei 0°C und 2 h bei Raumtemperatur. Der Ansatz wurde dann auf 1,5 1 Eiswasser gegossen und 4 h auf dem Wasserbad erwärmt. Der entstandene Niederschlag wurde abgesaugt. Das Filtrat wurde mit wenig Ammoniak-Lösung auf pH = 6 eingestellt und mit Methylenchlorid extrahiert. Die organische Phase wurde getrocknet und im Vakuum eingeengt. Dieser Rückstand und der erste Niederschlag wurden vereint. Man erhielt 13,4 g (64 %) des Produktes. Schmp. > 260°C.
   ¹H-NMR (D₆-DMSO):
   - δ =: 1,1 - 2,0 (8H); 2,3 - 2,5 (2H); 4,3 (1H); 7,1 (1H); 7,1 - 7,4 (breit, 2H); 7,7 (1H) und ca. 11,5 (1H) ppm.
h) 1-Cyclohexyl-6-nitro-7(3-trifluoracetamidomethylpyrrol -1-yl)-chinoxalin-2,3(1H,4H)-dion
   8 g (26 mmol) der Verbindung 2 g und 8,45 g (33 mmol) der Verbindung 4a wurden in 200 ml Eisessig 1 h auf 60°C erwärmt. Danach wurde alles auf Eiswasser gegossen und der Niederschlag abgesaugt. Man erhielt 11,7 g (93 %) des Produktes. Schmp. 247 - 248°C.
   ¹H-NMR (D₆-DMSO):
   - δ =: 1,0 - 3,0 (10H); 4,2 - 4,7 (3H); 6,75 (1H); 7,0 - 7,1 (2H); 7,7 (1H); 7,9 (1H); 9,9 (1H) und ca. 12,5 (1H) ppm.
i) 7-(3-Aminomethyl-pyrrol-1-yl)-1-cyclohexyl-6-nitro-chinoxalin-2,3(1H,4H)-dion
   11,1 g (23 mmol) der Verbindung 2 h wurden in 250 ml Ethanol suspendiert und mit 50 ml 2 M Natronlauge versetzt. Man rührte alles 10 Minuten bei Raumtemperatur. Danach wurde mit 2 M Salzsäure neutralisiert, die Lösung mit wäßriger Natriumhydrogenkarbonat-Lösung abgepuffert und der anfallende Niederschlag abgesaugt. Man erhielt 7,9 g (90 %) des Produktes. Schmp. > 300°C.
   ¹H-NMR (CD₃OD, NaOH):
   - δ =: 1,2 - 2,2 (10H); 3,3 (1H); 3,8 (2H); 6,25 (1H); 6,3 (1H) und 6,6 - 6,8 (3H) ppm.
j) N-(1-(1-Cyclohexyl-6-nitro-chinoxalin-2,3(1H,4H)-dion-7-yl)-pyrrol-3-yl)methyl-N'-phenylharnstoff
   1,5 g (4 mmol) des Produktes 2 i und 0,04 g (4 mmol) Triethylamin wurden in 25 ml wasserfreies Dimethylformamid gegeben und mit 0,52 g (4,4 mmol) Phenylisocyanat versetzt. Danach wurde 1 h bei 70°C gerührt. Der Ansatz wurde auf Eiswasser gegeben und mit Essigester extrahiert. Die organische Phase wurde getrocknet und im Vakuum eingeengt. Der Rückstand wurde mit heißem Isopropanol behandelt und abgesaugt. Man erhielt 1,5 g (74 %) des Produktes. Schmp. 172°C (Z.).
   ¹H-NMR (D₆-DMSO):
   - δ =: 1,1 - 1,9 (8H); 2,4 (2H); 4,15 (2H); 4,4 (1H); 6,25 (1H); 6,3 (1H), 6,9 (3H); 7,2 (2H); 7,4 (2H); 7,6 (1H); 7,8 (1H); 8,5 (1H) und 12,5 (breit) ppm.

### Beispiel 3

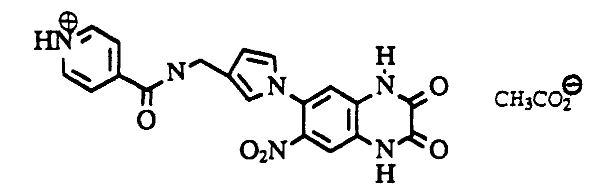

N-((1-(6-Nitrochinoxalin-2,3-(1H,4H)-dion-7-yl)-pyrrol-3-yl)-methyl)-4-carbamoylpyridiniumacetat
a) Herstellung von 6-Trifluoracetamidochinoxalin-2,3(1H,4H)-dion
   Eine Lösung von 28 g (0,16 mol) 6-Aminochinoxalin-2,3(1H,4H)-dion in 200 ml Trifluoressigsäure wurde mit 35,7 g (0,17 mol) Trifluoressigsäureanhydrid eine Stunde unter Rückfluß erhitzt. Nach Abkühlung der Reaktionsmischung wurde das angefallene Rohprodukt abfiltriert und nach den üblichen Methoden aufgearbeitet. Ausbeute: 83 %. Schmp. > 330°C.
   ¹H-NMR (D₆-DMSO):
   - δ =: 7,1 (1H); 7,3 (1H); 7,6 (1H); 11,3 (1H); 12,1 (1H) ppm.
b) Herstellung von 6-Trifluoracetamido-7-nitrochinoxalin-2,3-(1H,4H)-dion
   Eine Lösung von 39 g (0,14 mol) 6-Trifluoracetamidochinoxalin-2,3(1H,4H)-dion in 500 ml konzentrierter Schwefelsäure wurde auf 0°C abgekühlt, portionsweise mit 12,1 g (0,14 mol) Natriumnitrat versetzt und 30 Minuten bei Raumtemperatur gerührt. Anschließend wurde die Reaktionsmischung in Eiswasser gegossen, das Rohprodukt wurde abfiltriert und nach den üblichen Methoden aufgearbeitet. Ausbeute: 95 %. Schmp. >320°C.
   ¹H-NMR (D₆-DMSO):
   - δ =: 7,3 (1H); 7,8 (1H); 11,6 (1H); 12,2 (1H); 12,4 (1H) ppm.
c) Herstellung von 6-Amino-7-nitrochinoxalin-2,3(1H,4H)-dion
   Eine Lösung von 41 g (0,13 mol) 6-Trifluoracetamido-7-nitro-chinoxalin-2,3(1H,4H)-dion in 300 ml Ethanol und 700 ml 3-molare Salzsäure wurde 3 Stunden unter Rückfluß erhitzt. Nach Abkühlung der Reaktionsmischung wurde das angefallene Rohprodukt abgesaugt und mit Aceton gewaschen.
   Ausbeute: 84 %. Schmp. > 330°C.
   ¹H-NMR (D₆-DMSO):
   - δ =: 6,6 (1H), 7,2 - 7,6 (3H); 7,8 (1H); 11,7 (1H); 12,1 (1H) ppm.
d) Herstellung von N-(2,5-Dimethoxy-tetrahydrofuran-3-yl)-methyl)-isonicotinsäureamid
   3,8 g (31,0 mmol) Isonicotinsäure und 5,5 g (34,1 mmol) 3-Aminomethyl-2,5-dimethoxytetrahydrofuran (DE-OS 2,645,234) wurden in 100 ml wasserfreiem Dimethylformamid gelöst. Bei 0°C gab man eine Lösung von 7,3 ml (34,1 mmol) Diphenylphosphorsaureazid in 30 ml wasserfreiem Dimethylformamid und nach 15 Minuten noch 8,5 ml (68,24 mmol) Triethylamin hinzu. Man rührte 16 h. Danach wurde der Ansatz im Vakuum eingeengt, der Rückstand zwischen Essigester und wäßriger Natriumhydrogenkarbonat-Lösung verteilt, die organische Phase wurde abgetrennt, getrocknet und im Vakuum eingeengt. Man erhielt 5,5 g eines verunreinigten Produktes, das direkt weiter umgesetzt wurde.
e) N-((1-(6-Nitrochinoxalin-2,3-(1H,4H)-dion-7-yl)-pyrrol-3-yl)-methyl)-4-carbamoylpyridiniumacetat
   Zu 150 ml Essigsäure, die auf ca. 100°C geheizt wurden, gab man nacheinander 1,5 g (6,8 mmol) des Produktes 3 c und 1,8 g (6,8 mmol) des Produktes 3 d. Man rührte 30 Minuten bei 100°C und ließ danach abkuhlen. Der ausgefallene Niederschlag wurde abgesaugt, in Aceton aufgeschlämmt und erneut abgesaugt. Man erhielt 2,2 g (70 %) des Produktes. Schmp. 182 -.185°C (Z.).
   ¹H-NMR (D₆-DMSO) :
   - δ =: 1,9 (3H); 4,4 (2H); 6,25 (1H); 6,85 (2H); 7,1 (1H); 7,8 (3H); 8,7 (2H); 9,15 (1H); 11,9 (breit, 1H); 12,1 (1H) und 12.3 (1H) ppm.

### Beispiel 4

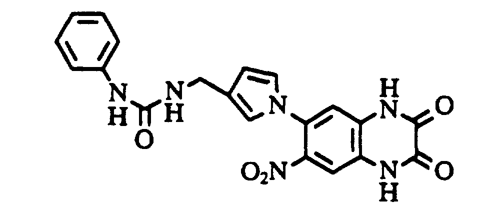

N-(1-(6-Nitro-chinoxalin-2,3-(1H,4H)-dion-7-yl)-pyrrol-3-ylmethyl)-N'-phenyl-harnstoff
a) Herstellung von N-((2,5-Dimethoxy-tetrahydrofuran-3-yl)-methyl)-trifluoressigsäureamid
   50 g (0,31 Mol) 3-Aminomethyl-2,5-dimethoxytetrahydrofuran, 31,7 g (0,31 Mol) Triethylamin und etwas 4-(N,N-Dimethylamino)pyridin wurden in 300 ml wasserfreiem Ether gelöst und bei 0 bis 5°C tropfenweise mit 65,1 g (0,31 Mol) Trifluoressigsäureanhydrid, das in 100 ml wasserfreiem Ether gelöst war, versetzt. Man rührte noch 1 h. Danach wurde der Ansatz mit Wasser gewaschen, getrocknet und im Vakuum eingeengt. Man erhielt 70,5 g des verunreinigten o.g. Produktes, das ohne weitere Reinigung weiter umgesetzt wurde.
b) Herstellung von 6-Nitro-7-(3-(trifluoracetamidomethyl)-1-pyrrolyl)-chinoxalin-2,3-(1H,4H)-dion
   4 g (18 mmol) der Verbindung 3 c und 6 g (23,3 mmol) des Rohproduktes 4 a wurden nacheinander zu 150 ml Eisessig, der auf 100°C vorgeheizt war, gegeben. Alles wurde ca. 15 bis 30 Minuten gerührt. Nach dem Abkühlung wurde die Essigsäure im Vakuum entfernt, der Rückstand in Ether aufgeschlämmt und abgesaugt. Man erhielt 6,0 g (85 %) des Produktes.
   Schmp. > 220°C (Z.).
   ¹H-NMR (D₆-DMSO):
   - δ =: 4,25 (2H); 6,2 (1H); 6,8 (2H); 7,1 (1H); 7,8 (1H) ; 9,8 (1H) und ca. 12,5 (2H) ppm.
c) Herstellung von 7-(3-Aminomethyl-pyrrol-1-yl)-6-nitrochinoxalin-2,3-(1H,4H)-dion
   20 g (50,4 mmol) der Verbindung 4 b wurden in 300 ml Tetrahydrofuran suspendiert und mit 5,4 g (22,4 mmol) Lithiumhydroxid, gelöst in 150 ml Wasser, versetzt. Es wurde 3 h bei Raumtemperatur gerührt. Anschließend wurde das Tetrahydrofuran im Vakuum entfernt, die zurückbleibende wäßrige Phase mit verdünnter Salzsäure angesäuert und danach mit wäßriger Natriumhydrogenkarbonat-Lösung abgepuffert. Der anfallende Niederschlag wurde abgesaugt, in Aceton aufgeschlämmt und erneut abgesaugt. Man erhielt 10 g (66 %) des Produktes. Schmp. > 300°C.
   ¹H-NMR (D₅-DMSO):
   - δ =: 3 - 4 (NH); 3,8 (2H); 6,25 (1H); 6,9 - 7,0 (3H); 7,75 (1H) und ca. 11,3 (breit) ppm.
d) N- (1-(6-Nitro-chinoxalin-2,3-(1H,4H)-dion-7-yl)-pyrrol-3-ylmethyl)-trifluoressigsäureamid
   1,5 g (5 mmol) der Verbindung 4 c, 0,7 g (5,9 mmol) Phenylisocyanat und eine Spatelspitze 4-(N,N-Dimethylamino)pyridin wurden bei 110 bis 120°C in 100ml wasserfreiem Dimethylformamid erwärmt, bis der Niederschlag sich aufgelöst hatte. Danach wurde alles auf Eiswasser gegossen, die erhaltene Phase mit wäßriger Natriumhydrogenkarbonat-Lösung versetzt und der anfallende Niederschlag abgesaugt. Dieser wurde zweimal mit Essigester aufgeschlämmt und abgesaugt. Man erhielt 1,3 g (62 %) des Produktes. Schmp. > 300°C.
   ¹H-NMR (D₆-DMSO):
   - δ =: 4,15 (2H); 6,1 (1H); 6,7 - 6,9 (5H); 7,2 (2H); 7,4 (1H); 7,65 (1H) und 8,9 (1H) ppm.

### Beispiel 5

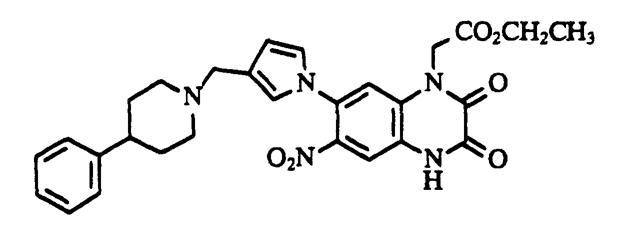

1-(Ethoxycarbonylmethyl)-6-nitro-7(3-((4-phenyl-1-piperidinyl)-methyl)-pyrrol-1-yl)-chinoxalin-2,3-(1H,4H)-dion
a) N-(4-Chlor-2-nitro-phenyl)-glycin
   26,2 g (0,137 Mol) 2,5-Dichlor-1-nitrobenzol, 20,6 g (0,274 Mol) Glycin und 18,9 g (0,137 Mol) Kaliumkarbonat wurden in 200 ml Diethylenglykol 1 h bei 120°C erwärmt. Nach dem Abkühlen wurden 100 ml Wasser zugegeben und die Lösung mit 1 M Salzsäure angesäuert. Der Niederschlag wurde abgesaugt. Man erhielt 17,1 g (54 %) des Produktes.
   ¹H-NMR (D₆-DMSO):
   - δ =: 4,2 (2H); 6,9 (1H); 7,5 (1H); 8,1 (1H); 8,4 (1H) und ca. 13 (breit) ppm.
b) N-(4-Chlor-2-nitrophenyl)aminoessigsäureethylester
   87,1 g (0,38 Mol) N-(4-Chlor-2-nitrophenyl)glycin wurden in 500 ml 10 %iger ethanolischer Schwefelsäure suspendiert und auf 80°C erwärmt. Die entstandene klare Lösung goß man auf 1,5 1 Eiswasser und neutralisierte anschließend die Lösung mit konzentrierter Ammoniak-Lösung und Natriumhydrogenkarbonat-Lösung. Der Niederschlag wurde abgesaugt. Man erhielt 84,4 g (89 %) des Produktes.
   ¹H-NMR (D₆-DMSO):
   - δ =: 1,2 (3H); 4,2 (2H); 4,3 (2H); 7,0 (1H); 7,5 (1H); 8,0 (1H) und 8,4 (1H) ppm.
c) N-(4-Chlor-2-nitrophenyl)-N-(ethoxycarbonylmethyl)-oxalsäuremonoethylesteramid
   86,1 g (0,33 Mol) der Verbindung 5 b wurden in 350 ml Pyridin gelöst und bei Raumtemperatur 67,6 g (0,495 Mol) Oxalsäuremonoethylesterchlorid zugetropft. Es wurde 16 h bei Raumtemperatur gerührt. Anschließend wurde der Ansatz auf Eiswasser gegossen und mit 4 M Salzsäure angesäuert. Der Niederschlag wurde abfiltriert, und man erhielt 120 g verunreinigtes Produkt.
   ¹H-NMR (D₆-DMSO) :
   - δ =: 1,1 - 1,3 (6H); 4,0 (2H); 4,2 (2H); 4,5 (1H); 4,6 (1H) ; 7,7 (1H); 8,0 (1H) und 8,3 (1H) ppm.
d) 6-Chlor-1-(ethoxycarbonylmethyl)-chinoxalin-2,3-(1H,4H)-dion
   101,3 g (0,28 Mol) N-(4-Chlor-2-nitrophenyl)-N-(ethoxycarbonyl-methyl)-oxalsäuremonoethylesteramid wurden in 1 1 Essigsäure gelöst und auf 80°C erwärmt. Man gab danach portionsweise 15,8g (0,28 Mol) Eisenpulver zu. Nach 2 h wurden erneut 15,8 g (0,28 Mol) Eisenpulver zugegeben. Eine halbe Stunde später wurde der Ansatz auf Eiswasser gegossen und mit 4 M Salzsäure sauer gestellt. Der Niederschlag wurde abgesaugt, und man erhielt 75,7 g (95 %) des Produktes.
   ¹H-NMR (D₆-DMSO):
   - δ =: 1,2 (3H); 4,2 (2H); 5,0 (2H); 7,2 (2H); 7,3 (1H) und 12,3 (1H) ppm.
e) 6-Chlor-1-(ethoxycarbonylmethyl)-7-nitro-chinoxalin-2,3-(1H,4H)-dion
   69,8 g (0,25 Mol) 6-Chlor-1-(ethoxycarbonylmethyl)-chinoxalin-2,3-(1H,4H)-dion wurden in 625 ml konzentrierter Schwefelsäure gelöst, und bei 0°C wurden 25 g (0,25 Mol) Kaliumnitrat portionsweise zugegeben. Danach wurde die Kühlung entfernt und der Ansatz weiterhin gerührt. Nach vollständigem Umsatz wurde alles auf Eiswasser gegossen und der Niederschlag abgesaugt. Man erhielt 77,8 g (95 %) des Produktes.
   ¹H-NMR (D₆-DMSO):
   - δ =: 1,3 (3H); 4,2 (2H); 5,0 (2H); 7,3 (1H); 8,2 (1H) und 12,5 (1H) ppm.
f) 7-Amino-1- (ethoxycarbonylmethyl)-chinoxalin-2, 3- (1H, 4H) -dion
   84.3 g (0,26 Mol) 6-Chlor-1-(ethoxycarbonylmethyl)-7-nitrochinoxalin-2,3-(1H,4H)-dion wurden in 1,5 l Isopropanol suspendiert und nacheinander 194,2 g (3,1 Mol) Ammoniumformiat, gelöst, in 500 ml Wasser, und 8,5 g Palladium/Kohle (10 %ig) zugegeben. Dann wurde 4 h auf 75°C erwärmt. Nach dem Abkuhlen wurde filtriert und der Filterkuchen dreimal mit 800 ml Dimethylformamid extrahiert. Die vereinigten Dimethylformamidphasen wurden im Vakuum eingeengt und der Rückstand mit Wasser gewaachen. Man erhielt 53,2 g (79 %) des Produktes.
   ¹H-NMR (D₆-DMSO):
   - δ =: 1,2 (3H); 4,2 (2H); 4,8 (2H); 5,2 (breit, 2H); 6,4 (1H); 6,5 (1H); 6,9 (1H) und 12 (1H) ppm.
g) 7-Acetamido-1-(ethoxycarbonylmethyl)-chinoxalin-2,3-(1H,4H)-dion
   52,95 g (0,2 Mol) 7-Amino-1-(ethoxycarbonylmethyl)-chinoxalin-2,3-(1H,4H)-dion und eine Spatelspitze 4-(N,N-Dimethylamino)-pyridin wurden in einem Gemisch aus 500 ml Eisessig und 300 ml Tetrahydrofuran suspendiert und auf 50°C erwärmt. Danach wurden 20,5 g (0,2 Mol) Essigsäureanhydrid zugetropft und alles 2 h auf 50°C erwärmt. Der Niederschlag wurde abgesaugt. Man erhielt 57,8 g (94 %) des Produktes.
   ¹H-NMR (D₆-DMSO):
   - δ =: 1,2 (3H); 2,05 (3H), 4,2 (2H); 4,9 (2H), 7,1 (1H); 7,3 (1H); 7,55 (1H); 10,0 (1H) und 12 (1H) ppm.
h) 7-Amino-1-(carboxymethyl)-6-nitro-chinoxalin-2,3-(1H,4H)-dion
   57,5 g (0,19 Mol) 7-Acetamido-1-(ethoxycarbonylmethyl)-chinoxalin-2,3-(1H,4H)-dion wurden in 575 ml konzentrierter Schwefelsäure gelöst. Die Lösung wurde auf 0°C gekühlt, und es wurden 19,0 g (0,19 Mol) Kaliumnitrat portionsweise zugegeben. Anschließend wurde die Kühlung entfernt und alles gerührt. Nach vollständigem Umsatz wurde der Ansatz in 2 1 Eiswasser gegossen und 2 h auf dem Wasserbad erhitzt. Danach wurde der pH mit wäßriger Ammoniak-Lösung auf ca. 4 - 5 eingestellt und der Niederrchlag abgesaugt. Das Filtrat wurde im Vakuum eingeengt und der Rückstand chromatographisch an Kieselgel (Laufmittel; Methanol; Tetrahydrofuran:Wasser = 5:4:1/+ 2,5 % Eisessig) gereinigt. Das so erhaltene Produkt wurde mit dem ersten Niederschlag vereinigt. Man erhielt 36,9g (70 %) Produkt.
   ¹H-NMR (D₆-DMSO) :
   - δ =: 4,5 (2H); 6,7 (1H); 7,3 (2H) und 7,7 (1H) ppm.
i) 7-Amino-1-(ethoxycarbonylmethyl)-6-nitro-chinoxalin-2,3-(1H, 4H)-dion
   30,7 g (0,11 Mol) 7-Amino-1-(carboxymethyl)-6-nitrochinoxalin-2,3-(1H,4H)-dion wurden in 500 ml 10 %iger ethanolischer Schwefelsäure suspendiert und 2 h unter Rückfluß erhitzt. Der Ansatz wurde danach auf Eiswasser gegossen und der entstandene Niederschlag abgesaugt. Man erhielt 29,3 g (87 %) des Produktes.
   ¹H-NMR (D₆-DMSO):
   - δ =: 1,2 (3H); 4,2 (2H); 4,8 (2H); 6,7 (1H); 7,0 - 7,6 (breit, 2H); 7,8 (1H) und 12,0 (1H) ppm.
j) 1-(Ethoxycarbonylmethyl)-7-(3-formyl-1-pyrrolyl)-6-nitrochinoxalin-2,3-(1H,4H)-dion
   5,0 g (1,6 mmol) des Produktes von Beispiel 5 i und 2,9 g (1,8 mmol) 2,5-Dimethoxy-tetrahydrofuran-3-yl-carbaldehyd wurden in 100 ml 80 - 90°C heiße Essigsäure eingetragen. Man rührte 30 Minuten. Anschließend wurde alles auf Eiswasser gegossen und der Niederschlag abgesaugt. Man erhielt 5,3 g (86%) des Produktes.
   ¹H-NMR (D₆-DMSO):
   - δ =: 1,2 (3H); 4,15 (3H); 4,15 (2H); 5,05 (2H); 6,7 (1H); 7,15 (H); 7,8 (1H); 8,0 (1H); 9,8 (1H) und 12,3 (breit) ppm.
k) 1-(Ethoxycarbonylmethyl)-6-nitro-7-(3((4-phenyl-1-piperidinyl)-methyl)-pyrrol-1-yl)-chinoxalin-2,3-(1H,4H)-dion
   1,5 g (3,9 mmol) der Verbindung 5 j, 1,3 g (7,8 mmol) 4-Phenylpiperidin und 0,5 ml (7,8 mmol) Essigsäure wurden in 150 ml Dimethylformamid/Ethanol (1:2) gegeben. Bei Raumtemperatur gab man portionsweise 0,24 g (3,9 mmol) Natriumcyanoborhydrid zu und rührte alles 16 h. Der Ansatz wurde im Vakuum eingeengt, der Rückstand zwischen Methylenchlorid und wäßriger Natriumhydrogencarbonat-Lösung verteilt und die organische Phase getrocknet und im Vakuum eingeengt. Man erhielt 1,1 g (55 %) des Produktes.
   ¹H-NMR (D₆-DMSO):
   - δ =: 1,2 (3H), 1,5 - 1,9 (5H); 1,9 - 2,2 (2H); 3,0 (2H); 3,4 (2H); 4,1 (2H); 5,1 (2H); 6,25 (1H); 6,85 (1H); 6,9 (1H), 7,1 - 7,5 (5H); 7,6 (1H); 7,8 (1H) und ca. 12 (breit) ppm.

### Beispiel 6

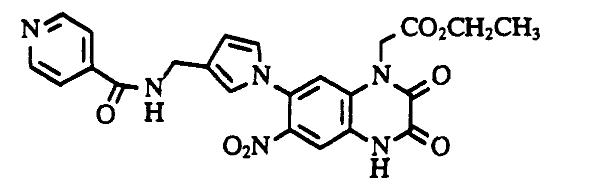

### 1-(Ethoxycarbonylmethyl)-6-nitro-7(3-(4-pyridylamido)methylpyrrol-1-yl)-chinoxalin-2,3-(1H,4H)-dion

1,1 g (3,6 mmol) der Verbindung 5 i und 1,9 g (7,2 mmol) des Rohproduktes 3 d wurden in 80 ml Eisessig 30 Minuten unter Rückfluß gekocht. Danach wurde im Vakuum eingeengt. Der Rückstand wurde danach aus Ethanol umkristallisiert. Man erhielt 1,2 g (69 %) des Produktes. Schmp. 165 - 167°C (Z.).
¹H-NMR (D₆-DMSO) :
- δ =: 1,2 (3H); 4,1 (2H); 4,4 (2H); 5,1 (2H); 6,3 (1H) ; 6,9 (2H); 7,55 (1H); 7,8 (2H); 7,85 (1H); 8,7 (2H); 9,1 (1H) und 12,5 (breit) ppm.

### Beispiel 7

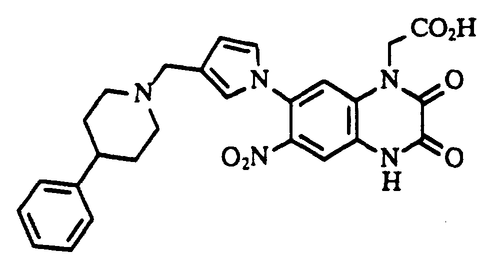

### 1-Carboxymethyl-6-nitro-7(3-((4-phenyl-1-piperidinyl)-methyl)-pyrrol-1-yl)-chinoxalin-2,3-(1H,4H)-dion

1,0 g (1,9 mmol) des Beispiels 5 wurden in 10 ml Tetrahydrofuran suspendiert und mit 0,14 g (5,6 mmol) Lithiumhydroxid, gelöst in 15 ml Wasser, versetzt. Es wurde 1 h bei Raumtemperatur gerührt. Danach wurde das Tetrahydrofuran im Vakuum entfernt und durch vorsichtigen Zusatz von 1 M Salzsäure das Produkt ausgefallt. Man erhielt 0,8 g (95 %). Schmp. > 210°C.
¹H-NMR (als NH₄*-Salz) (D₆-DMSO):
- δ =: 1,6 - 1,8 (4H); 2,0 (2H); 2,4 - 2,6 (1H); 3,0 (2H); 3,4 (2H); 4,5 (2H); 6,2 (1H); 6,75 (1H); 6,8 (1H); 6,95 (1H); 7,1 - 7,4 (5H) und 7,7 (1H) ppm.

### Beispiel 8

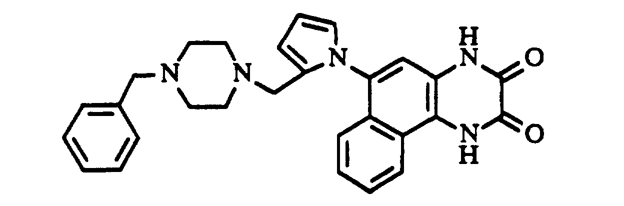

### 9-(2-(4-Benzylpiperazin-1-yl)-methylpyrrol-1-yl)-benzo[f]-chinoxalin-2,3-(1H,4H)-dion

a) 9-(2-Formyl-pyrrol-1-yl)-benzo[f]chinoxalin-2,3-(1H,4H)-dion
   7,0 g (30,8 mmol) 9-Amino-benzo[f]chinoxalin-2,3-(1H,4H)-dion und 6,4 g (30,8 mmol) 2,5-Dimethoxy-2-(1,1,-dimethoxymethyl)-tetrahydrofuran wurden in 170 ml Eisessig 30 Minuten unter Rückfluß gekocht. Der ausgefallene Niederschlag wurde abgesaugt und chromatographisch an Kieselgel (Fließmittel: Toluol/Aceton/Eisessig = 10/10/1) gereinigt. Man erhielt 2,5 g (27 %) des Produktes. Schmp. > 250°C.
   ¹H-NMR (D₆-DMSO):
   - δ =: 6,6 (1H); 7,0 (1H); 7,3 - 7,4 (2H); 7,45 (2H); 7,6 (1H); 8,65 (1H); 9,4 (1H) und ca. 12,3 (breit) ppm.
b) 9-(2-(4-Benzylpiperazin-1-yl)-methylpyrrol-1-yl)-benzo[f]-chinoxalin-2,3-(1H,4H)-dion
   1,1 g (3,6 mmol) der Verbindung 8 a, 1,3 g (7,2 mmol) N-Benzylpiperazin und 0,45 ml (7,2 mmol) Eisessig wurden in 150 ml Dimethylformamid / Ethanol = 1/2) gegeben und bei Raumtemperatur portionsweise mit 0,23 g (3,6 mmol) Natriumcyanoborhydrid versetzt. Man rührte 16 h. Anschließend wurde der Ansatz im Vakuum eingeengt, der Rückstand mit siedendem Ethanol behandelt und abgesaugt. Man erhielt 0,8 g (51 %) des Produktes. Schmp. > 250°C.
   ¹H-NMR (D₆-DMSO) :
   - δ =: 1,9 - 2,3 (8H); 3,0 (1H); 3,25 (1H); ca. 3,4 (2H); 6,2 (2H); 6,9 (1H); 7,05 (1H); 7,2 - 7,35 (5H); 7,35 (1H); 7,45 (1H); 7,6 (1H); 8,65 (1H) und 12,3 (breit) ppm.

### Beispiel 9

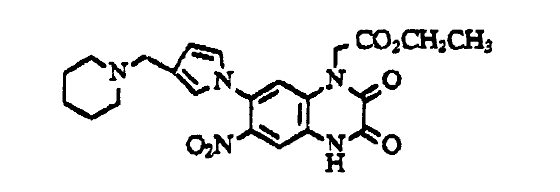

### 1-Ethoxycarbonylmethyl-6-nitro-7-(3-(1-piperidinyl)methyl-pyrrol-1-yl)chinoxalin-2,3-(1H,4H)-dion

2,0 g (52 mmol) der Verbindung 5 j und 0,9 g (10,4 mmol) Piperidin wurden analog der Vorschrift 5 k umgesetzt. Man erhielt 1,6 g (69%) des Produktes. Schmp. > 130°C (Z.).
¹H-NMR (D₆-DMSO):
- δ =: 1,2 (3H); 1,4 - 1,6 (6H); 2,3 - 2,5 (4H); 3,4 (2H); 4,2 (2H); 5,1 (2H); 6,2 (1H); 6,8 (1H); 6,9 (1H); 7,5 (1H) und 7,85 (1H) ppm.

### Beispiel 10

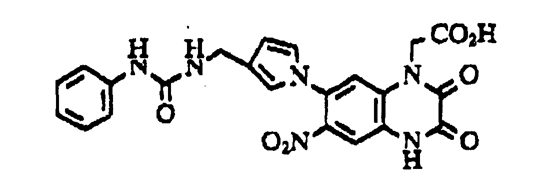

### N-(1-(1-Carboxymethyl-6-nitro-chinoxalin-2,3-(1H,4H)-dion-7-yl)-pyrrol-3-yl)methyl-N'-phenyl-harnstoff

a) 1-Ethoxycarbonylmethyl-6-nitro-7-(3-(trifluormethyl-amido-methyl)-pyrrol-1-yl)-chinoxalin-2,3-(1H,4H)-dion
   1,5 g (4,9 mmol) der Verbindung 5 i und 2.5 g (9,7 mmol) der Verbindung 4 a wurden nacheinander zu 100 ml Eisessig, die auf 100°C erwärmt wurden, gegeben. Danach rührte man noch ca. 10 Minuten. Anschließend wurde der Ansatz auf Eiswasser gegossen und die Wasserphase mit Essigester extrahiert. Die organische Phase wurde mit wäßriger Natriumhydrogenkarbonat-Lösung gewaschen, getrocknet und im Vakuum eingeengt. Der Rückstand wurde in Ethanol aufgeschlämmt und abgesaugt. Man erhielt 1,7 g (73 %) des Produktes, Schmp. 223°C.
   ¹H-NMR (D₆-DMSO) :
   - δ =: 1,2 (3H) ; 4,15 (2H); 4,3 (2H); 5,05 (2H) ; 6,2 (1H) ; 6,9 (2H) ; 7,55 (1H); 7,9 (1H) ; 9,8 (1H) und 12,5 (breit) ppm
b) 7-(3-(Aminomethyl)-pyrrol-1-yl)-1-carboxymethyl-6-nitrochinoxalin-2,3-(1H,4H)-dion
   0,9 g (1,9 mmol) der Verbindung 10 a wurden in 20 ml Tetrahydrofuran gelöst und mit 0,18 g (7,5 mmol) Lithiumhydroxid, gelöst in 25 ml Wasser, versetzt. Man rührte 2 h bei Raumtemperatur. Anschließend wurde mit 1 M Salzsäure neutralisiert, der ausgefallene Niederschlag abgesaugt, mit Methanol aufgeschlämmt und erneut abgesaugt. Man erhielt 0,55 g (100 %) des Produktes. Schmp. > 270°C.
   ¹H-NNR (als K⁺-Salz) (D₂O) :
   - δ =: 4,0 (2H); 4,8 (2H) ; 6,4 (1H) ; 6,9 (1H) ; 7,0 (1H) ; 7,1 (1H) und 7,9 (1H) ppm.
c) N-(1-(1-Carboxymethyl-6-nitro-chinoxalin-2,3-(1H,4H)-dion-7-yl)pyrrol-3-yl)methyl-N'-phenylharnstoff
   1,2 g (3,3 mmol) der Verbindung 10 b, 1 ml (6,7 mmol) Triethylamin und 0,44 g (3,7 mmol) Phenylisocyanat wurden in 40 ml wasserfreiem Dimethylformamid 30 Minuten auf 125°C erwärmt. Anschließend wurde der Ansatz im Vakuum eingeengt und der Rückstand zwischen Essigester und verdünnter Salzsäure verteilt. Die organische Phase wurde abgetrennt, getrocknet und im vakuum eingeengt. Der Rückstand wurde aus i-Propanol umkristallisiert. Man erhielt 0,9 g (57 %) des Produktes. Schmp. > 210°C (Z.).
   ¹H-NMR (D₆-OMSO) :
   - δ =: 4,15 (2H); 5,0 (2H) ; 6,25 (1H); 6,3 (1H, NH); 6,9 (3H) ; 7,2 (2H); 7.4 (2H) ; 7,5 (1H); 7,9 (1H) ; 8,4 (1H, NH) und 12,5 (breit) ppm.

### Beispiel 11

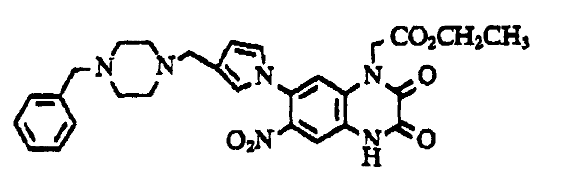

### 7-(3-((4-Benzylpiperazin-1-yl)-methyl)-pyrrol-1-yl)-1-ethoxycarbonylmethyl-6-nitro-chinoxalin-2,3-(1H,4H)-dion

1,9 g (4,9 mmol) der Verbindung 5 j und 1,7 g (9,8 mmol) 4-Benzylpiperazin wurden analog der Vorschrift 5 k umgesetzt. Man erhielt 1,3 g (49 %) des Produktes.
¹H-NMR (D₆-DMSO):
- δ =: 1,2 (3H) ; 2,3 - 2,6 (8H); 3,5 (2H); 4,15 (2H); 5,1 (2H); 6,2 (1H); 6,8 (1H); 6,9 (1H); 7,2 - 7,4 (5H); 7,6 (1H) und 7,9 (1H) ppm.

### Beispiel 12

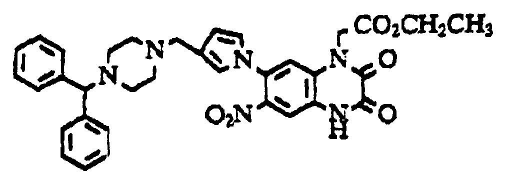

### 7-(3-((4-(1, 1-Diphenylmethyl)-piperazin-1-yl)-methyl)-pyrrol-1-yl)-1-ethoxycarbonylmethyl-6-nitro-chinoxalin-2,3-(1H,4H)-dion

2,0 g (5,2 nmol) der Verbindung 5 j und 2,6 g 4-(Diphenylmethyl)-piperazin wurden analog der vorschrift 5 k umgesetzt. Man erhielt 1,9 g (60 %) des Produktes.
¹H-NMR. (D₆-DMSO) :
- δ =: 1,2 (3H); 2,2 - 2,5 (8H); ca. 3,3 (2H); 4,1 (2H); 4,25 (1H); 5,05 (2H); 6,2 (1H); 6,7 (1H); 6,8 (1H); 7,1 - 7,5 (11H) und 7,75 (1H) ppm.

### Beispiel 13

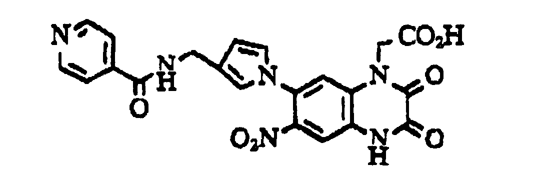

### 1- (Carboxymethyl)-6-nitro-7-(3-(4-pyridylamido)methyl-pyrrol-1-yl)chinoxalin-2,3-(1H,4H)-dion

1,2 g (2,4 mmol) des Beispiels 6 wurden in 30 ml Tetrahydrofuran suspendiert und mit 0,17 g (7,3 mmol), gelöst in 30 ml Wasser, versetzt. Man rührte 1 h bei Raumtemperatur. Anschließend wurde das Tetrahydrofuran im Vakuum entfernt, die wäßrige Phase mit 1 M Salzsäure neutralisiert und der ausgefallene Niederschlag abgesaugt. Man erhielt 1,0 g (91 %) des Produktes. Schmp. > 250°C.
¹H-NMR (K⁺-Salz in D₂O):
- δ =: 4,4 (2H); 4,7 (2H); 6,3 (1H); 6,8 (2H); 7,0 (1H); 7,6 (2H) ; 7,8 (1H) und 8,6 (2H) ppm.

### Beispiel 14

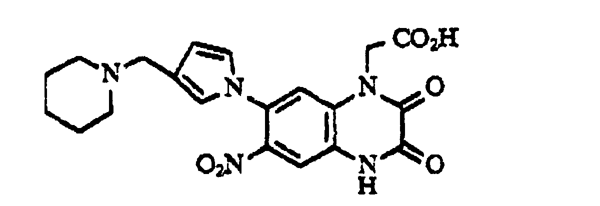

### 1-Carboxymethyl-6-nitro-7-(3-((1-piperidinyl)-methyl)pyrrol-1-yl)chinoxalin-2,3-(1H,4H)-dion

1,2 g (2,6 mmol) des Beispiels 9 und 0,2 g (7,9 mmol) Lithiumhydroxid wurden analog Beispiel 7 umgesetzt. Man erhielt 0,9 g (81%) des Produktes. Schmp. > 250°C.
¹H-NMR (K⁺-Salz in D₂O) :
- δ =: 1,4 - 1,9 (6H); 2,7 - 3,0 4H); 3,8 (2H); 4,4 (2H); 6,4 (1H); 6,9 (1H); 7,0 (1H); 7,1 (1H) und 7,9 (1H) ppm.

### Beispiel 15

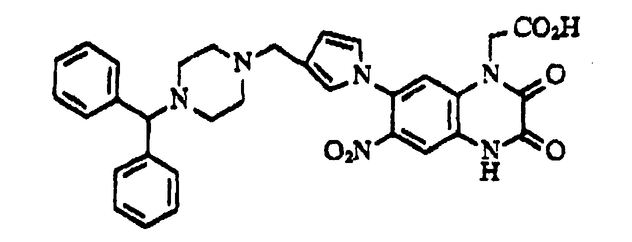

### 1-Carboxymethyl-7-(3-(4-(1,1-diphenylmethyl)-piperazin-1-yl)-methyl)-pyrrol-1-yl)-6-nitro-chinoxalin-2,3-(1H,4H)-dion

Analog zu Beispiel 7 wurden 1,5 g (2,4 mmol) Beispiel 12 und 0,17g (7,2 mmol) Lithiumhydroxid umgesetzt. Man erhielt 1,2 g (84%) des Produktes. Schmp. > 245°C.
¹H-NMR (D₆-DMSO) :
- δ =: 2,2 - 2,5 (4H) ; 3,3 - 3,7 (6H); 4,25 (1H) ; 4,6 (2H); 6,1 (1H); 6,8 (2H); 7,1 - 7.5 (11H) und 7,8 (1H) ppm.

### Beispiel 16

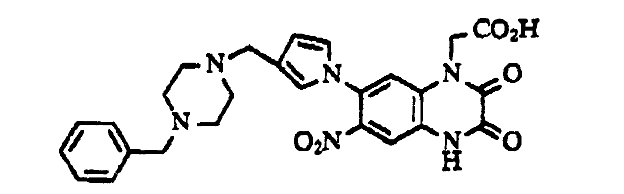

### 7-(3-(4-Benzylpiperazin-1-yl)-methyl)-pyrrol-1-yl)-1-carboxy-methyl-6-nitro-chinoxalin-2,3- (1H,4H)-dion

1,3 g (3,4 mmol) des Produktes 5 j und 1,2 g (6,7 mmol) N-Benzylpiperazin wurden analog Beispiel 1 j umgesetzt. Man erhielt 1,4 g (80 %) des Produktes. Schmp. > 230°C.
¹H-NMR (DMSO, KOH);
- δ =: 2,2 - 2,5 (4H); 3,3 - 3.6 (8H); 4,4 (2H); 6,1 (1H); 6,65 (1H); 6,7 (1H); 6,8 (1H); 7,2 - 7,4 (5H) und 7,5 (1H) ppm.

### Beispiel 17

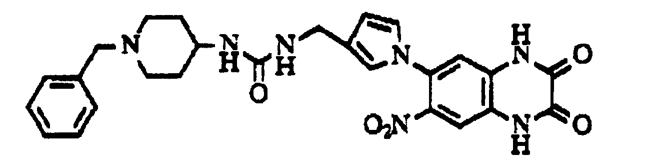

### N-(1-Benzyl-piperidin-4-yl)-N'-(1-(6-nitro-chinoxalin-2,3-(1H,4H)-dion-7-yl)-pyrrol-3-yl)-methyl-harnstoff

1,5 g (5 mmol) der Verbindung 4 c, 1,3 g (8 mmol) Carbonyldiimidazol und 1,5 g (8 mmol) 4-Amino-1-benzylpiperidin wurden analog Beispiel 18 in Dimethylformamid umgesetzt. Der erhaltene Niederschlag wurde zweimal in Ethanol aufgeschlämmt und abgesaugt. Man erhielt 1,2 g (46 %) des Produktes. Schmp. 234 - 237°C.
¹H-NMR (D₆-DMSO) ;
- δ =: 1,3 (2H); 1,7 (2H); 2,0 (2H); 2,7 (2H): 3,4 (3H); 4,05 (2H); 5,8 (1H) ; 5,9 (1H); 6,2 (1H); 6,75 (1H); 6,8 (1H); 7,05 (1H) ; 7,2 - 7,4 (5H) und 7,8 (1H) ppm.

### Beispiel 18

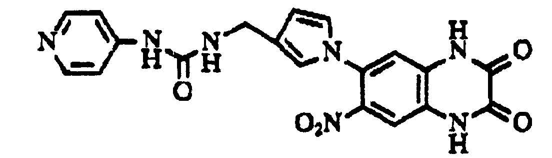

### N-(1-(6-Nitro-chinoxalin-2,3-(1H,4H)-dion-7-yl)-pyrrol-3-yl)-methyl-N'-(4-pyridyl)-harnstoff

0,7 g (7,4 mmol) 4-Aminopyridin und eine Spatelspitze 4-(N,N-Dimethylamino)pyridin wurden unter Schutzgas in 100 ml wasserfreiem Dimethylformamid gelöst und bei Raumtemperatur mit 1,3 g (8 mmol) Carbonyldiimidazol versetzt. Man rührte für 60 Minuten bei Raumtemperatur und danach noch 30 Minuten bei 50°C, Anschließend wurden 1,5 g (5 mmol) der Verbindung 4 c zugegeben und alles auf 80 - 90°C erwärmt, wobei sich der Niederschlag langsam auflöst. Die Reaktionslösung wurde auf Eiswasser gegossen, mit wäßriger Natriumhydrogenkarbonat versetzt und der anfallende Niederschlag abgesaugt. Dieser wurde in Tetrahydrofuran/Aceton aufgeschlämmt und erneut abgesaugt. Man erhielt 1,5 g (71 %) des Produktes, Schmp. 238 - 242°C (Z.).
¹H-NMR (D₆-DMSO) ;
- δ =: 4,2 (2H); 6,2 (1H); 6,8 (2H) ; 7,0 (1H); 7,4 (1H) ; 7,75 (1H); 8,25 (1H); 8,4 (1H); 9,1 (1H) und 10,1 (breit) ppm.

### Beispiel 19

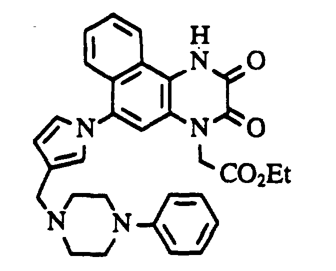

### 1-Ethoxycarbonylmethyl-9-(3-(4-phenylpiperazin-1-yl)-methylpyrrol-1-yl)-benzo[f]chinoxalin-2,3-(1H,4H)-dion

2,0 g (5,1 mmol) des Produktes 1 h und 1,7 g (10,2 mmol) N-Phenylpiperazin wurden analog Vorschrift 1 j umgesetzt. Man erhielt 1,5 g (59 %) des Produktes. Schmp. > 140°C (Z.).
¹H-NMR (D₆-DMSO):
- δ =: 1,2 (3H); 2,6 (4H); 3,2 (5H); 3,5 (1H); 4,2 (2H); 5,1 (2H); 6,3 (1H); 6,8 (1H); 6,9 - 7,0 (4H); 7,1 - 7,3 (2H); 7,4 - 7,8 (4H); 8,7 - 8,8 (1H) und ca. 12,3 (breit) ppm.

### Beispiel 20

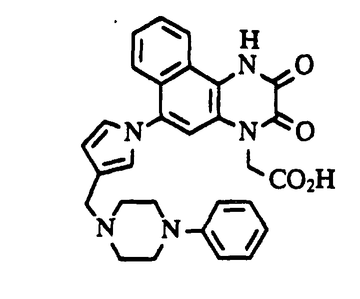

### 1-Carboxymethyl-9-(3-(4-phenylpiperazin-1-yl)-methyl-pyrrol-1-yl)-benzo[f]chinoxalin-2,3-(1H,4H)-dion

1,3 g (2,4 mmol) Beispiel 19 und 0,12 g (4,9 mmol) Lithiumhydroxid wurden analog zum Beispiel 7 umgesetzt. Man erhielt 0,9 g (76 %) des Produktes. Schmp. > 250°C (Z.).
¹H-NMR (CD₃COOD):
- δ =: 3,3 (4H); 3,8 (4H); 4,4 (2H); 5,2 (2H); 6,55 (1H); 6,95 (1H); 7,0 - 7,2 (3H); 7,3 (3H); 7,6 (2H); 7,8 (2H) und 8,5 (1H) ppm.

### Beispiel 21

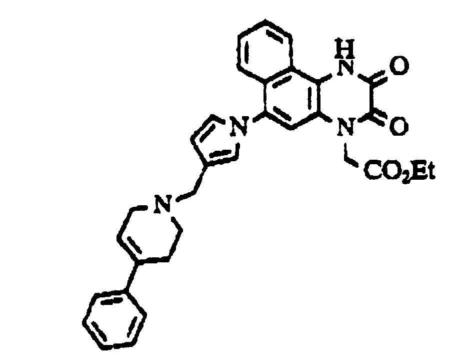

### 1-Ethoxycarbonylmethyl-9-(3-(4-phenyl-1,2,5,6-tetrahydropyridin-1-yl)methyl-pyrrol-1-yl)-benzo[f]-chinoxalin-2,3-(1H,4H)-dion

2 g (5,1 mmol) des Produktes 1 h und 2 g (10,2 mmol) 4-Phenyl-1,2,5,6-tetrahydropyridin wurden analog dem Beispiel 1 j umgesetzt. Man erhielt 1,4 g (53 %) des Produktes. Schmp. > 150°C (Z.).
¹H-NMR (D₆-DMSO) :
- δ =: 1,2 (3H), 2,55 (2H); 2,8 (2H); 3,2 (2H); 3,6 (2H); 4,2 (2H); 5,2 (2H); 6,2 (1H); 6,3 (1H); 7,05 (2H); 7,2 - 7,5 (5H); 7,6 (2H); 7,7 (2H); 8,8 (1H) und ca. 12 (breit) ppm.

### Beispiel 22

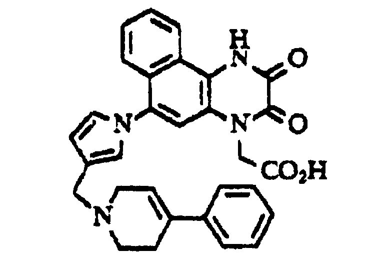

### 1-Carboxymethyl-9-(3-(4-phenyl-1,2,5,6-tetrahydropyridin-1-yl)-methyl-pyrrol-1-yl)-benzo[f]chinoxalin-2,3-(1H,4H)-dion

1,2 g (2,3 mmol) des Beispiel 21 und 0,11 g (4,6 mmol) Lithiumhydroxid wurden analog Beispiel 7 umgesetzt. Man erhielt 0,9 g (79 %) des Produktes. Schmp. > 250°C.
¹H-NMR (CD₃COOD):
- δ =: 2,8 (1H); 3,0 (1H); 3,35 (1H); 3,8 - 4,0 (2H); 4,2 (1H); 4,45 (2H); 5,2 (2H); 6,1 (1H); 6,5 (1H); 7,1 (1H); 7,2 - 7,4 (4H); 7.45 (2H); 7,6 (2H); 7,75 (2H) und 8,45 (1H) ppm.

### Beispiel 23

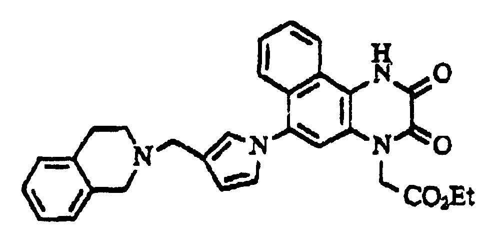

### 1-Ethoxycarbonylmethyl-9-(3-(1,2,3,4-tetrahydroisochinolin-2-yl)-methyl-pyrrol-1-yl)-benzo[f]chinoxalin-2,3-(1H,4H)-dion

2,0 g (5,1 mmol) des Produktes 1 h und 1,4 g (10,2 mmol) 1,2,3,4-Tetrahydroisochinolin wurden analog Vorschrift 1 j umgesetzt. Man erhielt 1,8 g (70 %) des Produktes.
Schmp. > 140°C (Z.).
¹H-NMR (D₆-DMSO):
- δ =: 1,2 (3H); 2,6 - 3,0 (4H); 3,6 (3H); 4,0 - 4,3 (3H); 5,15 (2H); 6,3 (1H); 6,9 - 7,2 (6H); 7,4 - 7,8 (4H); 7,75 (1H) und ca. 11,8 (breit) ppm.

### Beispiel 24

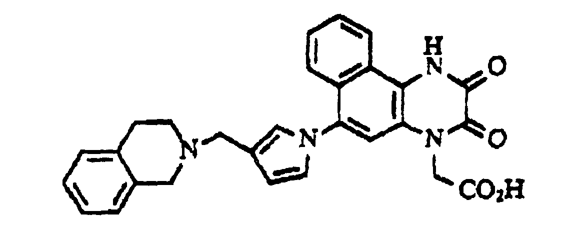

### 1-Carboxymethyl-9-(3-(1,2,3,4-tetrahydroisochinolin-2-yl)-methylpyrrol-1-yl)-benzo[f]chinoxalin-2,3-(1H,4H)-dion

1,6 g (3,2 mmol) des Beispiels 23 und 0,15 g (6,3 mmol) Lithiumhydroxid wurden analog zum Beispiel 7 umgesetzt. Man erhielt 1,3 g (87 %) des Produktes. Schmp. > 250°C.
¹H-NMR (CD₃COOD);
- δ =: 3,1 (1H) ; 3,3 - 3,5 (2H); 4,0 (1H) ; 4,35 (1H) ; 4,5 (2H); 4,7 (1H); 5,2 (2H) ; 6,6 (1H) ; 7,1 (1H) ; 7,15 - 7,4 (5H) ; 7,6 (2H) ; 7,8 (2H) und 8,5 (1H) ppm.

### Beispiel 25

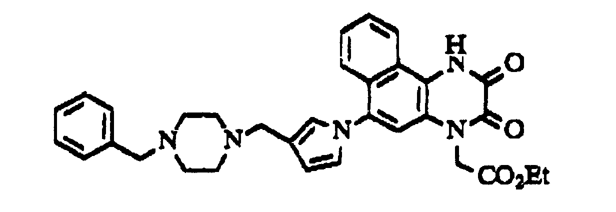

### 9-(3-(4-Benzyl-piperazin-1-yl)-methyl-pyrrol-1-yl)-1-ethoxycarbonylmethyl-benzo[f]chinoxalin-2,3-(1H,4H)-dion

6,0 g (15,3 mmol) des Produktes 1 h und 5,4 g (30,7 mmol) 4-Benzylpiperazin wurden analog Vorschrift 1 j umgesetzt. Man erhielt 4,5 g (54 %) des Produktes. Schmp. > 220°C (Z.).
¹H-NMR als Dihydrochlorid (D₆-DMSO) :
- δ =: 1,2 (3H); 3,2 - 3,8 (10H); 4,15 (2H); 4,3 (2H); 5,2 (2H); 6,6 (1H); 7,15 (1H); 7,3 (1H); 7,4 - 7,8 (9H); 8.75 (1H) und 12,5 (1H) ppm.

### Beispiel 26

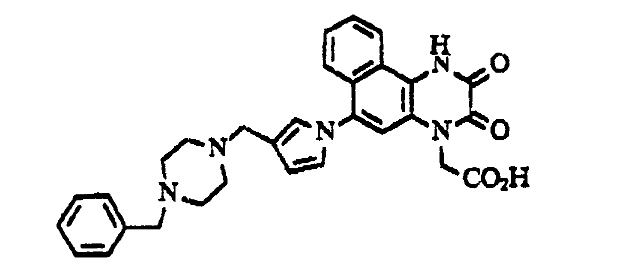

### 9-(3-(4-Benzyl-piperazin-1-yl)-methyl-pyrrol-1-yl)-1-carboxymethyl-benzo[f]chinoxalin-2,3-(1H,4H)-dion

4,5 g (8,2 mmol) des Beispiels 25 und 0,4 g (15,3 mmol) Lithiumhydroxid wurden analog Beispiel 7 umgesetzt. Man erhielt 3,5 g (84%) des Produktes. Schmp. > 230°C.
¹H-NMR als Kaliumsalz (D₆-DMSO):
- δ =: 2,3 - 2,5 (4H); 3,2 - 3,6 (8H); 4,5 (2H); 6,2 (1H); 6,9 (1H); 6,95 (1H); 7,2 - 7,6 (9H) und 7,85 (1H) ppm.

### Beispiel 27

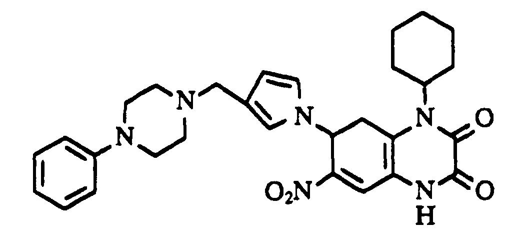

a) 1-Cyclohexyl-6-(3-formyl-1-pyrrolyl)-7-nitrochinoxalin-2,3-(1H,4H)-dion
   4 g (13 mmol) des Produktes 2 g, 2,3 g (14 mmol) 2,5-Dimethoxytetrahydrofuran-3-yl-carbaldehyd und eine Spatelspitze 4-Toluolsulfonsäure wurden in 200 ml Dimethylformamid/Toluol (1:1) am Wasserabscheider unter Rückfluß erhitzt. Nach vollständigem Umsatz (DC-Kontrolle) wurde im Vakuum eingeengt und der Rückstand zwischen Essigsäureethylester und Wasser verteilt. Die organische Phase wurde getrocknet und im Vakuum eingeengt. Dieser Rückstand wurde chromatographisch an Kieselgel (Fließmittel: Toluol/Aceton/ Eisessig = 40/20/1) gereinigt. Man erhielt 1,1g (22 %) des Produktes. Schmp. 176°C (Z.).
   ¹H-NMR (D₆-DMSO):
   - δ =: 1,1 - 2,5 (10H); 4,5 (1H); 6,7 (1H); 7,2 (1H); 7,25 (1H); 8,3 (1H); 9,8 (1H) und 12,5 (1H) ppm.
b) 1-Cyclohexyl-6-nitro-7-(3-(4-phenyl-1-piperazinyl)methylpyrrol-1-yl)-chinoxalin-2,3-(1H,4H)-dion
   2,0 g (5,2 mmol) des Produktes 28 a und 1,7 g (10,5 mmol) N-Phenylpiperazin wurden analog der Verbindung 1 j umgesetzt. Man erhielt 2,3 g (84 %) des Produktes. Schmp. 185°C (Z.).

### Beispiel 28

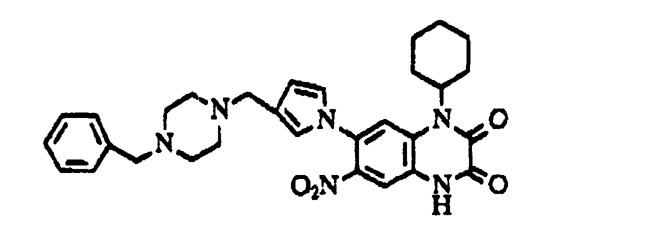

### 7-(3-(4-Benzyl-1-piperazinyl)methyl-pyrrol-1-yl)-1-cyclohexyl-6-nitro-chinoxalin-2,3-(1H, 4H)-dion

2,0 g (5,2 mmol) des Produktes 27 a und 1,8 g (10,5 mmol) N-Benzylpiperazin wurden analog dem Produkt 1 j umgesetzt. Man erhielt 2,1 g (73 %) des Produktes, Schmp. 185°C (Z.).

### Beispiel 29

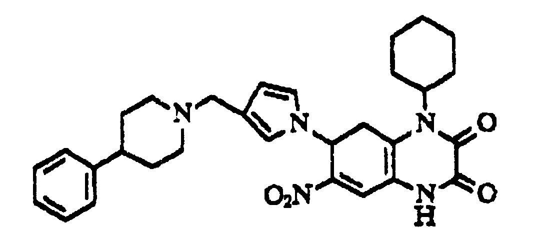

1-Cyclohexyl-6-nitro-7-(3-(4-phenyl-1-piperidinyl)methyl-pyrrol-1-yl)-chinoxalin-2,3-(1H,4H)-dion

2,0 g (5,2 mmol) des Produktes 27 a und 1,7 g (10,5 mmol) 4-Phenylpiperidin wurden analog dem Produkt 1 j umgesetzt. Man erhielt 2,4 g (86 %) des Produktes. Schmp. > 200°C (Z.).

### Beispiel 30

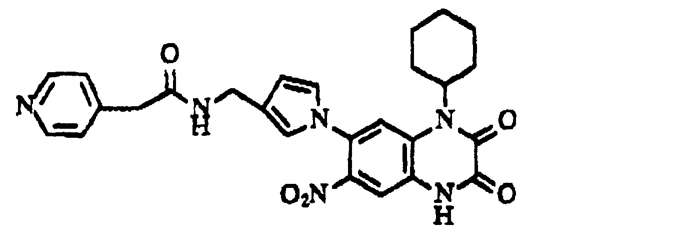

### 1-Cyclohexyl-6-nitro-7-3-((4-pyridylmethyl)amido)methyl-pyrrol-1-yl)-chinoxalin-2,3-(1H,4H)-dion

1,8 g (13,4 mmol) 4-Pyridylessigsäure wurden in 100 ml wasserfreiem Dimethylformamid gelost und mit 2,4 g (14,7 mmol) Carbonyldiimidazol versetzt. Man rührte 30 Minuten bei Raumtemperatur und anschließend weitere 30 Minuten bei 50°C. Danach wurden 4,3 g (11,3 mmol) des Produktes 2 i zugegeben und alles 1 h bei 80°C gerührt. Der Ansatz wurde auf Eiswasser gegossen und die entstandene wäßrige Phase mit Natriumhydrogencarbonat auf pH 9 eingestellt. Der Niederschlag wurde abgesaugt. Man erhielt 4,8 g (86 %) des Produktes. Schmp. > 200°C.
¹H-NMR (D₆-DMSO);
- δ =: 1,1 - 1,9 (8H) ; 2,2 - 2,4 (2H) ; 3,5 (2H) ; 4,1 (2H) ; 4,4 (1H) ; 6,1 (1H) ; 6,7 - 7,0 (2H) ; 7,2 (2H); 7,5 (1H) ; 7,B (1H) und 8.4 (3H) ppm.

### Beispiel 31

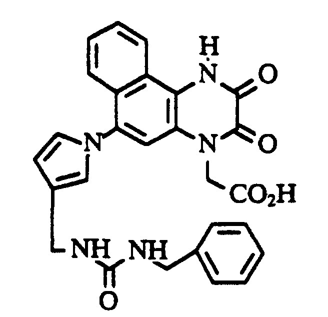

### N-(1-(1-carboxymethyl-benzo[f]chinoxalin-2,3-(1H,4H)-dion-9-yl)-pyrrol-3-yl)methyl-N'-phenyl-harnstoff

a) 1-Ethoxycarbonylmethyl-9-(3-(trifluormethylamidomethyl)-pyrrol-1-yl)benzo[f]chinoxalin-2,3-(1H,4H)-dion
   5,0 g (16 mmol) 9-Amino-1-ethoxycarbonylmethylbenzo[f]-chinoxalin-2,3-(1H,4H)-dion (Verbindung 1 g) und 4,1 g (16 mmol) der Verbindung 3 d wurden 20 Minuten in 170ml Eisessig gekocht. Nach dem Abkühlen wurde der Niederschlag abgesaugt. Man erhielt 6,2 g (80 %) des Produktes.
   Schmp. 246 - 247°C.
   ¹H-NMR (D₆-DMSO):
   - δ =: 1,2 (3H); 4,2 (2H); 4,3 (2H); 5,2 (2H); 6,3 (1H); 7,0 (2H); 7,5 - 7,8 (4H); 8,7 (1H); 9,8 (1H) und 12,5 (1H) ppm.
b) 9-(3-Aminomethyl-1-pyrrolyl)-1-carboxymethylbenzo[f]-chinoxalin-2,3-(1H,4H)-dion
   Zu 6,0 g (12,3 mmol) des Produktes 31 a wurden 160 ml. Tetrahydrofuran gegeben und mit 0,88 g (36,9 mmol) Lithiumhydroxid, gelöst in 100 ml Wasser, versetzt. Man rührte 90 Minuten bei Raumtemperatur. Danach wurde das Tetrahydrofuran im Vakuum entfernt und die erhaltene wäßrige Phase mit 1 M Salzsäure angesäuert. Der anfallende Niederschlag wurde abgesaugt. Man erhielt 4,0 g (90 %) des Produktes.
   ¹H-NMR (CD₃COOD, D₂O) :
   - δ =: 4,3 (2H); 5,2 (2H); 6,55 (1H); 7,1 (1H); 7,25 (1H); 7,5 (1H); 7,6 - 8,0 (3H) und 8,5 (1H) ppm.
c) N-(1-(1-carboxymethyl-benzo[f]chinoxalin-2,3-(1H,4H)-dion-9-yl)pyrrol-3-yl)-methyl-N'-phenylharnstoff
   0,9 g (2,5 mmol) des Produktes 31 b und 0,3 g (2,6 mmol) Phenylisocyanat wurden in 20 ml wasserfreiem Dimethylformamid 30 Minuten bei 120°C gerührt. Danach wurde im Vakuum eingeengt und der Rückstand aus Ethanol kristallisiert. Man erhielt 1,1 g (93 %) des Produktes. Schmp. > 230°C.
   ¹H-NMR (D₆-DMSO):
   - δ =: 4,2 (2H); 5,0 (2H); 6,3 (2H); 6,9 (1H); 7,0 (2H); 7,15 (2H); 7,35 (2H); 7,5 - 7,8 (4H); 8,4 (1H); 8,7 (1H) und 12,5 (1H) ppm.

### Beispiel 32

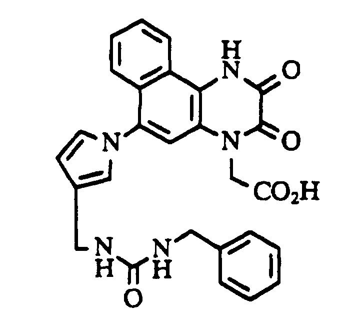

### N'-Benzyl-N-(1-(1-carboxymethyl-benzo[f]chinoxalin-2,3-(1H,4H)-dion-9-yl)pyrrol-3-yl)methyl-harnstoff

0,9 g (2,5 mmol) des Produktes 31 b und 0,37 g (2,7 mmol) Benzylisocyanat wurden 20 Minuten in 30 ml Dimethylformamid auf 120°C erhitzt. Danach wurde der Ansatz im Vakuum eingeengt und der Rückstand mit Ethanol kristallisiert. Man erhielt 1,1 g (92 %) des Produktes. Schmp. > 210°C (Z.).
¹H-NMR (D₆-DMSO):
- δ =: 4,1 - 4,3 (4H); 5,1 (2H); 5,2 (1H); 6,3 (1H); 6,35 (1H); 6,95 (1H); 7,0 (1H); 7,1 - 7.3 (5H); 7,6 (3H); 7,7 (1H); 8,7 (1H); 12,5 (1H) und ca. 13,5 (breit) ppm.

### Beispiel 33

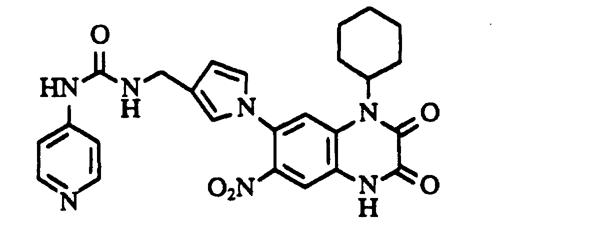

### N-(1-(1-Cyclohexyl-6-nitro-chinoxalin-2,3-(1H,4H)-dion-7-yl)-pyrrol-3-yl)methyl-N'-(4-pyridyl)-harnstoff

2,0 g (5,2 mmol) des Produktes 2 i und 0,5 g (5,2 mmol) 4-Aminopyridin wurden analog Beispiel 18 umgesetzt. Man erhielt 1,4 g (52 %) des Produktes. Schmp. > 200°C.
¹H-NMR (CD₃COOD):
- δ =: 1,3 (1H); 1,45 (2H); 1,7 (1H); 1,8 - 2,0 (4H); 2,5 - 2,7 (2H); 4,5 (3H); 6,4 (1H); 6,8 (1H); 6,9 (1H); 7,4 (1H); 7,65 (1H); 7,85 (1H); 7,90 (1H) und 9,1 (1H) ppm.

### Beispiel 34

### N-(1-(1-Cyclohexyl-6-nitro-chinoxalin-2,3-(1H,4H)-dion-7-yl)-2-pyrrolyl)methyl-N'-phenylharnstoff

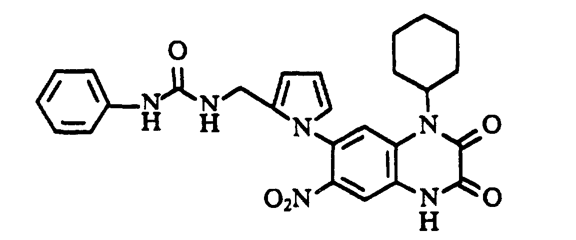

a) (N-((2,5-Dimethoxy-tetrahydrofuran-2-yl)methyl)-trifluoressigsäureamid
   25,0 g (155 mMol) 2-Aminomethyl-2,5-dimethoxy-tetrahydrofuran, 15,7 g (155 mMol) Triethylamin und 1 Spatelspitze 4-(N,N-Dimethylamino)pyridin wurden in 200 ml Ether gelöst. Bei 0 bis 5°C wurden 32,6 g (155 mMol) Trifluoressigsäureanhydrid zugetropft. Man ließ alles 1 h rühren. Danach wurde die Etherphase mit Wasser gewaschen, getrocknet und im Vakuum eingeengt. Man erhielt 32 g (80 %) eines Rohproduktes, das so weiter eingesetzt wurde.
b) 1-Cyclohexyl-6-nitro-7-(2-trifluoracetamidomethyl-1-pyrrolyl)-chinoxalin-2,3-(1H,4H)-dion
   9,1 g (30 mMol) des Produktes 2g und 9,7 g (37,5 mMol) des Produktes 34a wurden in 250 ml Eisessig bei 100°C 2 h gerührt. Danach wurde alles auf Eiswasser gegossen und der Niederschlag abgesaugt.
   Ausbeute: 12,3 g (86 %)
   ¹H-NMR (D₆-DMSO) :
   - δ =: 1,2-2,0 (8H), 2,3-2,5 (2H), 4,0-4,5 (3H), 6,2 (1H), 6,3 (1H), 6,7 (1H), 7,8 (1H), 7,5 (1H), 9,6 (1H) und ca. 12,5 (1H) ppm.
c) 7-(2-Aminomethyl-1-pyrrolyl)-1-cyclohexyl-6-nitrochinoxalin-2,3-(1H,4H)-dion
   12,0 g (25 mMol) des Produktes 34b wurden in 200 ml Ethanol gegeben, mit 75 ml 4 M Natronlauge versetzt und alles 45 min bei Raumtemperatur gerührt. Das Reaktionsgemisch wurde mit Wasser verdünnt, mit 4 M Salzsäure angesäuert, danach mit wäßriger Natriumhydrogencarbonat-Lösung abgepuffert und der entstandene Niederschlag abgesaugt. Der Rückstand wurde mit Isopropanol/Dimethylformamid (2/1) behandelt und abgesaugt.
   Ausbeute: 5,5 g (57 %); Schmp. > 300°C
   ¹H-NMR (CD₃COOD):
   - δ =: 1,2-2,0 (10H). 2,5 (2H), 4,1 (1H), 4,2 (2H), 6,3 (1H), 6,5 (1H), 6,8 (1H), 7,9 (1H) und 8,1 (1H) ppm.

   N-(1-(1-Cyclohexyl-6-nitro-chinoxalin-2,3-(1H,4H) -dion-7-yl)-2-pyrrolyl)methyl-N'-phenylharnstoff
   1,5 g (4 mMol) des Produktes 34c, 0,52 g (4,4 mMol) Phenylisocyanat und 1 Spatelspitze 4-(N,N-Dimethylamino)pyridin wurden in 25 ml wasserfreiem Dimethylformamid 1 h bei 70°C gerührt. Der Ansatz wurde auf Wasser gegeben, mit Essigester extrahiert, die organische Phase getrocknet und im Vakuum eingeengt. Der Rückstand wurde aus Isopropanol kristallisiert.
   Ausbeute: 1,5 g (76 %); Schmp. 256°C
   ¹H-NMR (CD₃COOD):
   - δ =: 1,2-1,9 (8H), 2,5 2H), 4,3-4,5 (3H), 6,2 (1H), 6,3 (1H), 6,7 (1H), 6,95 (1H); 7,1-7,2 (5H), 7,7 (1H) und 7,9 (1H) ppm.

### Beispiel 35

### 9-(3-(4-Benzylpiperazin-1-yl)-methyl-pyrrol-1-yl)-1-(2-ethoxycarbonylethyl)-benzo[f]chinoxalin-2,3-(1H,4H)-dion-dihydrochlorid

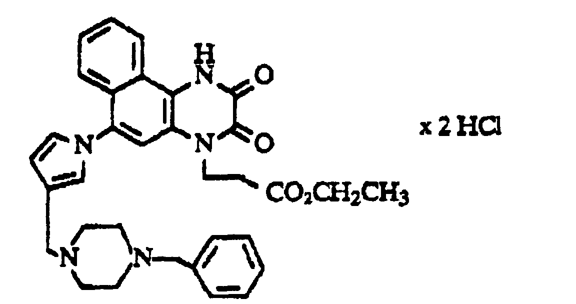

a) N- (1-Nitro-2-naphthyl)-3-aminopropyinsäure
   150 g (0,74 Mol) 2-Methoxy-1-nitro-naphthalin, 329 g (3,7 Mol) β-Alanin und 255 g (1,8 Mol) Kaliumcarbonat wurden in 1 Liter Heizbadflüssigkeit (BASF) 2 h auf 140°C erwärmt. Danach wurde das Reaktionsgemisch auf Eiswasser gegossen, mit 4 M Salzsäure angesäuert und der Niederschlag abgesaugt.
   Ausbeute: 185 g (97 %). Schmp. 159 bis 160°C
   ¹H-NMR (D₆-DMSO):
   2,7 (2H, 3,7 (2H), 7,2-8,8 (7H) und ca, 12,5 (1H) ppm.
b) N-(1-Nitro-2-naphthyl)-3-aminopropionsäureethylester
   10 g (38,4 mMol) des Produktes 35a wurden in 100 ml Ethanol suspendiert. Danach tropfte man 15 ml konzentrierte Schwefelsäure zu und erwärmte alles 1 h auf Rückfluß. Anschließend wurde die Reaktionslösung auf Eiswasser gegossen und mit Essigester extrahiert. Die organische Phase wurde getrocknet und im Vakuum eingeengt. Der Rückstand wurde aus Ethanol umkristallisiert.
   Ausbeute: 7,9 g (72 %), Schmp. 61 bis 62°C
   ¹H-NMR (D₆-DMSO):
   - δ =: 1,2 (3H), 2,7 (2H), 3,7 (2H), 4,1 (2H) und 7,3-8,7 (7H) ppm.
c) N-(2-Ethoxycarbonylethyl)-N-(1-nitro-2-naphthyl)-oxalsäuremonoethylesteramid
   4,0 g (13,9 mMol) des Produktes 35b und 3,8 ml (27,7 mMol) Triethylamin wurden in 100 ml wasserfreiem Tetrahydrofuran gelöst. Bei ca. 0°C tropfte man 1,7 ml (15.3 mMol) Oxalsäuremonoethylesterchlorid, gelöst in 20 ml Tetrahydrofuran, zu. Anschließend wurde alles 30 min bei 0°C und danach 15 min bei Rückflußtemperatur gerührt. Der Ansatz wurde auf Eiswasser gegossen und mit Essigester extrahiert. Die organische Phase wurde mit wäßriger Natriumhydrogencarbonat-Lösung gewaschen, getrocknet und im Vakuum eingeengt.
   Ausbeute: 5,4 g (100 %)
d) 1-(2-Ethoxycarbonylethyl)-benzo[f]chinoxalin-2,3-(1H,4H)-dion
   240 g (0,62 Mol) des Produktes 35c und 80 ml (1 Mol) Triethylamin wurden in 700 ml Tetrahydrofuran gelöst und nach Zugabe von 5 g Palladium/Kohle (10 %ig) hydriert. Danach wurde filtriert und das Filtrat im Vakuum eingeengt. Der Rückstand wurde mit Ethanol behandelt und abgesaugt.
   Ausbeute: 94 g (47 %), Schmp. 227 bis 228°C
   ¹H-NMR (D₆-DMSO) :
   - δ =: 1,2 (3H), 2,7 (2H), 4,1 (2H), 4,5 (2H), 7,4-8,0 (5H), 8,6 (1H) und 12,2 (1H) ppm.
e) 1-(2-Ethoxycarbonylethyl)-9-nitro-benzo[f]chinoxalin-2,3-(1H,4H)-dion
   90 g (0,29 Mol) des Produktes 35d wurden in 1 Liter konzentrierter Essigsäure gelöst und tropfenweise mit 180 ml 65 %iger Salpetersäure versetzt. Danach wurde alles noch ca. 30 min bei Raumtemperatur gerührt. Der ausgefallene Niederschlag wurde abgesaugt.
   Ausbeute: 87,5 g (82 %), Schmp. 230 bis 231°C
   ¹H-NMR (D₆-DMSO):
   - δ =: 1,2 (3H), 2,8 (2H), 4,1 (2H), 4,5 (1H), 7,8 (2H), 8,4 (1H), 8,5 (1H), 8,8 (1H) und 12,5 (1H) ppm.
f) 9-Amino-1(2-ethoxycarbonylethyl) -benzo[f]chinoxalin-2,3-(1H,4H)-dion
   87 g (0,24 Mol) des Produktes 35a wurden in 1 Liter Dimethylformamid gelöst und nach Zugabe von 2,5 g Palladium/Kohle (10 %ig) hydriert. Anschließend wurde filtriert und das Filtrat im Vakuum eingeengt. Der Rückstand wurde mit Ethanol behandelt und abgesaugt.
   Ausbeute: 80 g (100 %), Schmp. > 250°C
   ¹H-NMR (D₆-DMSO):
   - δ =: 1,2 (3H), 2,8 (2H), 4,1 (2H), 4,2 (2H), 6,9 (1H), 7,4 (1H), 7,5 (1H), 8,1 (1H), 8,5 (1H) und 12 (1H) ppm.
g) 1- (2-ethoxycarbonylethyl)-9-(3-formyl-1-pyrrolyl)-benzo[f]chinoxalin-2,3(1H,4H)-dion
   10 g (30,6 mMol) des Produktes 35f und 4,9 g (30,6 mMol) 2,5-Dimethoxy-tetrahydrofuran-3-yl-carbaldehyd wurden in 300 ml Eisessig 15 min unter Rückfluß gekocht. Danach wurde alles im Vakuum eingeengt und der anfallende Rückstand mit Ethanol behandelt und abgesaugt.
   Ausbeute: 3,3 g (76 %), Schmp. > 250°C
   ¹H-NMR (D₆-DMSO):
   - δ =: 1,2 (3H), 2,8 (2H), 4,0 (2H), 4,5 (2H), 6,8 (1H), 7,2-8,2 (6H), 8,7 (1H), 9,8 (1H) und ca. 12,5 (1H) ppm.
h) 9-(3-(4-Benzylpiperazin-1-yl)-methyl-pyrrol-l-yl)-1-(2-ethoxycarbonylethyl)-benzo[f]chinoxalin-2,3-(1H,4H)-diondihydrochlorid
   1,5 g (3,7 mMol) des Produktes 35g, 1,3 g (7,4 mMol) N-Benzylpiperazin und 0,22 g (3,7 mMol) Eisessig wurden in 70 ml wasserfreiem Dimethylformamid gelöst und mit 0,23 g (3,7 mMol) Natriumcyanoborhydrid versetzt. Alles wurde 72 h bei Raumtemperatur gerührt. Danach wurde die Reaktionslösung auf eine wäßrige Natriumhydrogencarbonat-Lösung gegossen und mit Essigester extrahiert. Die organische Phase wurde nochmals mit 2 M Salzsäure extrahiert. Die vereinigten Salzsäure-Phasen wurden anschließend mit Natronlauge neutralisiert, wobei ein Öl ausfiel, das in Methylenchlorid gelöst wurde. Diese organische Phase wurde getrocknet und im Vakuum eingeengt. Der Rückstand wurde in Ether aufgenommen und mit etherischer Chlorwasserstoff-Lösung versetzt. Das ausgefallene Produkt wurde abgesaugt.
   Ausbeute: 1,1 g (47 %), Schmp. > 180°C (Z.)
   ¹H-NMR (D₆-DMSO):
   - δ =: 1,1 (3H), 2,7 (2H), 3,0-3,7 (10H), 4,0 (2H), 4,2-4,4 (2H), 4,5 (2H), 6,6 (1H), 7,2 (1H), 7,3-7,8 (12H), 8,7 (1H), 9,8 (1H) und 12,4 (breit) ppm.

### Beispiel 36

9-(3-(4-Benzylpiperazin-1-yl)-methyl-pyrrol-1-yl)-1-carboxymethyl-benzo[f]chinoxalin-2,3-(1H,4H)-dion

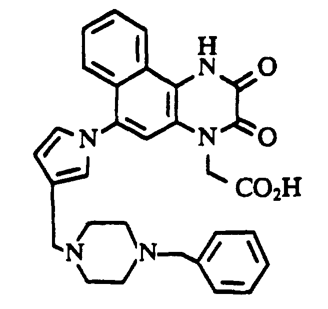

1,0 g (2,65 mMol) des Beispiels 35 wurden analog Beispiel 7 mit Lithiumhydroxid hydrolysiert.
Ausbeute: 0,7 g (50 %), Schmp. > 225°C
¹H-NMR (D₆-DMSO):
- δ =: 2,2 (2H), 2,3-2,6 (4H), 3,2-3,5 (8H), 4,3 (2H), 6,2 (1H), 6,8-7,0 (2H), 7,2-7,5 (9H) und 8,8 1H) ppm.

### Beispiel 37

N-(1-(1-Carboxymethyl-6-trifluormethyl-chinoxalin-2,3- (1H,4H)-dion-7-yl)-pyrrol-3-yl)methyl-N'-phenylharnstoff 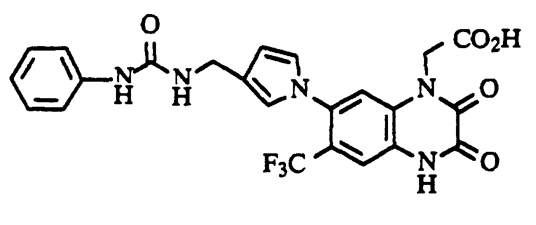
a) Oxalsäuremonoethylester-N-(2-nitro-4-trifluormethylphenyl)amid
   51,5 g (0,25 Mol) 2-Nitro-4-trifluormethylanilin, 45 ml (0,32 Mol) Triethylamin und 0,1 g N,N-Dimethylaminopyridin wurden unter Stickstoffatmosphäre in 500 ml wasserfreiem Tetrahydrofuran gelöst. Bei 0 bis 5°C tropfte man 44,4 g (0,32 Mol) Oxalsäuremonoethylesterchlorid zu. Anschließend rührte man bis zum vollständigen Umsatz bei Raumtemperatur (Dünnschichtchromatographische Kontrolle). Danach wurde alles im Vakuum eingeengt und der Rückstand zwischen Wasser und Essigsäureethylester verteilt. Die organische Phase wurde getrocknet und im Vakuum eingeengt. Das Rohprodukt wurde aus Ethanol umkristallisiert. Man erhielt 68,2 g (89 %) des Produktes.
   ¹H-NMR (CDCl₃):
   - δ =: 1,5 (3H), 4,5 (2H), 8,0 (1H), 8,6 (1H), 9,05 (1H) und 12,2 (1H) ppm.
b) Oxalsäuremonoethylester-N-(ethoxycarbonylmethyl)-N-(2-nitro-4-trifluormethylphenyl)amid
   70 g (0,23 Mol) des Produktes 37a wurden unter einer Stickstoffatmosphäre in 1 Liter wasserfreiem Tetrahydrofuran gelöst. Bei Raumtemperatur wurden 34,8 g (0,31 Mol) Kaliumtert.-butanolat portionsweise zugegeben. Nachdem man 30 min gerührt hatte, wurden 42,1 g (0,25 Mol) Bromessigsäureethylester zugetropft. Anschließend wurde alles 2 h bei Raumtemperatur gerührt. Danach wurde der Ansatz im Vakuum eingeengt und der Rückstand zwischen Essigsäureethylester und Wasser verteilt. Die organische Phase wurde getrocknet und im Vakuum eingeengt. Man erhielt 63 g (70 %) des Rohproduktes, das direkt weiterverarbeitet wurde.
c) 1-(Ethoxycarbonylmethyl)-6-trifluormethyl-chinoxalin-2,3-(1H,4H)-dion
   63 g (0,16 Mol) des Produktes 37b wurden in 1 Liter Essigsäure gelöst und unter Rückfluß gekocht. Dazu gab man portionsweise 54 g (0,97 Mol) Eisenpulver. Nachdem man noch 1 h erwärmt hatte, kühlte man den Ansatz ab und filtrierte. Das Filtrat wurde im Vakuum eingeengt und der Rückstand mit Wasser behandelt. Der anfallende Festkörper wurde abgesaugt und aus Ethanol umkristallisiert. Man erhielt 48,2 g (95 %) des Produktes.
   Schmp. 250 - 251°C.
   ¹H-NMR (D₆-DMSO):
   - δ =: 1,25 (3H), 4,7 (2H), 5,0 (2H), 7,5 (3H) und 12,4 (1H) ppm.
d) 1-(Ethoxycarbonylmethyl)-7-nitro-6-trifluormethyl-chinoxalin-2,3-(1H,4H)-dion
   47 g (0,15 Mol) des Produktes 37c wurden in 500 ml konzentrierter Schwefelsäure gelöst und bei 0°C portionsweise mit 15 g (0,149 Mol) Kaliumnitrat versetzt. Man rührte weitere 30 min und goß anschließend den Ansatz auf Eiswasser. Die wäßrige Phase wurde mit Essigsäureethylester extrahiert, der anfallende Niederschlag abgesaugt und aus Ethanol umkristallisiert. Man erhielt 45,9 g (89 %) des Produktes.
   ¹H-NMR (D₆-DMSO):
   - δ =: 1,25 (3H), 4,2 (2H), 5,0 (2H), 7,7 (1H), 8,25 (1H) und 12,7 (1H) ppm.
e) 7-Amino-1-(ethoxycarbonylmethyl)-6-trifluormethyl-chinoxalin-2,3-(1H,4H)-dion
   43 g (0,12 Mol) des Produktes 37d wurden in 300 ml Dimethylformamid gelöst und nach Zugabe von 2 g Palladium/Kohle (10 %) bei Raumtemperatur und 1 bar hydriert. Anschließend wurde filtriert und das Filtrat im Vakuum eingeengt. Der Rückstand wurde mit Ethanol behandelt und abgesaugt. Man erhielt 37,1 g (95 %) des Produktes.
   Schmp. > 250°C
   ¹H-NMR (D₆-DMSO):
   - δ =: 1,25 (3H), 4,2 (2H), 4,85 (2H), 5,5 (2H), 6,6 (1H), 7,2 (1H) und 12,0 (1H) ppm.
f) l-Ethoxycarbonylmethyl-7-(3-trifluoracetamidomethyll-pyrrolyl)-6-trifluormethyl-chinoxalin-2,3-(1H,4H)-dion
   4,0 g (12,1 mMol) des Produktes 37e und 3,1 g (12,1 mMol) des Produktes 4a wurden in 75 ml Eisessig 10 min unter Rückfluß gekocht. Danach wurde alles im Vakuum eingeengt, der Rückstand mit Ethanol behandelt und abgesaugt.
   Ausbeute: 4,8 g (79 %), Schmp. > 200°C (Z.)
   ¹H-NMR (D₆-DMSO) :
   - δ =: 1,2 (3H), 4,2 (2H), 4,3 (2H), 5,0 (2H), 6,2 (1H), 6,8 (2H), 7,5-7,7 (2H), 9,9 (1H) und 12,5 (1H) ppm.
g) 7- (3-Aminomethyl-1-pyrrolyl)-1-carboxymethyl-6-trifluormethyl-chinoxalin-3,3-(1H,4H)-dion
   4,7 g (9,3 mMol) des Produktes 37f wurden in 50 ml Tetrahydrofuran gegeben und mit 75 ml einer 0,5 M Lithiumhydroxid-Lösung versetzt. Alles wurde 1 h bei Raumtemperatur gerührt. Anschließend wurde das Tetrahydrofuran im Vakuum entfernt und die resultierende wäßrige Phase mit 1 M Salzsäure neutralisiert. Der anfallende Niederschlag wurde abgesaugt.
   Ausbeute: 3,3 g (94 %), Schmp. > 250°C
   ¹H-NMR (CD₃COOD) :
   - δ =: 4,2 (2H), 5,0 (2H), 6,45 (1H), 6,95 (1H), 7,1 (1H), 7,4 (1H) und 7,9 (1H) ppm.
h) N- (1-(1-Carboxymethyl-6-trifluormethyl-chinoxalin-2,3-(1H,4H) -dion-7-yl)methyl-N'-phenylharnstoff
   1,0 g (2,6 mMol) des Produktes 37g und 0,31 g (2,6 mMol) Phenylisocyanat wurden in 20 ml wasserfreiem Dimethylformamid 15 min auf 70°C erhitzt. Danach wurde das Reaktionsgemisch im Vakuum eingeengt. Der Rückstand wurde in Ethanol gelöst. Durch Zugabe von Ether wurde dann das Produkt ausgefällt und danach abgesaugt.
   Ausbeute: 1,1 g (84 %), Schmp. > 190°C (Z.)
   ¹H-NMR (D₆-DMSO):
   - δ =: 4,2 (2H), 4,9 (2H), 6,2 (1H), 6,3 (1H), 6,8-6,9 (2H), 7,1-7,7 (5H), 8,45 (1H) und ca. 12,5 (breit) ppm.

### Beispiel 38

### N'-Benzyl-N-(1-(1-carboxymethyl-6-trifluormethyl-chinoxalin-2,3-(1H,4H)-dion-7-yl)-3-pyrrolyl)methylharnstoff

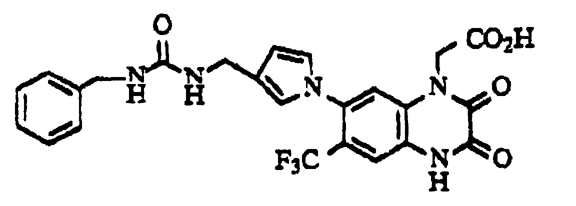

1,0 g (2,6 mMol) des Produktes 37g und 0,35 g (2,6 mMol) Benzylisocyanat wurden analog dem Produkt 37h umgesetzt.
Ausbeute: 1,2 g (89 %), Schmp. 178 bis 180°C
¹H-NMR (D₆-DMSO):
- δ =: 4,1 (2H), 4,2 (2H), 4,9 (2H), 6,1 (1H), 6,2 ((1H), 6,3 (1H), 6,8 (2H), 7,2-7,7 (7H) und Ca. 12,5 (breit) ppm.

### Beispiel 39

### 1-Ethoxycarbonylmethyl-6-nitro-7-(3- (4-(2-phenylethyl)-piperazin-1-yl)methyl-1-pyrrolyl)-chinoxalin-2,3-(1H,4H)-dion

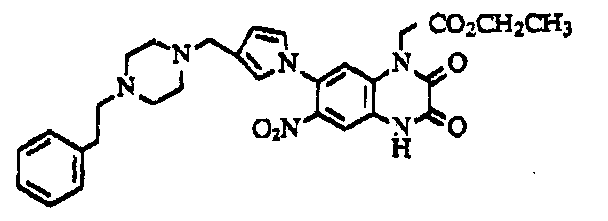

2,0 g (5,2 mMol) des Produktes 5j und 1,97 g (10,4 mMol) 4-(2-Phenylethyl)piperazin wurden analog Vorschrift 5k umgesetzt.
Ausbeute : 0,65 g (22 %), Schmp. 124°C
¹H-NMR (D₆-DMSO):
- δ =: 1,2 (3H), 2,4-2,6 (8H), 2,7 (2H), 3,3 (2H), 4,2 (2H), 5,1 (2H), 6,2 (1H), 6,8 (1H), 6,85 (1H), 7,1-7,3 (5H), 7,5 (1H) und 7,8 (1H) ppm.

### Beispiel 40

### 1-Carboxymethyl-6-nitro-7-(3(4(2-Phenylethyl)-piperazin-1-yl)-methyl-pyrrol-1-yl)-chinoxalin-2,3-(1H,4H)-dion

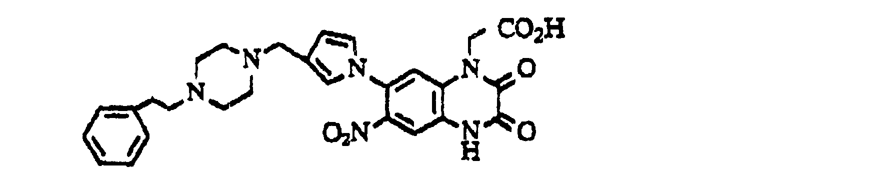

0,6 g (1,1 mMol) des Beispiels 39 wurden analog Vorschrift li mit Lithiumhydroxid hydrolysiert.
Ausbeute: 0,49 g (86 %), Schmp. > 300°C (Z.)
¹H-NMR (CD₃COOD):
- δ =: 3,1 (2H), 3,4 (2H), 3,6-3,9 (6H), 4,3 (2H), 5,1 (2H), 6,4 (1H), 6,9 (1H), 7,1 (1H), 7,2-7,4 (5H), 7,5 (1H) und 8,0 (1H) ppm.

### Beispiel 41

### 1-Ethoxycarbonylmethyl-6-nitro-7-(3-(4-pyridylmethylamido)methyl-1-pyrrolyl)-chinoxalin-2,3-(1H,4H)-dion

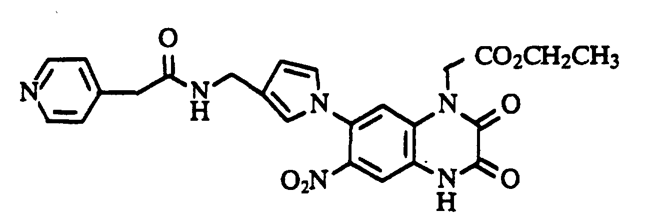

a) 7-(3-Ammonio-methyl-1-pyrrolyl)-1-ethoxycarbonylmethyl-6-nitro-chinoxalin-2,3-(1H,4H)-dion-hydrogensulfat
   14 g (20 mMol) des Produktes 10a wurden in 250 ml Ethanol gegeben und nach Zugabe von 50 ml 2 M Natronlauge 5 min bei Raumtemperatur gerührt. Danach wurde alles auf Eis gegossen, mit 2 M Salzsäure sauer gestellt und anschließend mit wäßriger Natriumhydrogencarbonat-Lösung abgepuffert. Der Niederschlag wurde abgesaugt, das Filtrat im Vakuum eingeengt, der Rückstand mit Dimethylformamid extrahiert und die Dimethylformamid-Lösung im Vakuum eingeengt. Dieser Rückstand wurde mit dem obigen Niederschlag vereint und 2 h in 10 %iger ethanolischer Schwefelsäure unter Rückfluß gekocht. Das Produkt wurde abgesaugt.
   Ausbeute: 6,2 g (56 %), Schmp. 122°C
   ¹H-NMR (D₆DMSO):
   - δ =: 1,2 (3H), 3,9 (2H), 4,1 (2H), 5,0 (2H), 6,4 (1H), 7,0 (1H), 7,1 (1H), 7,5 (1H), 7,9 (1H) ppm.
b) 1-Ethoxycarbonylmethyl-6-nitro-7-(3-(4-pyridylmethylamido)-methyl-1-pyrrolyl)-chinoxalin-2,3-(1H,4H)-dion
   0,64 g (4,7 mMol) 4-Pyridylessigsäure wurden in 100 ml wasserfreiem Dimethylformamid gelöst und mit 0,84 g (5,2 mMol) 1,1-Carbonyldiimidazol versetzt. Alles wurde 30 min bei Raumtemperatur und danach weitere 30 min bei 50°C gerührt. 1,9 g (3,9 mMol) des Produktes 41a, das in 25 ml wasserfreiem Dimethylformamid suspendiert und nach Zugabe von 0,4 g (4 mMol) Triethylamin noch 5 min gerührt worden war, wurden zugegeben und der Reaktionsansatz 1 h bei 80°C gerührt. Anschließend wurde der Ansatz auf Eiswasser gegossen und der pH-Wert durch Zugabe von wäßriger Natriumhydrogencarbonat-Lösung auf 9 eingestellt. Der entstandene Niederschlag wurde abgesaugt.
   Ausbeute: 1,8 g (89 %), Schmp. > 200°C (Z.)
   ¹H-NMR (D₆-DMSO):
   - δ =: 1,2 (3H), 3,5 (2H), 4,2 (2H), 5,0 (2H), 6,1 (1H), 6,8 (2H), 7,3 (3H), 7,7 (1H) und 8,5 (2H) ppm.

### Beispiel 42

### N-(1-Ethoxycarbonylmethyl-6-nitro-chinoxalin-2,3-(1H,4H)-dion-7-yl)-pyrrol-3-yl)methyl-N'-(2-(4-pyridyl)-ethyl)-harnstoff

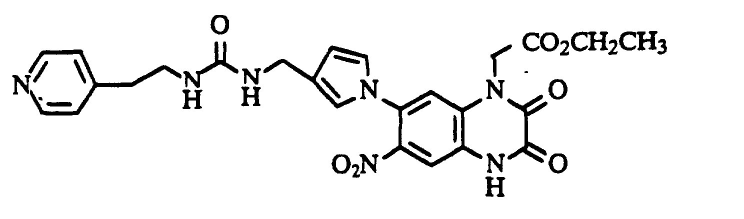

1,9 g (3,9 mMol) des Produktes 41a und 0,48 g (3,9 mMol) 2-(4-Pyridyl)ethylamin wurden analog Vorschrift 41b umgesetzt.
Ausbeute: 1,95 g (93 %), Schmp. > 200°C (Z.)
¹H-NMR (CD₃COOD):
- δ =: 1,4 (3H), 3,25 (2H), 3,7 (2H), 4,3 (4H), 5,2 (2H), 6,4 (1H), 6,9 (2H), 7,5 (1H), 8,0 (2H), 8,1 (1H) und 8,9 (2H) ppm.

### Beispiel 43

### 7-(3-(4-Benzyl-piperazin-1-yl)methyl-1-pyrrolyl)-1-ethoxycarbonylmethyl-6-trifluormethyl-chinoxalin-2,3-(1H,4H)-dion

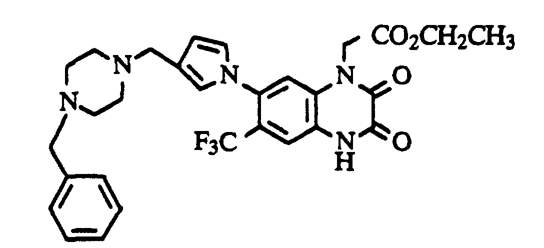

a) 1-Ethoxycarbonylmethyl)-7-(3-formyl-1-pyrrolyl)-6-trifluormethyl-chinoxalin-2,3-(1H,4H)-dion
   25 g (75,5 mMol) des Produktes 37e und 12 g (75,5 mMol) 2,5-Dimethoxytetrahydrofuran-3-yl-carbaldehyd wurden in 300 ml Essigsäure 1 h auf 85°C erwärmt. Anschließend wurde alles im Vakuum eingeengt und chromatographisch an Kieselgel (Fließmittel: Methylenchlorid/Aceton = 3/1) gereinigt. Man erhielt 20,3 g (66 %) des Produktes. Schmp. 236 bis 237°C.
   ¹H-NMR (D₆-DMSO):
   - δ =: 1,2 (3H), 4,2 (2H), 5,0 (2H), 6,6 (1H), 7,05 (1H), 7,65 (1H), 7,8 (2H), 9,8 (1H) und 12,3 (1H) ppm.
b) 7-(3-(4-Benzyl-piperazin-1-yl)methyl-1-pyrrolyl)-1-ethoxycarbonylmethyl-6-trifluormethyl-chinoxalin-2,3-(1H,4H)-dion
   1,5 g (3,7 mMol) des Produktes 43a und 1,3 g (7,2 mMol) 4-Benzylpiperazin wurden analog Vorschrift 8b umgesetzt.
   Ausbeute: 0,98 g (42 %), Schmp. > 190°C (Z.)
   ¹H-NMR (D₆-DMSO):
   - δ =: 1,2 (3H), 3,2-3,8 (8H), 4,1-4,4 (6H), 5,0 (2H), 6,5 (1H), 7,0 (1H), 7,2 (1H), 7,4-7,8 (7H) und ca. 12,7 (breit) ppm.

### Beispiel 44

### 7-(3-(4-Benzyl-piperazin-1-yl)methyl-1-pyrrolyl)-1-carboxymethyl-6-trifluormethyl-chinoxalin-2,3-(1H,4H)-dion

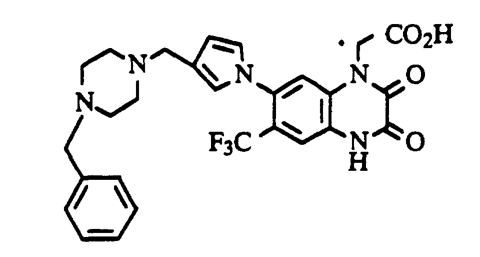

0,9 g (1,4 mMol) des Produktes 43 wurden analog Beispiel 7 mit Lithiumhydroxid hydrolysiert.
Ausbeute: 0,45 g (60 %), Schmp. > 245°C (Z.)
¹H-NMR (CD₃COOD):
- δ =: 3,5-3,8 (8H), 4,3 (2H), 4,4 (2H), 5,05 (2H), 6,4 (1H), 6,9 (1H), 7,1 (1H),7,4-7,6 (6H) und 7,7 (1H) ppm.

### Beispiel 45

### N-(1-(1-Cyclohexyl-6-nitro-chinoxalin-2,3-(1H,4H)-dion-7-yl)-pyrrol-3-yl)methyl-N'-(4-pyridyl)-harnstoff

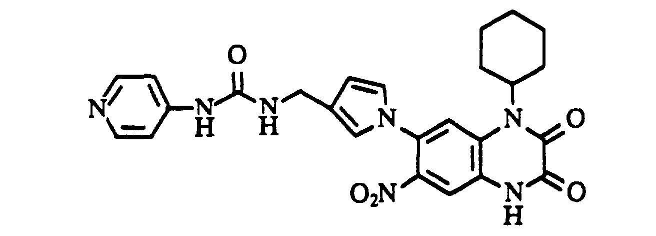

a) N-(1-(1-Cyclohexyl-6-nitro-chinoxalin-2,3-(1H,4H)-dion-7-yl)-pyrrol-3-yl)methyl-N'-(1-imidazolyl)-harnstoff
   2,0 g (5,2 mMol) des Produktes 2i und 0,92 g 1,1-Carbonyldiimidazol wurden analog Vorschrift 2j umgesetzt.
   Ausbeute: 2,0 g (77 %)
b) N-(1-(1-Cyclohexyl-6-nitro-chinoxalin-2,3-(1H,4H)-dion-7-yl)-3-pyrrolylmethyl-N'-(4-pyridyl)-harnstoff
   1,7 g (3,6 mMol) des Produktes 45a und 0,34 g (3,6 mMol) 4-Aminopyridin wurden analog Vorschrift 2j bei 120°C umgesetzt.
   Ausbeute: 0,45 g (25 %), Schmp. > 240°C (Z.)
   ¹H-NMR (D₆-DMSO):
   - δ =: 1,1-1,8 (8H), 2,4 (2H), 4,2 (2H), 4,4 (1H), 6,2 (1H), 6,65 (1H), 7,4 (2H), 7,5 (1H), 7,8 (1H), 8,3 (2H), 9,0 (1H) und ca. 12,5 (breit) ppm.

### Beispiel 46

### N-(1-(1-Cyclohexyl-6-nitro-chinoxalin-2,3-(1H,4H)-dion-7-yl)-pyrrol-3-yl)methyl-N'-phenyl-thioharnstoff

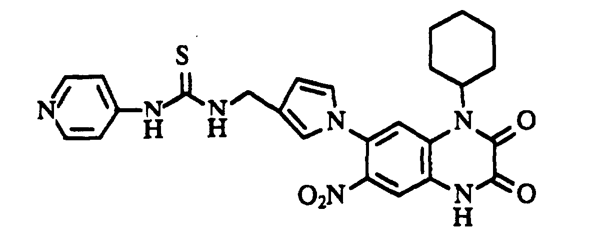

1,0 g (2,6 mMol) des Produktes 2i und 0,35 g (2,8 mMol) Phenylisothiocyanat wurden analog Vorschrift 2j umgesetzt.
Ausbeute: 1,2 g (89 %), Schmp. > 210°C (Z.)
¹H-NMR (CD₃COOD):
- δ =: 1,1-1,9 (8H), 2,4 (2H), 4,5 (1H), 4,6 (2H), 6,3 (1H), 7,0 (2H), 7,1 (1H), 7,3 (2H), 7,5 (2H), 7,6 (1H), 7,8 (1H), 7,9 (1H), 9,6 (1H) und ca. 12,5 (breit) ppm.

### Beispiel 47

### 6-Nitro-7- (3-(4-(3-trifluormethylphenyl)piperazin-1-yl)methylpyrrol-1-yl)-chinoxalin-2,3-(1H,4H)-dion

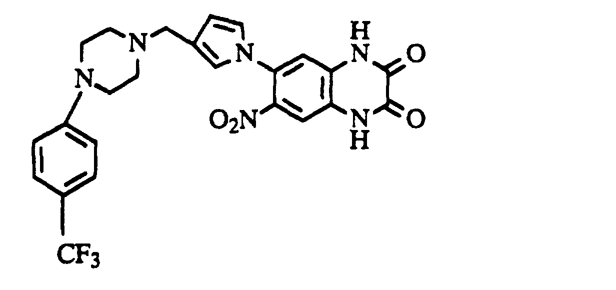

a) 7-(3-Formyl-1-pyrrolyl)-6-nitro-chinoxalin-2,3-(1H,4H)-dion
   2,0 g (9,0 mMol) des Produktes 3c und 1,4 g (9,0 mMol) 2,5-Dimethoxy-tetrahydrofuran-3-yl-carbaldehyd wurden in 50 ml Essigsaure 1 h unter Rückfluß gekocht. Der Ansatz wurde anschließend auf Wasser gegossen und der anfallende Niederschlag abgesaugt. Dieser Rückstand wurde nach Zugabe von etwas Aktivkohle und Kieselgel in 60 ml Dimethylformamid/ Tetrahydrofuran (1/5) aufgekocht. Die Suspension wurde filtriert und das Filtrat im Vakuum eingeengt. Der Rückstand wurde in Wasser suspendiert und abgesaugt. Man erhielt 0,3 g (13 % d.Th.) des Produktes. Schmp. > 250°C.
   ¹H-NMR (D₆-DMSO):
   - δ =: 6,65 (1H), 7,1 (1H), 7,15 (1H), 7,9 (1H), 7,95 (1H), 9,7 (1H) und ca. 12,3 (breit) ppm.
b) 6-Nitro-7-(3-(4-(3-trifluormethylphenyl)piperazin-1-yl)methyl-pyrrol-1-yl)-chinoxalin-2,3-(1H,4H)-dion
   1,5 g (5 mMol) des Produktes 47a und 2,3 g (10 mMol) Phenylpiperazin wurden analog Vorschrift 1j umgesetzt.
   Ausbeute: 1,76 g (69 %), Schmp. > 300°C
   ¹H-NMR (D₆-DMSO):
   - δ =: 2,6 (4H), 3,3 (4H), 3,4 (2H), 6,2 (1H), 6,8 (1H), 6,9 (1H), 7,0-7,3 (4H), 7,4 (1H), 7,8 (1H) und ca. 12,3 (breit) ppm.

### Beispiel 48

### N-(1-(1-(2-carboxyethyl)-benzo[f]chinoxalin-2,3-(1H,4H)-dion-9-yl)-pyrrol-3-yl)methyl-N'-phenylharnstoff

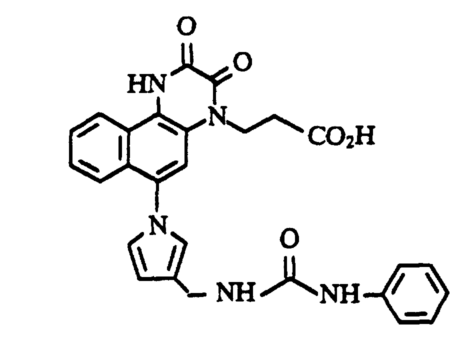

a) 1-(2-Ethoxycarbonylethyl)-9-(3-trifluoracetamidomethyl-1-pyrrolyl)-benzo[f]chinoxalin-2,3-(1H,4H)-dion
   23 g (70,3 mMol) des Produktes 35f und 18,0 g (70,3 mMol) des Produktes 4a wurden in 500 ml Eisessig für 15 min unter Rückfluß gekocht. Danach wurde der Ansatz auf Eiswasser gegossen und der anfallende Niederschlag abgesaugt.
   Ausbeute: 31,8 g (86 %), Schmp. > 125°C (Z.)
   ¹H-NMR (D₆-DMSO):
   - δ =: 1,2 (3H), 2,7 (2H), 4,0 (2H), 4,4 (2H), 4,5 (2H), 6,3 (1H), 7,1 (2H), 7,5-7,8 (4H), 8,7 (1H), 9,8 (1H) und 12,3 (1H) ppm.
b) 9-(3-Aminomethyl-pyrrol-1-yl)-1-(2-carboxyethyl)-benzo[f]-chinoxalin-2,3(1H,4H)-dion
   31,5 g (62,7 mMol) des Produktes 48a wurde analog Vorschrift 31c mit Lithiumhydroxid hydrolysiert.
   Ausbeute: 20,0 g (85 %), Schmp. > 250°C
   ¹H-NMR (D₆-DMSO):
   - δ =: 3,0 (2H), 4,3 (2H), 4,7 (2H), 6,5 (1H), 7,1 (1H), 7,3 (1H), 7,7-7,9 (4H) und 8,5 (1H) ppm.
c) N-((1-(1-(2-carboxyethyl)-benzo[f]chinoxalin-2,3-(1H,4H)-dion-9-yl)-pyrrol-3-yl)methyl-N'-phenylharnstoff
   3,5 g (9,3 mMol) des Produktes 48b und 1,2 g (10,2 mMol) Phenylisocyanat wurden in 70 ml wasserfreiem Dimethylformamid für 30 min auf 120°C erhitzt. Danach wurde alles im Vakuum eingeengt und der Rückstand chromatographisch (Fließmittel: Toluol/Tetrahydrofuran/Methanol/Eisessig = 10/20/10/1) gereinigt.
Ausbeute: 3,0 g (67 %), Schmp. > 214°C (Z.)
¹H-NMR (D₆-DMSO):
- δ =: 2,5 (2H), 4,2 (2H), 4,5 (2H), 6,2 (1H), 6,5 (1H), 6,8 (1H), 7,0-7,8 (10H), 8,6 (1H), 8,8 1H) und ca. 12,3 (breit) ppm.

### Beispiel 49

### N'-Benzyl-N-(1-(1-(2-carboxyethyl)-benzo[f]chinoxalin-2,3-(1H,4H)-dion-9-yl)-pyrrol-3-yl)methylharnstoff

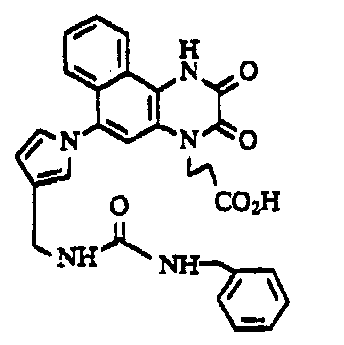

1,6 g (4,2 mMol) des Produktes 48b und 0,62 g (4,7 mMol) Benzylisocyanat wurden analog Vorschrift 48c umgesetzt.
Ausbeute: 2,0 g (93 %), Schmp. > 163°C (Z.)
¹H-NMR (D₆-DMSO) :
- δ =: 2,7 (2H), 4,2 (4H), 4,5 (2H), 6,3 (1H), 6,4 (1H), 6,45 (1H), 7,0 (2H), 7,1 (1H), 7,2-7,4 (5H), 7,5-7,8 (4H), 8,7 (1H) und ca. 12,2 (breit) ppm.

### Beispiel 50

### N-{1-(1-{2-Carboxyethyl)-benzo[f] chinoxalin-2,3-(1H, 4H)-dion-9-yl)-pyrrol-3-yl) methyl-N'-(4-methoxyphenyl)-harnstoff

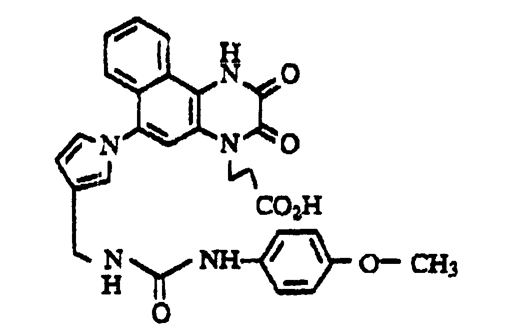

1,5 g (4 mMol) des Produktes 48b und 0,65 g (4,4 mMol) 4-Methoxyphenylisocyanat wurden analog Vorschrift 48c umgesetzt.
Ausbeute: 1,6 g (77 %), Schmp. > 240°C
¹H-NMR (D₆-DMSO) :
- δ =: 2,7 (2H), 3,7 (3H), 4,3 (2H), 4,5 (2H), 6,3 (2H), 6,7 (2H), 7,3 (2H), 7,5 (2H), 7,7 (1H), 7,8 (1H), 8,3 (1H), 8,7 (1H) und ca. 12,5 (breit) ppm.

### Beispiel 51

### N-(1-(1-(2-Carboxyethyl)-benzo[f]chinoxalin-2,3-(1H,4H)-dion-9-yl]-pyrrol-3-yl)methyl-N'-(2-methylphenyl)-harnstoff

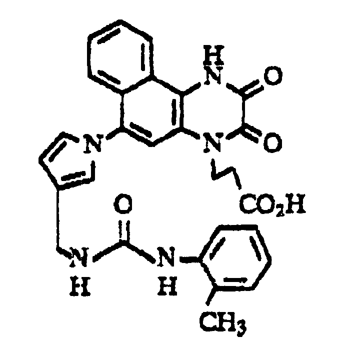

1,5 g (4 mMol) des Produktes 48b und 0,65 g (4,4 mMol) 2-Methylphenylisocyanat wurden analog Vorschrift 49c umgesetzt.
Ausbeute: 1,9 g (94 %), Schmp. > 192°C (Z.)
¹H-NMR (D₆-DMSO):
- δ =: 2,2 (3H), 2,75 (2H), 4,3 (2H), 4.5 (2H), 6,4 (1H), 6,9 (2H), 7,1 (4H), 7,4-7,8 (6H), 8,7 (1H) und ca. 12,5 (breit) ppm.

### Beispiel 52

### N-(1(1-(2-Carboxyethyl)-benzo[f]chinoxalin-2,3-(1H,4H)-dion-9-yl)-pyrrol-3-yl)methyl-N'-(4-nitrophenyl)-harnstoff

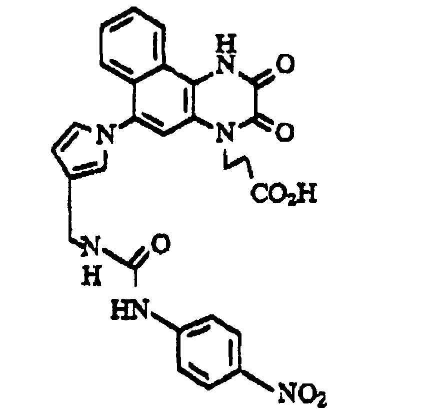

1,6 g (4,2 mMol) des Produktes 48b und 0,73 g (4,4 mMol) 4-Nitrophenylisocyanat wurden analog Vorschrift 49c umgesetzt.

Ausbeute: 1,9 g (87 %), Schmp. > 210°C (Z.)
¹H-NMR (D₆-DMSO):
- δ =: 2,6 (2H), 2,75 (3H), 2,9 3H), 4,3 (2H), 4,5 (2H), 6,3 (1H), 6,7 (1H), 7,1 (2H), 7,5-7,9 (6H), 8,0 (1H), 8,2 (2H), 8,7 (1H), 9,3 (1H) und ca. 12,5 (breit) ppm.

### Beispiel 53

### N-(1-(1-Cyclohexyl-6-nitro-chinoxalin-2,3-(1H,4H)-dion-7-yl)-pyrrol-3-yl)methyl-N'-phenylguanidin

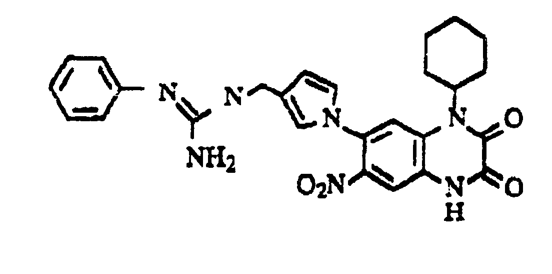

1,9 g (4,8 mMol) des Produktes 2i, 1,4 g (4,8 mMol) S-Methyl-N-phenyl-isothioharnstoffhydroiodid und eine Spatelspitze 4-(N,N-Dimethylamino)pyridin wurden 3 h in 50 ml Pyridin unter Rückfluß gekocht. Danach wurde alles auf Wasser gegeben und der Niederschlag abgesaugt.
Ausbeute: 2,0 g (84 %), Schmp. 249°C
¹H-NMR (CD₃COOD):
- δ =: 1,2-2,0 (8H), 2,6 (2H), ca. 4,5 (3H), 6,4 (1H), 6,8 (1H), 7,0 (1H), 7,3 (3H), 7,4 (2H), 7,7 (1H) und 7,9 (1H) ppm.

### Beispiel 54

### N-(1-(1-Carboxymethyl-6-trifluormethyl-chinoxalin-2,3-(1H,4H)-dion-7-yl)-3-pyrrolyl)-methyl-N'-(3-nitrophenyl)-harnstoff

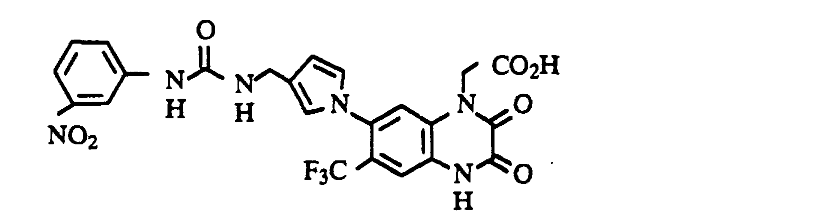

1,2 g (3,1 mMol) des Produktes 37g und 0,57 g (3,4 mMol) 3-Nitrophenylisocyanat wurden analog der Vorschrift 37h bei 120°C umgesetzt.
Ausbeute: 1,2 g (71 %), Schmp. > 200°C (Z.)
¹H-NMR (D₆-DMSO):
- δ =: 4,2 (2H), 4,9 (2H), 6.2 (2H), 6,6 (1H), 6,9 (2H), 7,4-7,9 (5H), 8,5 (1H), 9,1 (1H), 12,5 (1H) und ca. 13,5 (breit) ppm.

### Beispiel 55

### 1-Carboxymethyl-6-nitro-7- (3-(4-picolylamido)methyl-1-pyrrolyl)-chinoxalin-2,3-(1H,4H)-dion

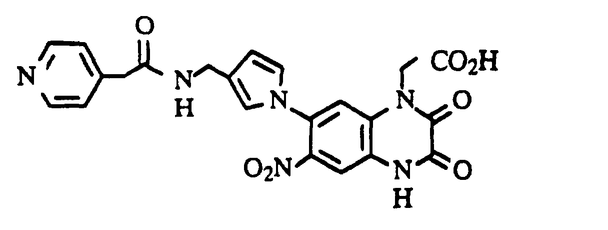

1,6 g (3,2 mMol) des Produktes 41 wurden analog Vorschrift 7 mit Lithiumhydroxid hydrolysiert.
Ausbeute: 12 g (76 %), Schmp. > 300°C
¹H-NMR (D₆-DMSO):
- δ =: 3,5 (2H), 4,1 (2H), 4,6 (2H), 6,1 (1H), 6,8 (2H), 7,1 (1H), 7,3 (2H), 7,9 (1H), 8,45 (2H), 8,5 (1H) und ca. 12,5 (breit) ppm.

### Beispiel 56

### N-(1-(1-Carboxymethyl-6-trifluormethyl-chinoxalin-2,3- (1H,4H)-dion-7-yl)-3-pyrrolyl)-methyl-N'-(4-trifluormethylphenyl)-harnstoff

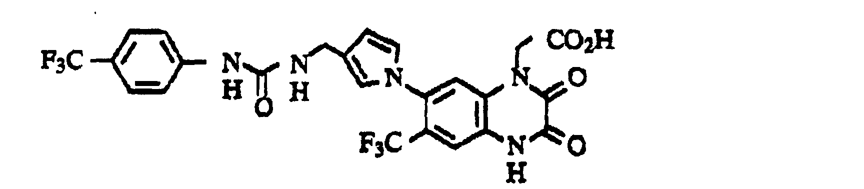

1,6 g (4,2 mMol) des Produktes 37g und 0,8 g (4,2 mMol) 4-Trifluormethylphenylisocyanat wurden analog Vorschrift 37h bei 115°C umgesetzt.
Ausbeute: 1,8 g (83 %), Schmp. > 200°C
¹H-NMR (D₆-DMSO):
- δ =: 4,2 (2H), 5,0 (2H), 6,3 (1H), 6,5 (1H), 6,9 (2H), 7,4-7,8 (4H), 8,9 (1H), 12,5 (1H) und ca. 13,2 (breit) ppm.

### Beispiel 57

### N'-(4-Bromphenyl)-N-(1-(1-carboxymethyl-6-trifluormethylchinoxalin-2,3-(1H,4H)-dion-3-yl)-3-pyrrolyl)-methyl-harnstoff

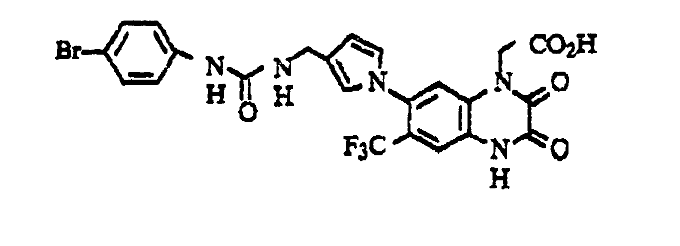

1,2 g (3,1 mMol) des Produktes 37g und 0,62 g (3,1 mMol) 4-Bromphenylisocyanat wurden analog der Vorschrift 37h bei 120°C umgesetzt.
Ausbeute: 1,8 g (99 %), Schmp. > 200°C
¹H-NMR (D₆-DMSO):
- δ =: 4,2 (2H), 4,9 (2H), 6,2 (1H), 6,4 (1H), 6,9 (2H), 7,3-7,7 (5H), 8,7 (1H) und 12,5 (1H) ppm.

### Beispiel 58

### N-(1-(1-carboxymethyl-6-trifluormethyl-chinoxalin-2,3-(1H,4H)-dion-7-yl)-3-pyrrolyl)-methyl-N'-(4-nitrophenyl)-harnstoff

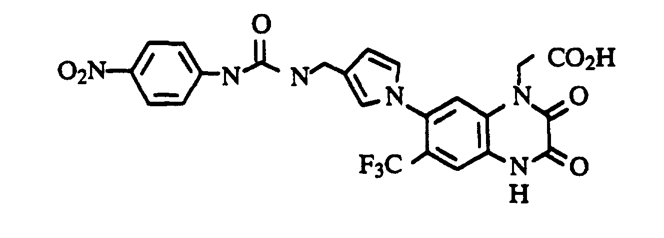

1,0 g (2,6 mMol) des Produktes 37g und 0,48 g (2,9 mMol) 4-Nitrophenylisocyanat wurden analog Vorschrift 37 h bei 120°C umgesetzt
Ausbeute: 1,25 g (88 %), Schmp. > 220°C (Z.)
¹H-NMR (D₆-DMSO):
- δ =: 4,2 (2H), 4,8 (2H), 6,2 (1H), 6,8 (1H), 6,9 (1H), 7,05 (1H), 7,4 (1H), 7,6-7,8 (3H), 8,1 (2H), 10,0 (1H), 12,6 (breit) ppm.

### Beispiel 60

### N-(1-(1-(2-Carboxyethyl)-benzo[f]chinoxalin-2,3-(1H,4H)-dion-9-yl)-pyrrol-3-yl)methyl-N'-(4-picolyl)-harnstoff

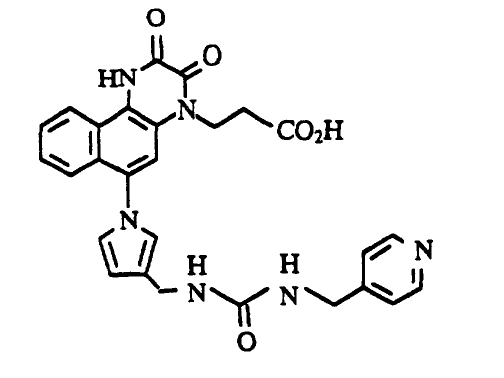

0,64 g (5,9 mMol) 4-Picolylamin und 0,96 g (5,9 mMol) 1,1-Carbonyldiimidazol wurden in 75 ml wasserfreiem Dimethylformamid für 30 min bei Raumtemperatur und 30 min bei 50°C gerührt. Danach wurden 1,5 g (4 mMol) des Produktes 48b zugegeben und alles für 20 min auf 125°C erhitzt. Anschließend wurde alles im Vakuum eingeengt und der Rückstand mit Dimethylformamid/Ethanol behandelt und abgesaugt. Der Festkörper wurde noch chromatographisch (Fließmittel: Toluol/Tetrahydrofuran/Methanol/Dimethylformamid/Eisessig = 16/8/8/8/1) gereinigt.
Ausbeute: 0,5 g (25 %), Schmp. > 180°C (Z.)
¹H-NMR (CD₃COOD):
- δ =: 3,0 (2H), 4,4 (2H), 4,7 (2H), 4,8 (2H), 6,4 (1H), 7,05 (1H), 7,1 (1H), 7,6 (1H), 7,8 (3H), 7,95 (2H), 8,5 (1H) und 8,8 (2H) ppm.

### Beispiel 61

Cis-N-(1-(1-(2-Hydroxy-1-cyclohexyl)-6-trifluormethyl-chinoxalin-2,3-(1H,4H)-dion-7-yl)-pyrrol-3-yl)methyl-N'-phenylguanidin 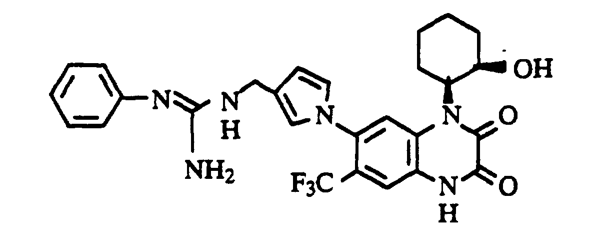
a) 2-(N-(2-Nitro-4-trifluormethyl-phenyl)amino)-cyclohexanol
   44,2 g (0,2 Mol) 4-Chlor-3-nitrobenzotrifluorid, 29,7 g 2-Aminocyclohexanol und 54,2 g Kaliumcarbonat wurden in 500 ml Dimethylformamid für 2 h bei 100°C gerührt. Danach wurde alles auf Eiswasser gegossen und der Niederschlag abgesaugt.
   Ausbeute: 56,1 g (94 %)
   ¹H-NMR (D₆-DMSO):
   - δ =: 1,2-2,2 (8H), 3,5 (2H), 5,0 (1H), 7,3 (1H), 7,7 (1H) und 8,4 (2H) ppm.
b) 4-(2-Acetyloxy-1-cyclohexyl)amino-3-nitro-benzoinfluorid
   93,5 g (0,31 Mol) des Produktes 61a wurden in 1 Liter Pyridin gelöst und bei ca. 10°C tropfenweise mit 30,2 g (0,38 Mol) Acetylchlorid versetzt. Danach wurde alles noch ca. 30 min gerührt. Das Reaktionsgemisch wurde im Vakuum eingeengt und der Rückstand auf Eiswasser gegeben. Diese wäßrige Phase wurde mit Essigester extrahiert. Die organische Phase wurde anschließend getrocknet und im vakuum eingeengt. Der Rückstand wurde mit heißem n-Heptan extrahiert, das anschließend im Vakuum eingeengt wurde.
   Ausbeute: 100,2 g (94 %)
   ¹H-NMR (D₆-DMSO) ;
   - δ =: 1,3-2,1 (11H), 3,9 (1H), 4,9 (1H), 7,4 (1H), 7,8 (1H), 8,2 (1H) und 8,3 (1H) ppm.
c) 4- (2-Acetyloxy-1-cyclohexyl)amino-3-amino-benzotrifluorid
   100,2 g (0,29 Mol) des Produktes 61c wurden in 500 ml Tetrahydrofuran/Methanol (1:1) gelöst und nach Zugabe von 5 g Palladium/Kohle (10 %ig) hydriert. Danach wurde filtriert und das Filtrat im Vakuum eingeengt.
   Ausbeute: 91,6 g (100 %)
   ¹H-NMR (D₆-DMSO):
   - δ =: 1,2-2,1 (11H), 3,4 (1H), 4,6-5,0 (3H), 6,7 (1H) und 6,8 (2H) ppm.
d) 1-(2-Acetoxy-cyclohexyl)-6-trifluormethyl-chinoxalin-2,3-(1H,4H)-dion
   90,4 g (0,29 Mol) des Produktes 51c und 43,4 g (0,43 Mol) Triethylamin wurden in 500 ml Tetrahydrofuran gelöst und bei 5 bis 10°C mit 48,7 g (0,35 Mol) Oxalsauremonoethylesterchlorid, gelöst in 50 ml wasserfreiem Tetrahydrofuran, tropfenweise versetzt. Alles wurde danach noch für 1 h gerührt. Der Niederschlag wurde abgesaugt und das Filtrat im Vakuum eingeengt. Der Rückstand wurde zwischen Eiswasser und Essigester verteilt. die organische Phase getrocknet und im Vakuum eingeengt. Das so erhaltene Öl wurde in 500 ml Diphenylether für ca. 1,5 h auf 175°C erwärmt. Danach kühlte man auf 100°C ab und gab vorsichtig 2 l Ligroin zu. Bei Raumtemperatur wurde denn der anfallende Niederschlag abgesaugt.
   Ausbeute: 75,7 g (72 %), Schmp. 237°C
   ¹H-NMR (D₆-DMSO):
   - δ =: 1,3-2,2 (11H), 4,6 (1H), 5,7 (1H), 7,4-7,5 (2H), 7,9 (1H) und ca. 12,2 (breit) ppm.
e) Cis-1-(2-Acetoxy-cyclohexyl)-7nitro-6-trifluormehtylchinoxalin-2,3-(1H,4H)-dion
   5,0 g (13,5 mMol) des Produktes 61d wurde in 100 ml konzentrierter Schwefelsäure gelöst und bei 0°C mit 1,4 g (13,5 mMol) Kaliumnitrat versetzt. Der Ansatz wurde noch 2 h bei 0°C gerührt, Danach wurde alles vorsichtig auf Eis gegossen und die wäßrige Phase mit Essigester extrahiert, Die organische Phase wurde getrocknet und im Vakuum eingeengt. Der Rückstand wurde aus i-Propanol/Methanol umkristallisiert und der Niederschlag abgesaugt. Die Mutterlauge wurde danach im Vakuum eingeengt. Dieser Rückstand wurde mit Essigsäureanhydrid behandelt und erneut abgesaugt. Aus dem Filtrat wurde durch zugabe von Ether das Produkt ausgefällt und abgesaugt.
   Ausbeute: 1,1 g (20 %), Schmp. 277°C
   ¹H-NMR (D₆-DMSO):
   - δ =: 1,3-2,2 (11H), 4,2 (1H), 4,9 (1H), 7,6 (1H), 9,2 (1H) und ca. 12 (breit) ppm.
f) Cis-1-(2-Acetoxy-cyclohexyl)-7-amino-6-trifluormethylchinoxalin-2,3-(1H,4H)-dion
   15,7 g (38 mMol) des Produktes 61e wurden in 300 ml Tetrahydrofuran/Methanol (1/1) gegeben und nach Zugabe von 1,5 g Palladium/Kohle (10 %ig) hydriert. Danach wurde filtriert und das Filtrat im Vakuum eingeengt.
   Ausbeute: 14,5 g (99 %)
g) Cis-1-(2-Acetoxy-cyclohexyl)-7-(3-trifluoracetamidomethyl-1-pyrrolyl)-6-trifluormethyl-chinoxalin-2,3-(1H,4H)-dion
   7,7 g (20 mMol) des Produktes 61f und 7,7 g (30 mMol) des Produktes 4a wurden in 200 ml Eisessig 1,5 h bei 70°C und danach 1,5 h 100°C erwärmt. Das heiße Reaktionsgemisch wurde mit Aktivkohle versetzt und filtriert. Das Filtrat wurde danach auf Eiswasser gegossen und der anfallende Niederschlag abgesaugt.
   Ausbeute: 10,1 g (90 %), Schmp. > 180°C (Z.)
   ¹H-NMR (D₆-DMSO):
   - δ =: 1,3-2,0 (11H), 4,3 (2H), 4,9 (1H), 5,2 (1H), 6,2 (1H), 6,8 (2H), 7,6 (1H), 7,8 (1H), 9,8 (1H) und ca. 12,2 (1H) ppm.
h) Cis-7- (3-Aminomethyl-1-pyrrolyl) -1-(2-hydroxy-cyclohexyl)-6-trifluormethyl-chinoxalin-2,3-(1H,4H)-dion
   2,0 g (3,6 mMol) wurden in 20 ml Ethanol gegeben und mit 20 ml 1 M Natronlauge versetzt. Alles wurde 1 h bei Raumtemperatur gerührt. Anschließend wurde das Reaktionsgemisch mit 1 M Salzsäure neutralisiert und Ethanol im vakuum entfernt. Der ausgefallene Niederschlag wurde abgesaugt.
   Ausbeute: 1,1g (73 %), Schmp. > 220°C (Z.)
   ¹H-NMR (D₆-DMSO):
   - δ =: 1,3-1,9 (8H), 2,6 (1H), 4,1 (2H), 4,8 (1H), 5,3 (1H), 6.25 (1H), 6,85 (1H), 6,9 (1H), 7,5 (2H) und 8,2 (1H) ppm.
i) Cis-N-(1-(1-(2-Hydroxy-cyclohexyl) -6-trifluormethylchinoxalin-2,3-(1H,4H)-dion-3-yl)-pyrrol-3-yl)-methyl-N'phenyl-guanidin
   0,93 g (2,2 mMol) des Produktes 61h wurden in 40 ml wasserfreies Pyridin gegeben und mit 0,65 g (2,2 mMol) S-Methyl-N-phenyl-isothioharnstoff versetzt und danach alles 1,5 h unter Rückfluß gekocht. Danach wurde filtriert und das Filtrat auf Wasser gegeben. Der ausgefallene Niederschlag wurde abgesaugt.
   Ausbeute: 0,69 g (57 %), Schmp. > 260°C (Z.)
   ¹H-NMR (D₆-DMSO) :
   - δ =: 1,3-2,0 (8H), 2,6 (1H), 4,1 (1H), 4,3 (2H), 4,9 (1H), 6,2 (1H), 6,8 (1H), 6,9 (1H), 7,0 (3H), 7,3 (2H), 7,4 (1H) und 8,1 (1H) ppm.

### Beispiel 62

### Cis-7-(3-(4-Benzyl-1-piperazinyl)methyl-1-pyrrolyl)-1-(2-hydroxy-Cyclohexyl)-7-trifluormethyl-chinoxalin-2,3-(1H,4H)-dion

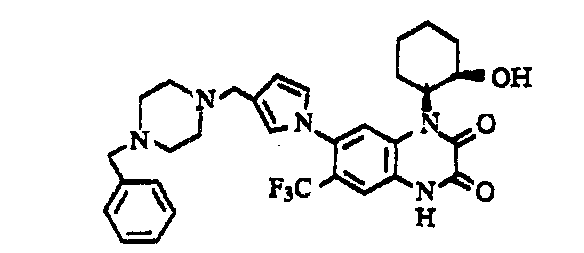

a) Ci*s*-1-(2-ACetoxy-cyclohexyl)-*7-*(3-formyl-1-pyrrolyl)-6-trifluormethyl-chinoxalin-2,3-(1H,4H)-dion
   1,5 g (4 mMol) des Produktes 61f und 0,8 g (5 mMol) 2, 5-Dimethoxy-tetrahydrofuran-3-yl-carbaldehyd wurden in 25 ml Bisessing für 30 min bei 80°C gerührt. Danach wurde das Reaktionsgemisch auf Wasser gegeben und der Niederschlag abgesaugt.
   Ausbeute: 1,3 g (71 %), Schmp. > 200°C (Z.)
   ¹H-NMR (D₆-DMSO) :
   - δ =: 1,4-2,0 (11H), 4,8 (1H), 5,2 (1H), 6,7 (1H), 7,1 (1H), 7,6 (1H), 7,9 (2H), 9,8 (1H) und 12,3 (1H) ppm.
b) Cis-7- (3-Formyl-1-pyrrolyl)-1-(2-hydroxy-cyclohexyl)-6-trifluormethyl-chinoxalin-2,3-(1H,4H)-dion
   1,2 g (2,6 mMol) des Produktes 62a wurden in 20 ml Methanol gegeben und mit 10 ml 2 M Natronlauge versetzt. Alles wurde für 45 min bei Raumtemperatur gerührt. Anschließend wurde mit 2 M Salzsaure neutralisiert und der Alkohol im Vakuum entfernt, Der ausgefallene Niederschlag wurde abgesaugt.
   Ausbeute: 1,0 g (95 %), Schmp. > 246°C (Z.)
   ¹H-NMR (D₆-DMSO) :
   - δ =: 1,3-2,0 (8H), 2,6 (1H), 4,1(1H) 4,9 (1H), 5,2 (1H), 5,7 (1H), 7,1 (1H), 7,6 (1H), 7,8 (1H), 8,4 (1H), 9,8 (1H) und 12,3 (1H) ppm.
c) Cis-7-(3-(4-Benzyl-1-piperazinyl)methyl-1-pyrrolyl)-1-(2-hydroxy-cyclohexyl)-7-trifluormethyl-chinoxalin-2,3-(1H,4H)-dion
   0,9 g (2,1 mMol) des Produktes 62b und 0,75 g (4,3 mMol) 4-Benzylpiperazin wurden analog Vorschrift 5h umgesetzt.
   Ausbeute: 0,66 g (53 %), Schmp. > 300°C
   ¹H-NMR (CD₃COOD):
   - δ =: 1,4-2,0 (8H), 2,9 (1H), 3,6-3,8 (8H), 4,35 (2H), 4,4 (2H), 4,5 (1H), 4,9 (1H), 6,45 (1H), 6,9 (1H), 7,2 (1H), 7,4-7,6 (5H), 7,6 (1H) und 8,1 (1H) ppm.

### Beispiel 63

### Cis-N-(1- (1-(2-Hydroxycyclohexyl)-6-trifluormethyl-chinoxalin-2,3-(1H,4H)-dion-7-yl)-pyrrol-3-yl)methyl-N'-phenylharnstoff

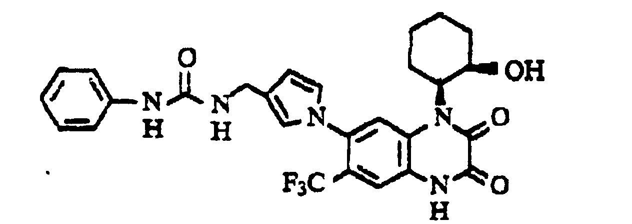

1,0 g (2,4 mMol) des Produktes 61h und 0,28 g (2,4 mMol) Phenylisocyanat wurden analog Vorschrift 2j umgesetzt.
Ausbeute: 0,4 g (31 %), Schmp. > 230°C (Z.)
¹H-NMR (D₆-DMSO):
- δ =: 1,4-2,0 (8H), 2,6 (1H), 4,1 (1H), 4,2 (2H), 5,9 (1H), 6,2 (1H), 6,3 (1H), 6,9-7,0 (3H), 7,2 (2H), 7,4 (2H), 7,5 (2H), 8,3 (1H), 8,45 (1H) und 12,3 (1H) ppm.

### Beispiel 64

### Cis-N-(1-(1-(2-Hydroxycyclohexyl) -6-trifluormethyl-chinoxalin-2,3-(1H,4H)-dion-7-yl)-pyrrol-3yl)-methyl-N'-(4-nitrophenyl)-harnstoff

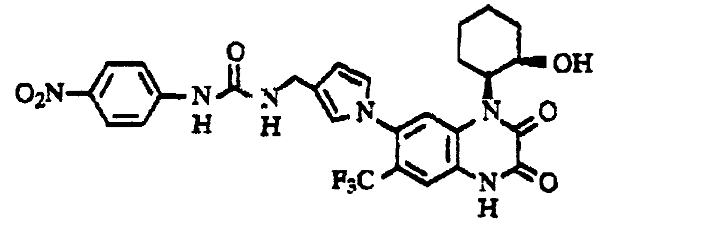

1,0 g (2,4 mMol) des Produktes 61h und 0,39 g (2,4 mMol) 4-Nitrophenylisocyanat wurden analog Vorschrift 2j umgesetzt.
Ausbeute: 0,4 g (27 %), Schmp. > 230°C (Z.)
¹H-NMR (D₆-DMSO):
- δ =: 1,2-2,0 (8H), 4,1-4,3 (3H), 4,8 (1H), 5,2 (1H), 6,2 (1H), 6,7 (1H), 6,9 (2H), 7,5-7,7 (3H), 8,1 (1H), 8,3 (1H), 9,3 (1H) und ca. 12,3 (breit) ppm.

### Beispiel 65

### N-(1-(1-(2-Hydroxyethyl)-6-trifluormethyl-chinoxalin-2,3-(1H,4H)-dion-7-yl)-pyrrol-3-yl)-methyl-N'-phenylharnstoff

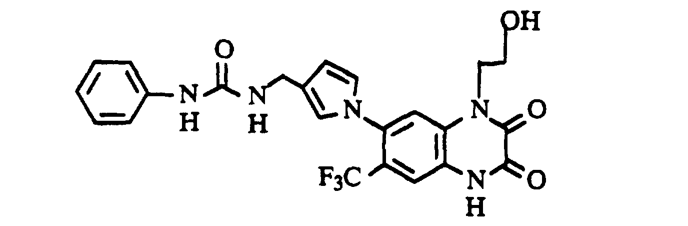

a) 4-(2-Hydroxyethylamino)-3-nitrobenzotrifluorid
   112,8 g (0,5 Mol) 4-Chlor-3-nitrobenzotrifluorid und 61,1 g (1 Mol) 2-Ethanolamin wurden in 500 ml Dimethylformamid für 2 h auf 100°C erwärmt. Danach wurde der Ansatz im Vakuum eingeengt und der Rückstand mit wasser versetzt. Der Niederschlag wurde abgesaugt und aus Cyclohexan umkristallisiert.
   Ausbeute: 116 g (46 %), Schmp. 68 bis 70°C
   ¹H-NMR (D₆-DMSO):
   - δ =: 3,5 (2H), 3,7 (2H), 5,0 (1H), 7,3 (1H), 7,8 (1H), 8,3 (1H) und 8,6 (1H) ppm.
b) 3-Amino-4-(2-hydroxyethylamino)-benzotrifluorid
   115 g (0,46 Mol) des Produktes 65a wurden in 1 Liter Isopropanol gelöst und mit 11,5 g Palladium/Kohle (10 %ig) in 200 ml Wasser versetzt. Alles wurde auf 80°C erwärmt. Danach wurden zügig 91 g (1,4 Mol) Ammoniumformiat, gelöst in 175 ml Wasser, zugetropft. Nachdem die Reaktion beendet war, wurde filtriert und der Alkohol aus dem Filtrat im Vakuum entfernt. Der dabei anfallende Niederschlag wurde abgesaugt, mit Toluol behandelt und erneut abgesaugt.
   Ausbeute: 68,4 g (68 %), Schmp. 92 bis 94°C
   ¹H-NMR (D₆-DMSO):
   - δ =: 3,2 (2H), 3,6 (2H), 4,8 (1H), 4,9 (2H), 5,1 (1H), 6,5 (1H) und 8,6 (2H) ppm.
c) 1-(2-Hydroxyethyl)-6-trifluormethyl-chinoxalin-2,3-(1H,4H)-dion
   60 g (0,27 Mol) des Produktes 65b wurden in 500 ml Oxalsäureethylester für 3 h unter Rückfluß gekocht. Nach dem Abkühlen wurde der Niederschlag abgesaugt.
   Ausbeute: 55 g (74 %), Schmp. 275 bis 276°C
   ¹H-NMR (D₆-DMSO):
   - δ =: 3,7 (2H), 4,2 (2H), 4,9 (1H), 7,4 (1H) und 12,2 (1H) ppm.
d) 1-(2-Hydroxyethyl)-7-nitro-6-trifluormethyl-chinoxalin-2,3-(1H,4H)-dion
   50 g (0,18 Mol) des Produktes 65c wurden in 500 ml konzentrierter Schwefelsäure gelöst und bei 0°C mit 21 g (0,21 Mol) Kaliumnitrat portionsweise versetzt. Alles wurde danach noch 30 min gerührt. Der Ansatz wurde anschließend auf Eis gegossen und der Niederschlag abgesaugt.
   Ausbeute: 25 g (44 %), Schmp. 254 bis 256°C.
   ¹H-NMR (D₆-DMSO):
   3,6 (2H), 4,1 (2H), 4,5 (1H), 7,6 (1H), 8,3 (1H) und ca. 12 (breit) ppm.
e) 7-Amino-1-(2-hydroxyethyl)-6-trifluormethyl-chinoxalin-2,3-(1H,4H)-dion
   25 g (78 mMol) des Produktes 65d wurden analog Vorschrift 65b mit 50 g (0,79 Mol) Ammoniumformiat reduziert.
   Ausbeute: 13,2 g (58 %), Schmp. 278°C (Z.)
   ¹H-NMR (D₆-DMSO):
   - δ =: 3,6 (2H), 4,1 (2H), 4,4 (breit), 5,5 (2H), 6,9 (1H), 7,1 (1H) und 11,8 (breit) ppm.
f) 1-(2-Hydroxyethyl)-7-(3-trifluoracetamidomethyl-pyrrol-1-yl)-6-trifluormethyl-chinoxalin-2,3-(1H,4H)-dion
   5 g (17 mMol) des Produktes 65e und 7,5 g (29 mMol) des Produktes 4a wurden analog Vorschrift 4b umgesetzt.
   Ausbeute: 7,6 g (96 %), Schmp. 100 bis 102°C (Z.)
   ¹H-NMR (D₆-DMSO):
   - δ =: 3,6 (2H), 4,1-4,4 (4H), 4,8 (1H), 6,2 (1H), 6,9 (2H), 7,5 (2H) und 9,8 (1H) ppm.
g) 7-(3-Aminomethyl-1-pyrrolyl)-1-(2-hydroxyethyl)-6-trifluormethyl-chinoxalin-2,3-(1H,4H)-dion
   7,0 g (15 mMol) des Produktes 65f wurden mit 1,4 g (59 mMol) Lithiumhydroxid analog Vorschrift 4c hydrolysiert.
   Ausbeute: 5,3 g (96 %)
   ¹H-NMR (D₆-DMSO):
   - δ =: 3,6 (2H), 3,9 (2H), 4,2 (2H), 6,4 (1H), 6,9 (1H), 7,4 (1H) und 7,8 (1H) ppm.
h) N-(1-(1-(2-Hydroxyethyl)-6-trifluormethyl-chinoxalin-2,3-(1H,4H)-dion-7-yl)-pyrrol-3-yl)-methyl-N'-phenylharnstoff
   1,0 g (2,7 mMol) des Produktes 65g und 0,6 g (5 mMol) Phenylisocyanat wurden analog Vorschrift 4d bei 120°C umgesetzt.
Ausbeute: 0,2 g (15 %)
¹H-NMR (D₆-DMSO):
- δ =: 3,6 (2H), 4,2 (4H), 6,2 (1H), 6,5 (1H), 6,9 (2H) und 7,2-7,6 (7H) ppm.

### Beispiel 66

### N-(1-(1-(2-Hydroxyethyl)-6-trifluormethyl-chinoxalin-2,3-(1H,4H)-dion-7-yl)-pyrrol-3-yl)-methyl-N'-(4-nitrophenyl)-harnstoff

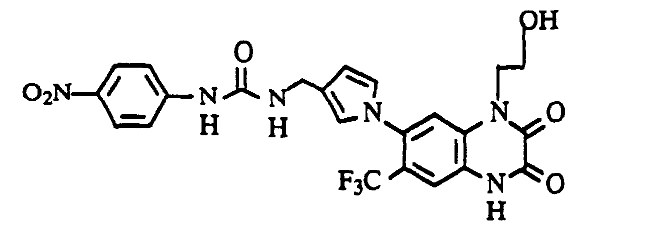

### 1 g (2,7 mMol) des Produktes 65g und 0,75 g (4,5 mMol) 4-Nitrophenylisocyanat wurden analog Vorschrift 4d bei 120°C umgesetzt.

Ausbeute: 0,1 g (7 %)
¹H-NMR (D₆-DMSO):
- δ =: 3,7 (2H), 4,2 (4H), 4,8 (1H), 6,2 (1H), 6,7 (1H), 6,9 (2H), 7,4-7,9 (4H), 8,1 (1H), 8,2 (1H), 9,3 (1H) und ca. 12,3 (breit) ppm.

### Beispiel 67

### N-(1-(1-Carboxymethyl-6-trifluormethyl-chinoxalin-2,3-(1H,4H)-dion-7-yl)-pyrrol-3-yl)methyl-N'-phenyl-guanidin

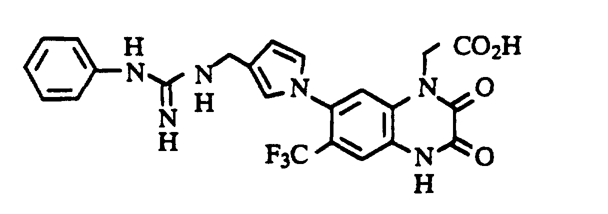

1,5 g (3,9 mMol) des Produktes 37g, 1,2 g (4,0 mMol) S-Methyl-N-phenyl-isothioharnstoffhydroiodid und eine Spatelspitze 4-(N,N-Dimethylamino)pyridin wurden in 50 ml Pyridin 3 h auf 100°C erhitzt. Anschließend wurde der Reaktionsansatz im Vakuum eingeengt und in wenig Ethanol gelöst. Durch Zugabe einer 10 %igen wäßrigen Natriumchlorid-Lösung wurde das Produkt ausgefällt, abgesaugt und mit Wasser gewaschen. Danach wurde das Produkt chromatographisch (Fließmittel: Toluol/Methanol/Tetrahydrofuran/ Eisessig = 5/5/10/0,1) gereinigt.
Ausbeute: 0,6 g (24 %), Schmp. > 230°C
¹H-NMR (D₆-DMSO):
- δ =: 4,3 (2H), 4,6 (2H), 6,2 (1H), 6,8 (1H), 6,9 (1H), 7,1-7,7 (7H), 8,0 (NH), 9,2 (NH), 11,5 breit) und 12,4 (breit) ppm.

### Beispiel 68

### N-(1-(1-Carboxymethyl-benzo[f]chinoxalin-2,3-(1H,4H)-dion-9-yl)-pyrrol-3-yl)-methyl-N'-(4-nitrophenyl)-harnstoff

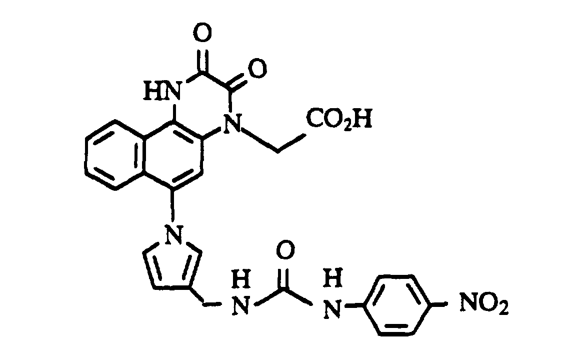

1,0 g (2,7 mMol) des Produktes 31b und 0,45 g (2,75 mMol) 4-Nitrophenylisocyanat wurden analog Vorschrift 31c umgesetzt.
Ausbeute: 0,7 g (49 %), Schmp. > 220°C
¹H-NMR (D₆-DMSO):
- δ =: 4,2 (2H), 5,0 (2H), 6,3 (1H), 6,8 (1H), 7,0 (2H), 7,3-7,7 (6H), 8,0 (2H), 8,7 (1H), 9,4 (1H) und ca. 12,3 (breit) ppm.

### Beispiel 69

### N-(1-(1-Carboxymethyl-6-trifluormethyl-chinoxalin-2,3-(1H,4H)-dion-7-yl)-pyrrol-3-yl)methyl-N'-(4-nitrophenyl)-guanidin

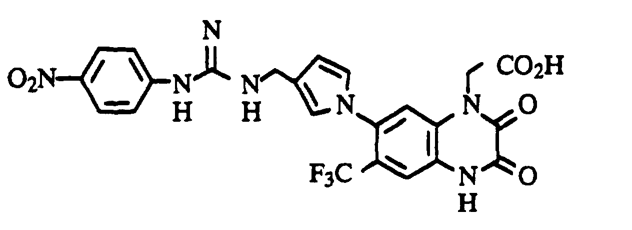

2,0 g (5,2 mMol) des Produktes 37g und 1,95 g (5,8 mMol) S-Methyl-N- (4-nitrophenyl)-isothioharnstoffhydroiodid wurden analog Beispiel 67 umgesetzt.
Ausbeute: 1,2 g (43 %), Schmp. > 212°C (Z.)
¹H-NMR (D₆-DMSO):
- δ =: 4,4 (2H), 5,0 (2H), 6,4 (1H), 6,9 (1H), 7,1 (1H), 7,4 (3H), 7,7 (1H), 8,1-8,6 (3H), 8,8 (1H), 10,7 (1H) und ca. 12,5 (breit) ppm.

### Beispiel 70

### N-(1-(1-Carboxymethyl-6-nitro-chinoxalin-2,3-(1H,4H)-dion-7-yl)-pyrrol-3-yl)methyl-N'-(2-(4-pyridyl)ethyl)-harnstoff

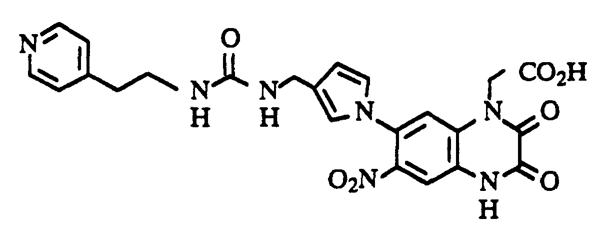

2,1 g (3,9 mMol) des Beispiels 42 wurden mit 0,28 g (11,7 mMol) Lithiumhydroxid analog Vorschrift 4c hydrolysiert.
Ausbeute: 1,4 g (69 %), Schmp. > 300°C
¹H-NMR (D₆-DMSO):
- δ =: 2,7 (2H), 3,3 (2H), 4,0 (2H), 4,9 (2H), 5,9 (1H), 6,1 (1H), 6,2 (2H), 6,8 (1H), 6,9 (1H), 7,2 (2H), 7,4 (1H), 7,8 (1H), 8,4 (2H) und 12,5 (breit) ppm.

### Beispiel 71

### N-(1-(1-Carboxymethyl-benzo[f]chinoxalin-2,3-(1H,4H)-dion-9-yl)-pyrrol-3-yl)methyl-N'- (4-ethoxycarbonylphenyl)-harnstoff

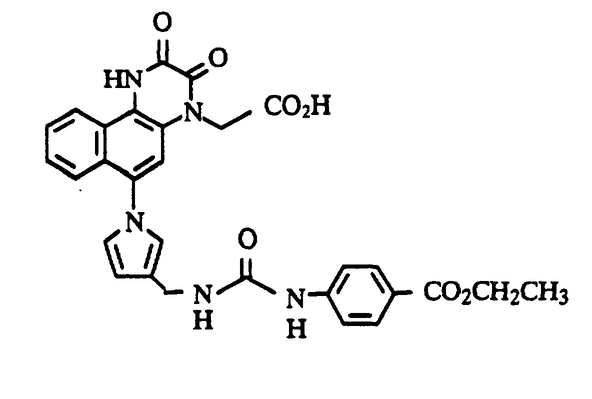

2,0 g (5,5 mMol) des Produktes 31b und 1,0 g (5,5 mMol) 4-Ethoxy-Carbonylphenylisocyanat wurden analog Vorschrift 31c bei 120°C umgesetzt.
Ausbeute: 2,1 g (71 %), Schmp. > 250°C
¹H-NMR (D₆-DMSO) :
- δ =: 1,3 (3H), 4,2 (4H), 4,8 (2H), 6,3 (1H), 6,8-7,1 (3H), 7,3-7,9 (6H), 8,7 (1H), 9,3 (1H) und ca. 12,4 (breit) ppm.

### Beispiel 72

### N-(1-(1-Carboxymethyl-benzo[f]chinoxalin-2,3-(1H, 4H)-dion-9-yl)-pyrrol-3-yl)methyl-N'- (4-carboxyphenyl)-harnstoff

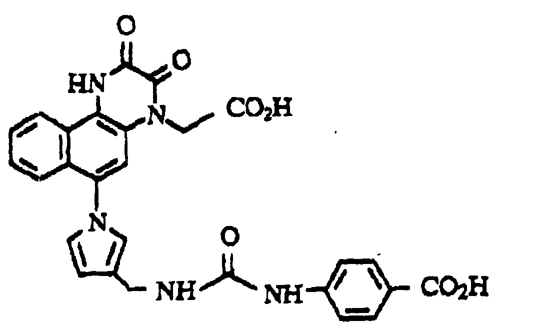

1,0g (1,8 mMol) des Produktes 71 wurden analog Vorschrift 7 hydryolysiert.
Ausbeute: 0,6 g (63 %)
¹H-NMR als Dikaliumsalz (D₆-DMSO):
4,2 (2H), 4,5 (2H), 6,2 (1H), 6,85 (1H), 6,9 (2H), 7,1-7,5 (6H), 7,7 (2H), 8,8 (1H) und 8,9 (1H) ppm.

### Beispiel 73

### N-(1-(1-Carboxymethyl-benzo[f]chinoxalin-2,3-(1H,4H)-dion-9-yl)-pyrrol-3-yl)-methyl-N'-(4-nitrophenyl)-guanidin

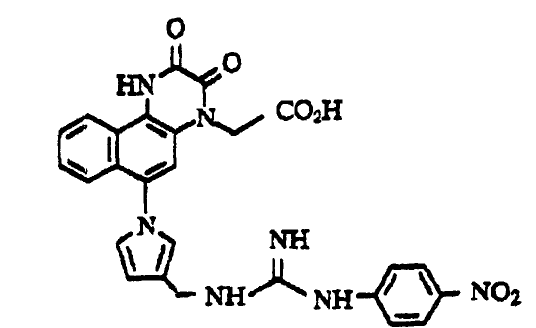

2 g (5,5 mMol) des Produktes 31b und 2 g (6,0 mMol) S-Methyl-N-(4-nitrophenyl)-isothioharnstoff-hydroiodid wurden analog Beispiel 67 umgesetzt.
Ausbeute: 1,9 g (66 %)
¹H-NMR (D₆-DMSO):
- δ =: 4,5 (2H), 5,1 (2H), 6,4 (1H), 7,1 (1H), 7,2 (1H), 7,3-7,9 (5H), 8,2-9,0 (5H), 10,7 (1H), 12,5 (1H) und ca. 13,3 (breit) ppm.

## Patentansprüche

1. Chinoxalin-2,3-(1H, 4H)-dione der allgemeinen Formel I 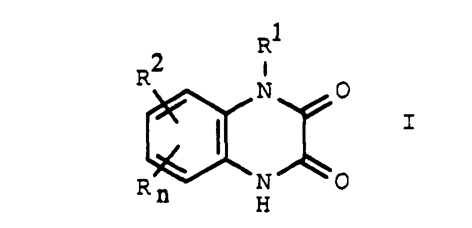 und ihre tautomeren und enantiomeren Formen sowie ihre physiologisch verträglichen Salze, wobei die Variablen folgende Bedeutung haben:
R¹
- Wasserstoff,
- ein cycloaliphatischer Rest mit bis zu 8 C-Atomen
- ein aliphatischer Rest mit 1 bis 6 C-Atomen, der einen oder zwei gleiche oder verschiedene der Substituenten Phenyl, Cyclopentyl, Cyclohexyl, -COR³, -CO-O-R³, 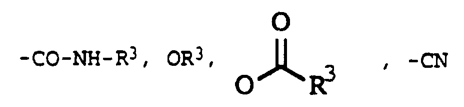 tragen kann, wobei R³ Wasserstoff, C₁-C₄-Alkyl, Phenyl, Benzyl, 1-Phenylethyl oder 2-Phenylethyl bedeutet, und wobei der in R¹ enthaltene oder R¹ darstellende cycloaliphatische Rest oder Phenylring bis zu drei gleiche oder verschiedene der folgenden Substituenten tragen kann: C₁-C₄-Alkyl, C₁-C₄-Halogenalkyl, C₁-C₄-Alkoxy, C₁-C₄-Halogenalkoxy, Halogen, Nitro, Cyano, -CO-OR⁵, -CO-NH-R⁵, -OH, 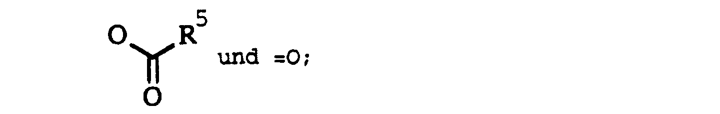 und wobei R⁵ unabhängig von R³ die gleichen Bedeutungen wie R³ hat,
R²
- eine Pyrrolgruppe 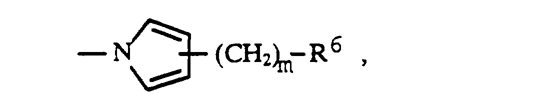
wobei R⁶ einer der folgenden Reste ist: 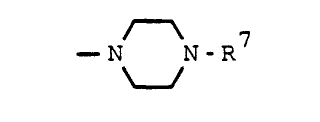 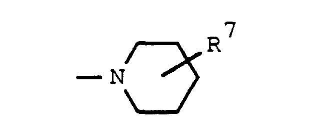 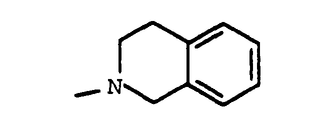 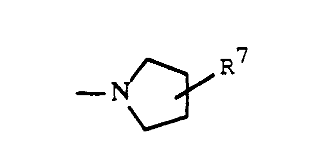 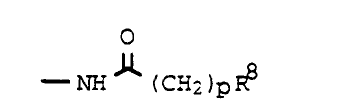 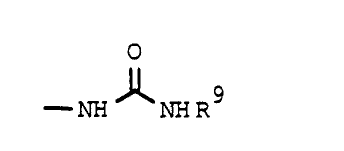 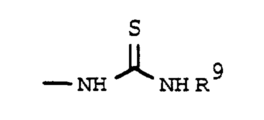 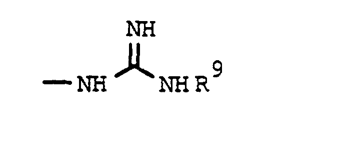 wobei R⁷ Wasserstoff, verzweigtes oder geradliniges C₁-C₄-Alkyl, das noch einen oder zwei Phenylringe tragen kann, bedeutet, und R⁸ einen der Reste 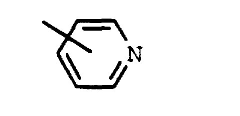 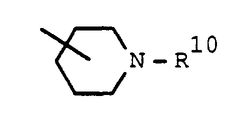 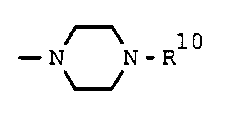 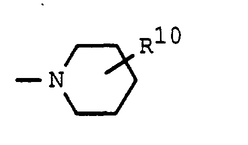 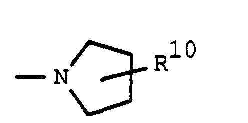 bedeutet, wobei R¹⁰ Wasserstoff und ein verzweigtes oder geradliniges C₁-C₄-Alkyl, das noch einen oder zwei Phenylringe tragen kann, bedeutet,
R⁹ Wasserstoff, Phenyl, ein verzweigtes oder geradliniges C₁-C₄-Alkyl, das noch einen oder zwei Phenylringe tragen kann, sowie 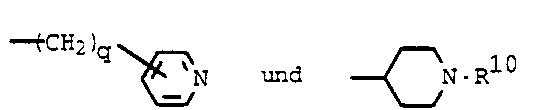 bedeutet,
m eine ganze Zahl von 1 bis 4 ist,
p und q unabhangig voneinander eine Zahl von 0 bis 4 bedeuten,
wobei die in R⁷, R⁹ und R¹⁰ ggf. enthaltenen Phenyl-Gruppen sowie der für R⁸ und R⁹ ggf. stehende oder in R⁹ ggf. enthaltene Pyridinring durch Halogen, -NO₂, -CF₃, -CN, -OH, -OCH₃, -NH₂, -NHCOCH₃, -OCF₃, -CO₂-(C₁-C₄-Alkyl), -CO₂H, -CONH₂, -CONH-(C₁-C₄-Alkyl), -CH₂-NHCOCF₃. -CH₂NH₂ und C₁-C₄-Alkyl substituiert sein können,
R - gleiche oder verschiedene der folgenden Reste : C₁-C₄-Alkyl, C₁-C₄-Alkoxy, Trifluormethyl, Trichlormethyl, Trifluormethoxy, Trichlormethoxy, Fluor, Chlor, Brom, Jod, Nitro und Cyano, sowie einen anellierten Benzolring, der seinerseits bis zu zwei der oben für R genannten Reste (ohne den anellierten Benzolring) tragen kann, und
n - eine ganze Zahl von 0 bis 2, für den anellierten Benzolring 0 oder 1 bedeutet.

2. Chinoxalin-2,3(1H,4H)-dione der Formel I gemäß Anspruch 1 zur Verwendung als Arzneimittel für die Human- und Veterinärmedizin.

3. Verwendung der Chinoxalin-2,3(1H,4H)-dione der Formel I gemäß Anspruch 1 zur Herstellung von Arzneimitteln zur Behandlung neurodegenerativer Erkrankungen und neurotoxischer Störungen des zentralen Nervensystems sowie zur Herstellung von Antiepileptika, Anxiolytika und Antidepressiva.

4. Arzneimittelzubereitung zur peroralen oder parenteralen Anwendung, enthaltend neben den üblichen Arzneimittelhilfsstoffen pro Einzeldosis 0,1 bis 100 mg pro kg Körpergewicht mindestens eines Chinoxalin-2,3(1H,4H)-dions I gemäß Anspruch 1.

5. Arzneimittelzubereitung zur topischen Anwendung, enthaltend neben den üblichen Arzneimittelhilfsstoffen 0,0001 bis 1 Gew.-% mindestens eines Chinoxalin-2,3(1H,4H)-dions I gemäß Anspruch 1.

## Claims

1. Quinoxaline-2,3-(1H,4H)-diones of general formula I: 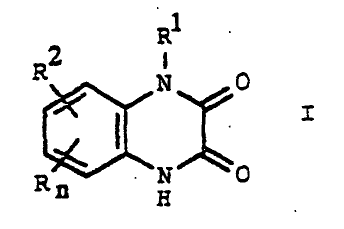 and their tautomeric and enantiomeric forms, as well as their physiologically compatible salts, wherein the variables are defined as follows:
R¹
- hydrogen,
- a cycloaliphatic radical having up to 8 C atoms,
- an aliphatic radical having 1 to 6 C atoms which can be substituted with one or two identical or different substituents selected from phenyl, cyclopentyl, cyclohexyl, -COR³, -CO-O-R³, -CO-NH-R³, OR³, 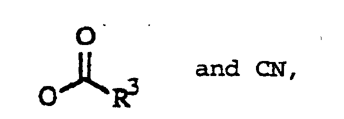
R³ being hydrogen, C₁-C₄-alkyl, phenyl, benzyl, 1-phenylethyl or 2-phenylethyl, and it being possible for the cycloaliphatic radical or phenyl ring contained in R¹ or representing R¹ to be substituted with up to three identical or different substituents selected from C₁-C₄-alkyl, C₁-C₄-halogenoalkyl, C₁-C₄-alkoxy,
C₁-C₄-halogenoalkoxy, halogen, nitro, cyano, -CO-OR⁵, -CO-NH-R⁵, -OH, 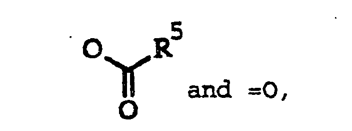
R⁵ independently of R³ being defined in the same way as R³,
R²
- a pyrrole group 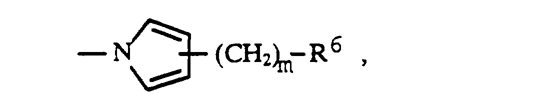
R⁶ being one of the following radicals: 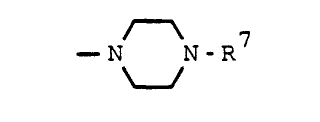 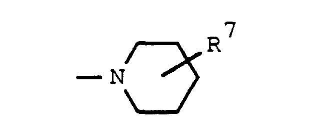 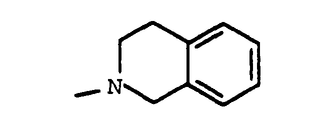 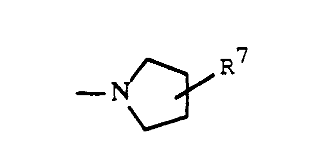 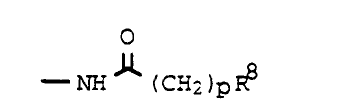 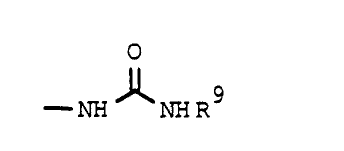 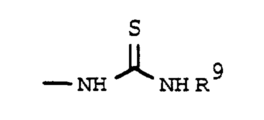 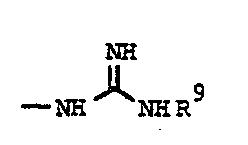
R⁷ being hydrogen or a branched or linear C₁-C₄ alkyl which can also be substituted with one or two phenyl rings, and R⁸ being one of the radicals 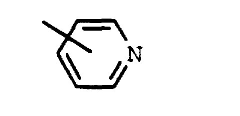 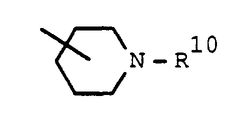 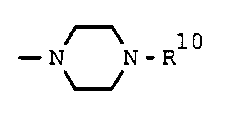 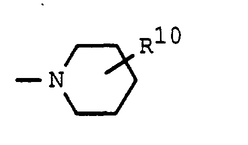 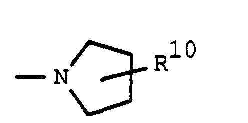
R¹⁰ being hydrogen and a branched or linear C₁-C₄-alkyl which can also be substituted with one or two phenyl rings,
R⁹ being hydrogen, phenyl, a branched or linear C₁-C₄-alkyl which can also be substituted with one or two phenyl rings, 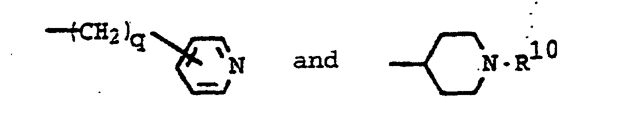
m being an integer from 1 to 4;
p and q independently of one another being a number from 0 to 4,
it being possible for the phenyl groups which may be contained in R⁷, R⁹ and R¹⁰ and the pyridine ring which may represent R⁸ and R⁹ or be contained in R⁹ to be substituted by halogen, NO₂, -CF₃, -CN, -OH, -OCH₃, -NH₂, -NHCOCH₃, -OCF₃, -CO₂- (C₁-C₄-alkyl), -CO₂H, -CONH₂, -CONH-(C₁-C₄-alkyl), -CH₂-NHCOCF₃, -CH₂NH₂ and C₁-C₄-alkyl,
R
- identical or different radicals selected from C₁-C₄-alkyl, C₁-C₄-alkoxy, trifluoromethyl, trichloromethyl, trifluoromethoxy, trichloromethoxy, fluorine, chlorine, bromine, iodine, nitro and cyano, as well as a fused benzene ring which in turn can be substituted with up to two of the radicals mentioned above for R (except for the fused benzene ring), and
n
- an integer from 0 to 2 or, for the fused benzene ring, 0 or 1.

2. Quinoxaline-2,3-(1H,4H)-diones of formula I according to Claim 1 for use as drugs for human and veterinary medicine.

3. Use of the quinoxaline-2,3-(1H,4H)-diones of formula I according to Claim 1 for the preparation of drugs for the treatment of neurodegenerative diseases and neurotoxic disorders of the central nervous system; and for the preparation of antiepileptics, anxiolytics and antidepressants.

4. Pharmaceutical formulation for oral or parenteral administration, containing 0.1 to 100 mg per kg body weight of at least one quinoxaline-2,3-(1H,4H)-dione I according to Claim 1 per single dose, in addition to the conventional pharmaceutical adjuncts.

5. Pharmaceutical formulation for topical application, containing 0.0001 to 1 wt.% of at least one quinoxaline-2,3-(1H,4H)-dione I according to Claim 1, in addition to the conventional pharmaceutical adjuncts.

## Revendications

1. Quinoxaline-2,3-(1H, 4H) -diones de formule générale I 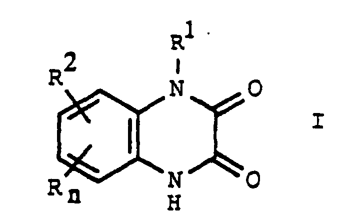 et ses formes tautomères et énantiomères, ainsi que ses sels physiologiquement acceptables, tandis que les variables ont la signification suivante:
R1
- hydrogène,
- un reste cycloaliphatique ayant jusqu'à 8 atomes de carbone,
- un reste aliphatique ayant 1 à 6 atomes de carbone, qui peut porter un ou deux, identiques
ou différents, des substituants phényle, cyclopentyle, cyclohexyle, -COR³, -CO-O-R³, -CO-NH-R³, OR³, 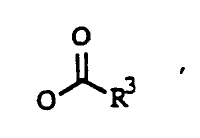 -CN
tandis que R³ représente un hydrogène, alkyle en C₁-C₄, phényle, benzyle, 1-phényléthyle ou 2-phényléthyle, et tandis que le reste cycloaliphatique ou le cycle phényle contenu dans R¹ ou représentant R¹ peut porter jusqu'à trois des substituants suivants, identiques ou différents: alkyle en C₁-C₄, halogénoalkyle en C₁-C₄, alcoxy en C₁-C₄, halogénoalcoxy en C₁-C₄, halogéno, nitro, cyano, -CO-OR⁵, -CO-NH-R⁵, -OH, 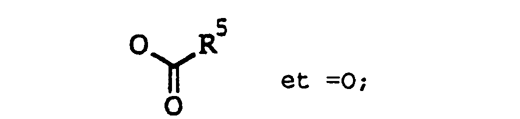 et tandis que R⁵ a, indépendamment de R³, les mêmes significations que R³,
R²
- un groupe pyrrole 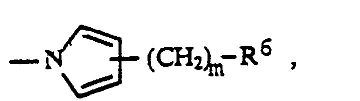
où R⁶ est l'un des restes suivants: 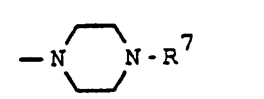 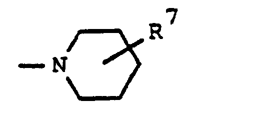 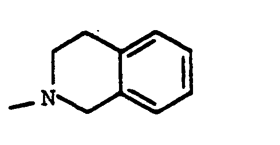 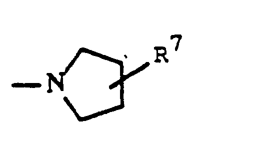 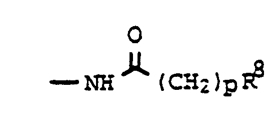 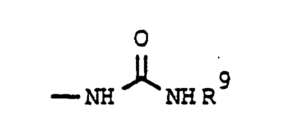 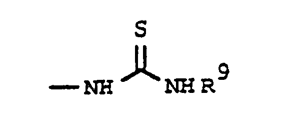 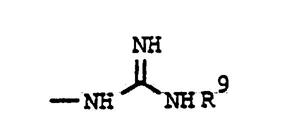 tandis que R⁷ désigne un hydrogène, alkyle en C₁-C₄, ramifié ou linéaire, qui peut encore porter un ou deux cycles phényle, et R⁸ représente l'un des restes 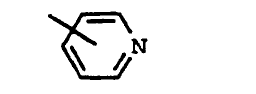 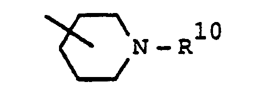 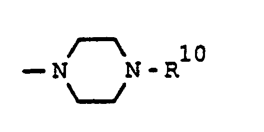 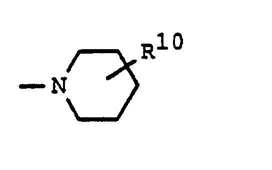 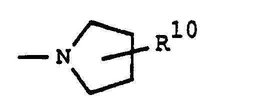 tandis que R¹⁰ désigne un hydrogène et un alkyle en C₁-C₄, ramifié ou linéaire, qui peut encore porter un ou deux cycles phényle,
R⁹ représente
un hydrogène, phényle, un alkyle en C₁-C₄ ramifié
ou linéaire, qui peut encore porter un ou deux cycles phényle, ainsi que 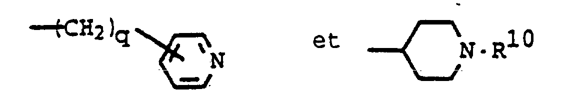
m est un nombre entier de 1 à 4,
p et q représentent, indépendamment l'un de l'autre, un nombre de 0 à 4, tandis que les groupes phényle éventuellement contenus dans R⁷, R⁹ et R¹⁰, ainsi que le cycle pyridine se trouvant éventuellement pour R⁸ et R⁹ ou éventuellement contenu dans R⁹ peuvent être substitués par des groupes halogéno, -NO₂, -CF₃, -CN, -OH, -OCH₃, -NH₂, -NHCOCH₃, -OCF₃, -CO₂-(alkyle en C₁-C₄), -CO₂H, -CONH₂, -CONH-(alkyle en C₁-C₄), -CH₂-NHCOCF₃, -CH₂NH₂ et alkyle en C₁-C₄,
R
- identiques ou différents des restes suivants: alkyle en C₁-C₄, alcoxy en C₁-C₄, trifluorométhyle, trichlorométhyle, trifluorométhoxy, trichlorométhoxy, fluoro, chloro, bromo, iodo, nitro et cyano, ainsi qu'un cycle benzène annelé, qui peut quant à lui porter jusqu'à deux des restes indiqués plus haut pour R (sans le cycle benzène annelé), et
n
- un nombre entier de 0 à 2, pour le cycle benzène annelé 0 ou 1.

2. Quinoxaline-2,3(1H,4H)-diones de formule I selon la revendication 1 pour utilisation comme médicament pour la médecine humaine et vétérinaire.

3. Utilisation des quinoxaline-2,3(1H,4H)-diones de formule I selon la revendication 1 pour la préparation de médicaments pour le traitement des affections neurodégénératives et des perturbations neurotoxiques du système nerveux central, ainsi que pour la préparation d'antiépileptiques, d'anxiolytiques et d'antidépresseurs.

4. Composition pharmaceutique pour utilisation perorale ou parentérale, contenant, outre les adjuvants médicamenteux classiques, par dose unitaire 0,1 à 100 mg par kg de poids corporel d'au moins une quinoxaline-2,3(1H,4H)-dione I selon la revendication 1.

5. Composition pharmaceutique pour utilisation topique, contenant, outre les adjuvants médicamenteux classiques, 0,0001 à 1 % en poids d'au moins une quinoxaline-2,3(1H,4H)-dione I selon la revendication 1.
